# EUROPEAN PATENT APPLICATION

(11) **EP 4 122 928 A1**
(43) Date of publication of application: **25.01.2023**
(21) Application number: 21792938.9
(22) Date of filing: 20.04.2021
(51) Int. Cl.: C07D 401/14, C07D 403/14, A61K 31/4439, A61K 31/444, A61P 35/00, A61P 31/00

(54) **SOLID FORM OF PYRAZINE SUBSTITUTED NICOTINAMIDE, AND PREPARATION AND USE THEREOF**

(30) Priority: 20.04.2020 CN 202010309497
(71) Applicant: Shenzhen TargetRx, Inc., Shenzhen, Guangdong 518057 (CN)
(72) Inventor: WANG, Yihan, Shenzhen, Guangdong 518057 (CN); ZHAO, Jiuyang, Shenzhen, Guangdong 518057 (CN)
(74) Representative: Novitas Patent AB
(86) International application number: PCT/CN2021/088378
(87) International publication number: WO 2021/213380

(57) **Abstract**

Provided are a crystal form of a free base or a pharmaceutically acceptable salt of (R)-N-(4-(chlorodifluoromethoxy)phenyl)-6-(3-fluoropyrrolidin-1-yl)-5-(pyrazin-2-yl)nicotinamide (compound A), a preparation method therefor, and use of the compound in preparation of drugs for treating diseases mediated by Bcr-Abl kinase and mutants thereof, such as chronic granulocytic leukemia. Also provided are a method for preparing compound A, and a preparation containing compound A.

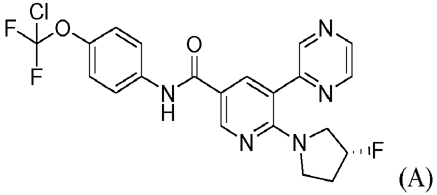

## Description

### FIELD OF THE INVENTION

The present disclosure belongs to the field of pharmaceutical technology and relates in particular to crystalline forms of the free base of (R)-N-(4-(chlorodifluoromethoxy)phenyl)-6-(3-fluoropyrrolidin-1-yl)-5-(pyrazin-2-yl)nicotinamide (compound A or compound of formula (A)) or pharmaceutically acceptable salts thereof, as well as methods of preparation thereof, and the use of the compound in the manufacture of a medicament for the treatment of diseases mediated by Bcr-Abl kinase and its mutants, such as chronic myelocytic leukemia. The present disclosure also relates to methods of preparing compound A, and to formulations containing compound A.

### BACKGROUND OF THE INVENTION

The chemical formula of (R)-N-(4-(chlorodifluoromethoxy)phenyl)-6-(3-fluoropyrrolidin-1-yl)-5-(pyrazin-2-yl)nicotinamide (compound A) is C₂₁H₁₇ClF₃N₅O₂, the molecular weight is 463.85 g/mol, and its chemical structure is:

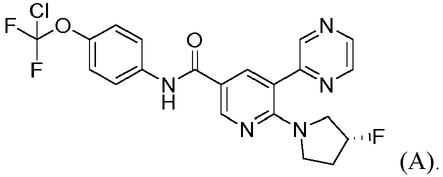

Compound A is an allosteric inhibitor of Abl1 targeting the myristoyl binding site, which can be used to treat diseases mediated by Bcr-Abl kinase and its mutants, such as chronic myelocytic leukemia. International Patent Publication No. WO 2018/133827 A1 first disclosed this compound, but did not disclose the crystalline form of Compound A. The applicant of WO 2018/133827 A1 is Shenzhen TargetRx Inc. The corresponding Chinese application CN 201880000986.9 of WO 2018/133827 A1 was published with the Grant No. CN 108602800 B on August 27, 2019. In addition, WO 2018/133827 A1 has corresponding US application US 16/479,299, European application EP 18741306.7 and Japanese application JP 2019-560440. The contents of each of the above applications are incorporated herein by reference in their entirety.

### SUMMARY OF THE INVENTION

In one aspect, the present disclosure provides various crystalline forms of the free base of (R)-N-(4-(chlorodifluoromethoxy)phenyl)-6-(3-fluoropyrrolidin-1-yl)-5-(pyrazin-2-yl)nicotinamide (compound A).

In one embodiment, the present disclosure provides the crystal form I of (R)-N-(4-(chlorodifluoromethoxy)phenyl)-6-(3-fluoropyrrolidin-1-yl)-5-(pyrazin-2-yl)nicotinamide (the crystal form I of compound A).

In another embodiment, the present disclosure provides the crystal form II of (R)-N-(4-(chlorodifluoromethoxy)phenyl)-6-(3-fluoropyrrolidin-1-yl)-5-(pyrazin-2-yl)nicotinamide (the crystal form II of compound A).

In another embodiment, the present disclosure provides the crystal form III of (R)-N-(4-(chlorodifluoromethoxy)phenyl)-6-(3-fluoropyrrolidin-1-yl)-5-(pyrazin-2-yl)nicotinamide (the crystal form III of compound A).

In another embodiment, the present disclosure provides the crystal form IV of (R)-N-(4-(chlorodifluoromethoxy)phenyl)-6-(3-fluoropyrrolidin-1-yl)-5-(pyrazin-2-yl)nicotinamide (the crystal form IV of compound A).

In another embodiment, the present disclosure provides the crystal form V of (R)-N-(4-(chlorodifluoromethoxy)phenyl)-6-(3-fluoropyrrolidin-1-yl)-5-(pyrazin-2-yl)nicotinamide (the crystal form V of compound A).

In another embodiment, the present disclosure provides the crystal form VI of (R)-N-(4-(chlorodifluoromethoxy)phenyl)-6-(3-fluoropyrrolidin-1-yl)-5-(pyrazin-2-yl)nicotinamide (the crystal form VI of compound A).

In another embodiment, the present disclosure provides the crystal form VII of (R)-N-(4-(chlorodifluoromethoxy)phenyl)-6-(3-fluoropyrrolidin-1-yl)-5-(pyrazin-2-yl)nicotinamide (the crystal form VII of compound A).

In another embodiment, the present disclosure provides the crystal form VIII of (R)-N-(4-(chlorodifluoromethoxy)phenyl)-6-(3-fluoropyrrolidin-1-yl)-5-(pyrazin-2-yl)nicotinamide (the crystal form VIII of compound A).

In another embodiment, the present disclosure provides the crystal form IX of (R)-N-(4-(chlorodifluoromethoxy)phenyl)-6-(3-fluoropyrrolidin-1-yl)-5-(pyrazin-2-yl)nicotinamide (the crystal form IX of compound A).

In another embodiment, the present disclosure provides the crystal form X of (R)-N-(4-(chlorodifluoromethoxy)phenyl)-6-(3-fluoropyrrolidin-1-yl)-5-(pyrazin-2-yl)nicotinamide (the crystal form X of compound A).

In another embodiment, the present disclosure provides the crystal form XI of (R)-N-(4-(chlorodifluoromethoxy)phenyl)-6-(3-fluoropyrrolidin-1-yl)-5-(pyrazin-2-yl)nicotinamide (the crystal form XI of compound A).

In another aspect, the present disclosure provides various crystalline forms of the salts of compound A.

In one embodiment, the present disclosure provides (R)-N-(4-(chlorodifluoromethoxy)phenyl)-6-(3-fluoropyrrolidin-1-yl)-5-(pyrazin-2-yl)nicotinamide hydrochloride and the crystal form I of the hydrochloride (the crystal form I of compound A hydrochloride).

In one embodiment, the present disclosure provides (R)-N-(4-(chlorodifluoromethoxy)phenyl)-6-(3-fluoropyrrolidin-1-yl)-5-(pyrazin-2-yl)nicotinamide benzenesulfonate and the crystal form I of the benzenesulfonate (the crystal form I of compound A benzenesulfonate).

In one embodiment, the present disclosure provides (R)-N-(4-(chlorodifluoromethoxy)phenyl)-6-(3-fluoropyrrolidin-1-yl)-5-(pyrazin-2-yl)nicotinamide p-toluenesulfonate and the crystal form I of the p-toluenesulfonate (the crystal form I of compound A p-toluenesulfonate).

In one embodiment, the present disclosure provides (R)-N-(4-(chlorodifluoromethoxy)phenyl)-6-(3-fluoropyrrolidin-1-yl)-5-(pyrazin-2-yl)nicotinamide p-toluenesulfonate and the crystal form II of the p-toluenesulfonate (the crystal form II of compound A p-toluenesulfonate).

In one embodiment, the present disclosure provides (R)-N-(4-(chlorodifluoromethoxy)phenyl)-6-(3-fluoropyrrolidin-1-yl)-5-(pyrazin-2-yl)nicotinamide mesylate and the crystal form I of the mesylate (the crystal form I of compound A mesylate).

In one embodiment, the present disclosure provides (R)-N-(4-(chlorodifluoromethoxy)phenyl)-6-(3-fluoropyrrolidin-1-yl)-5-(pyrazin-2-yl)nicotinamide hydrobromide and the crystal form I of the hydrobromide (the crystal form I of compound A hydrobromide).

In another aspect, the present disclosure provides a method for preparing the crystal form VI of compound A, which comprises converting the crystal form VII of compound A to the crystal form VI of compound A.

In one embodiment, the present disclosure provides a method for preparing a compound of formula (A): 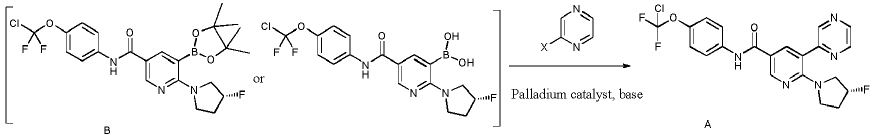 wherein X is a halogen; alternatively I or Br; still alternatively I;
comprising reacting the compound of formula (B), the boronic acid hydrolysis product thereof, or a mixture thereof with 2-halopyrazine in DMSO or DMF in the presence of a palladium catalyst and a base.

In another embodiment, the present disclosure provides a method for preparing a compound of formula (A): 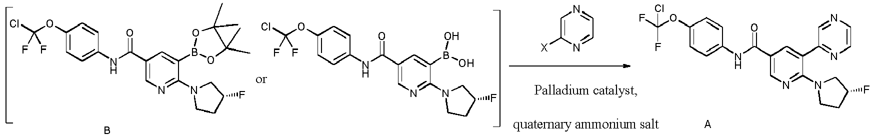 wherein X is a halogen; alternatively I or Br; still alternatively I;
comprising reacting the compound of formula (B), the boronic acid hydrolysis product thereof, or a mixture thereof with 2-halopyrazine in the presence of a palladium catalyst and a quaternary ammonium salt.

In another aspect, the present disclosure provides a pharmaceutical composition comprising (i) a pharmaceutically active ingredient: a crystal form of the free base of compound A or a crystal form of a pharmaceutically acceptable salt thereof, (ii) a diluent, (iii) a disintegrant, (iv) a glidant, and (v) a lubricant.

In another aspect, the present disclosure provides a pharmaceutical composition comprising (i) a compound of formula (A), (ii) a diluent, (iii) a disintegrant, (iv) a glidant, and (v) a lubricant, wherein the glidant accounts for 1-5%, alternatively 2-4%, or alternatively about 3%, by weight, of the total weight of the pharmaceutical composition.

In another aspect, the present disclosure provides the use of the above crystal form in the manufacture of a medicament for the treatment and/or prevention of diseases caused by Bcr-Abl.

In another aspect, the present disclosure provides the above crystal form for use in the treatment and/or prevention of diseases caused by Bcr-Abl.

In another aspect, the present disclosure provides a method of treating and/or preventing a disease caused by Bcr-Abl in a subject, comprising administering to the subject the above crystal form.

In another aspect, the above diseases caused by Bcr-Abl include solid tumors, sarcomas, acute lymphocytic leukemia, acute myelocytic leukemia, chronic lymphocytic leukemia, chronic myelocytic leukemia, gastrointestinal stromal tumor, thyroid cancer, gastric cancer, rectal cancer, multiple myeloma, neoplasia, and other proliferative diseases; or the disease caused by Bcr-Abl1 is metastatic invasive cancer, viral infection, or CNS disorder.

### BRIEF DESCRIPTION OF THE DRAWINGS

FIG. 1 shows XRPD pattern of the crystal form I of compound A.
FIG.2 shows DVS curve of the crystal form I of compound A.
FIG.3 shows comparison of XRPD pattern of the crystal form I of compound A before and after DVS test.
FIG.4 shows XRPD pattern of the crystal form II of compound A.
FIG.5 shows XRPD pattern of the crystal form III of compound A.
FIG.6 shows XRPD pattern of the crystal form IV of compound A.
FIG.7 shows XRPD pattern of the crystal form V of compound A.
FIG.8 shows XRPD pattern of the crystal form VI of compound A.
FIG.9 shows ¹H NMR spectrum of the crystal form VI of compound A.
FIG. 10 shows ¹³C NMR spectrum of the crystal form VI of compound A.
FIG. 11 shows NMR DEPT spectrum of the crystal form VI of compound A.
FIG. 12 shows NMR ¹⁹F-NMR spectrum of the crystal form VI of compound A.
FIG. 13 shows NMR H-F NOESY spectrum of the crystal form VI of compound A.
FIG. 14 shows NMR HSQC spectrum of the crystal form VI of compound A.
FIG. 15 shows NMR HMBC spectrum of the crystal form VI of compound A.
FIG. 16 shows NMR COSY spectrum of the crystal form VI of compound A.
FIG. 17 shows NMR NOESY spectrum of the crystal form VI of compound A.
FIG. 18 shows mass spectrum of the crystal form VI of compound A.
FIG. 19 shows UV spectrum of the crystal form VI of compound A.
FIG.20 shows DSC curve of the crystal form VI of compound A.
FIG.21 shows TGA curve of the crystal form VI of compound A.
FIG.22 shows DVS curve of the crystal form VI of compound A.
FIG.23 shows IR spectrum of the crystal form VI of compound A.
FIG.24 shows XRPD pattern of the crystal form VII of compound A.
FIG.25 shows XRPD pattern of the crystal form VIII of compound A.
FIG.26 shows XRPD pattern of the crystal form IX of compound A.
FIG.27 shows XRPD pattern of the crystal form X of compound A.
FIG.28 shows XRPD pattern of the crystal form XI of compound A.
FIG.29 shows XRPD pattern for stability study of the crystal form I of compound A.
FIG.30 shows XRPD patterns of the crystal form III, the crystal form VI and the crystal form VII of compound A before and after grinding.
FIG.31 shows XRPD pattern of the crystal form I of compound A hydrochloride.
FIG.32 shows DVS curve of the crystal form I of compound A hydrochloride.
FIG.33 shows comparison of XRPD pattern of the crystal form I of compound A hydrochloride before and after DVS test.
FIG.34 shows XRPD pattern of the crystal form I of compound A benzenesulfonate.
FIG.35 shows DVS curve of the crystal form I of compound A benzenesulfonate.
FIG.36 shows comparison of XRPD pattern of the crystal form I of compound A benzenesulfonate before and after DVS test.
FIG.37 shows XRPD pattern of the crystal form I of compound A p-toluenesulfonate.
FIG.38 shows DVS curve of the crystal form I of compound A p-toluenesulfonate.
FIG.39 shows comparison of XRPD pattern of the crystal form I of compound A p-toluenesulfonate before and after DVS test.
FIG.40 shows XRPD pattern of the crystal form II of compound A p-toluenesulfonate.
FIG.41 shows XRPD pattern of the crystal form I of compound A mesylate.
FIG.42 shows DVS curve of the crystal form I of compound A mesylate.
FIG.43 shows comparison of XRPD pattern of the crystal form I of compound A mesylate before and after DVS test.
FIG.44 shows XRPD pattern of the crystal form I of compound A hydrobromide.
FIG.45 shows DVS curve of the crystal form I of compound A hydrobromide.
FIG.46 shows comparison of XRPD pattern of the crystal form I of compound A hydrobromide before and after DVS test.
FIG.47 shows XRPD pattern for stability study of the crystal form I of compound A p-toluenesulfonate.
FIG.48 is XRPD pattern showing crystal form change of the crystal form I of compound A dissolved in SGF.
FIG.49 is XRPD pattern showing crystal form change of the crystal form I of compound A dissolved in FaSSIF.
FIG.50 is XRPD pattern showing crystal form change of the crystal form I of compound A dissolved in FeSSIF.
FIG.51 is XRPD pattern showing crystal form change of the crystal form I of compound A p-toluenesulfonate dissolved in SGF.
FIG.52 is XRPD pattern showing crystal form change of the crystal form I of compound A p-toluenesulfonate dissolved in FaSSIF.
FIG.53 is XRPD pattern showing crystal form change of the crystal form I of compound A p-toluenesulfonate dissolved in FeSSIF.
FIG.54 shows crystal structure diagram of compound A.
FIG.55 shows XRPD pattern for stability study of the crystal form VI of compound A.

### DETAILED DESCRIPTION OF THE INVENTION

The present disclosure can be understood more readily by reference to the following detailed description of embodiments of the disclosure and the examples included herein. It is to be understood that the terms used herein are intended to describe specific embodiments only and are not intended to be limiting. It is to be further understood that unless specifically defined by the context, terms used herein shall be given their ordinary meanings as known in the relevant art.

As used herein, the singular forms "a", "an" and "the" include plural references unless specified otherwise. For example, the term "a" compound includes one or more compounds.

The term "about" means having a value that falls within the standard error of the accepted mean when considered by a person of ordinary skill in the art. For example, "about" means ±10% of the indicated amount, or ±5% of the indicated amount.

As used herein, the term "substantially" means taking into account the typical variability of a particular method and the standard error of a measured value. For example, with respect to the location of an X-ray powder diffraction peak, the term "substantially" is meant to take into account the typical variability in peak location and intensity. Those skilled in the art will recognize that peak location (2θ) will exhibit some variability, typically up to ±0.2°. In addition, those skilled in the art will recognize that relative peak intensities will reveal inter-device variability as well as variability due to crystallinity, preferred orientation, sample surface tested, and other factors known to those of skill in the art. Similarly, the NMR spectra (ppm) of ¹H, ¹³C and ¹⁹F show variability, typically up to ±0.2 ppm.

As used herein, the terms "crystalline" and "crystal form" refer to a solid composed of molecules with regular repetitive arrangement. Crystalline forms may differ in terms of thermodynamic stability, physical parameters, X-ray structure and preparation processes.

The term "amorphous" refers to a solid composed of molecules with disordered arrangement.

As used herein, the term "solvate" refers to a crystalline form having a stoichiometric or non-stoichiometric amount of a solvent (e.g., water, methanol, ethyl acetate, etc., or mixtures thereof) in the crystal lattice through non-covalent intermolecular bonding. The term "hydrate" refers to a solvate in which the solvent is water.

As used herein, the term "anhydrous" refers to a crystalline form that contains less than about 1% (w/w) adsorbed moisture as determined by standard methods such as Karl Fisher analysis.

### Compound A and crystal forms thereof

The compound, (R)-N-(4-(chlorodifluoromethoxy)phenyl)-6-(3-fluoropyrrolidin-1-yl)-5-(pyrazin-2-yl)nicotinamide, is referred to herein as compound A, or the free base of compound A, and has the formula of:

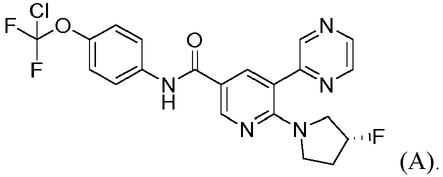

The present disclosure relates to various crystalline forms of compound A, such as "the crystal form I of compound A", "the crystal form II of compound A", "the crystal form III of compound A", "the crystal form IV of compound A", "the crystal form V of compound A", "the crystal form VI of compound A", "the crystal form VII of compound A", "the crystal form VIII of compound A", "the crystal form IX of compound A", "the crystal form X of compound A", and "the crystal form XI of compound A". In some embodiments, the crystal forms of compounds may be in a solvated, hydrated, or unsolvated form.

### The crystal form I of compound A

In one embodiment, the present disclosure provides the crystal form I of compound A, which is an ethanol solvate, wherein the molar ratio of ethanol to the free base is 1:2.

In another embodiment, the X-ray powder diffraction pattern of the crystal form I obtained using CuK_{α} radiation includes at least the characteristic peaks located at the following °2θ: 18.3±0.2 and 24.4±0.2. In another embodiment, the X-ray powder diffraction pattern further includes the characteristic peaks located at the following °2θ: 9.6±0.2, 19.3±0.2, 21.8±0.2, 22.5±0.2 and 24.9±0.2. In another embodiment, the X-ray powder diffraction pattern further includes the characteristic peaks located at the following °2θ: 10.7±0.2, 12.2±0.2, 16.2±0.2, 22.8±0.2, 23.4±0.2 and 27.8±0.2.

In another embodiment, the X-ray powder diffraction pattern has the following characteristic peaks:

| Angle °2θ±0.2 °2θ | Relative intensity % |
|---|---|
| 9.6 | 48.1 |
| 18.3 | 59.6 |
| 19.3 | 25.8 |
| 21.8 | 31.2 |
| 22.5 | 31.3 |
| 24.4 | 100 |
| 24.9 | 36.3 |

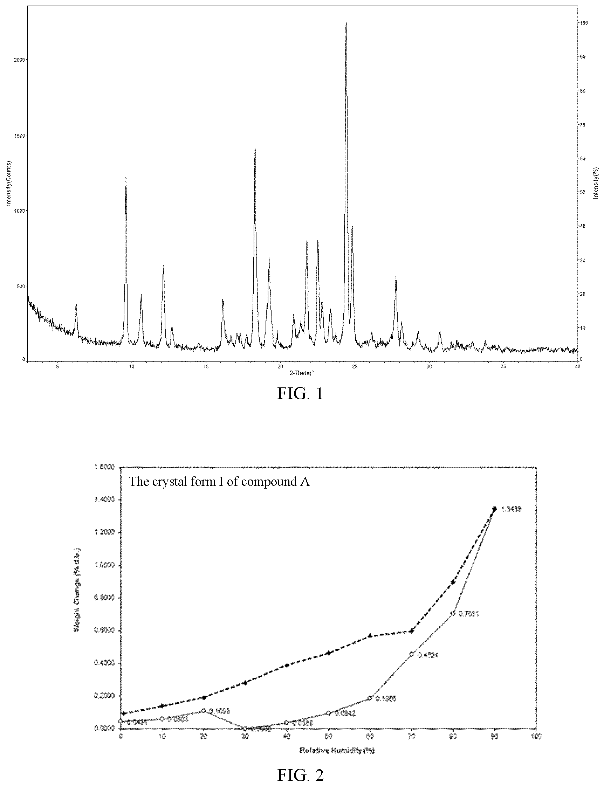

In another embodiment, the X-ray powder diffraction pattern comprises one or more peaks at the 2θ value in Table 3.1. In another embodiment, the X-ray powder diffraction pattern is substantially as shown in FIG. 1.

In another embodiment, the crystal form I has an endothermic peak at 173±2°C in differential scanning calorimetry analysis.

In another embodiment, the crystal form I has a weight loss of about 5.1% prior to 200°C in thermogravimetric analysis.

### The crystal form II of compound A

In one embodiment, the present disclosure provides the crystal form II of compound A, which is an acetonitrile solvate, wherein the molar ratio of acetonitrile to the free base is 1:5.

In another embodiment, the X-ray powder diffraction pattern of the crystal form II obtained using CuK_{α} radiation includes at least the characteristic peaks located at the following °2θ: 11.0±0.2, 11.6±0.2, 13.0±0.2, 17.1±0.2, 19.6±0.2, 19.8±0.2 and 22.9±0.2. In another embodiment, the X-ray powder diffraction pattern further includes the characteristic peaks located at the following °2θ: 5.8±0.2, 6.4±0.2, 10.8±0.2, 14.9±0.2, 15.3±0.2, 16.4±0.2, 19.3±0.2, 20.6±0.2, 23.3±0.2, 25.1±0.2 and 26.9±0.2.

In another embodiment, the X-ray powder diffraction pattern has the following characteristic peaks:

| Angle °2θ±0.2 °2θ | Relative intensity % |
|---|---|
| 11.0 | 90.1 |
| 11.6 | 60.1 |
| 13.0 | 52.5 |
| 17.1 | 100 |
| 19.6 | 54.4 |
| 19.8 | 50.6 |
| 22.9 | 53.7 |

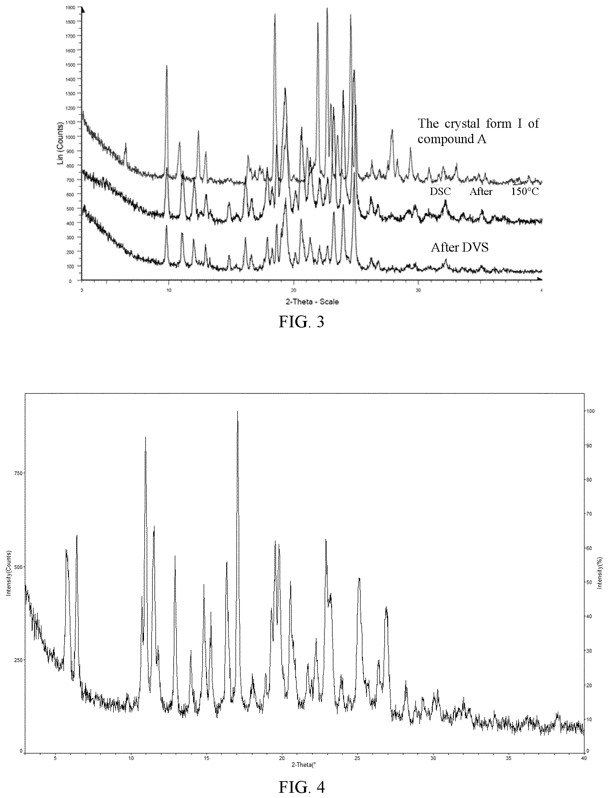

In another embodiment, the X-ray powder diffraction pattern comprises one or more peaks at the 20 value in Table 3.2. In another embodiment, the X-ray powder diffraction pattern is substantially as shown in FIG. 4.

In another embodiment, the crystal form II has an endothermic peak at 115±2°C in differential scanning calorimetry analysis.

In another embodiment, the crystal form II has a weight loss of about 1.9% prior to 200°C in thermogravimetric analysis.

### The crystal form III of compound A

In one embodiment, the present disclosure provides the crystal form III of compound A, which is a hydrate wherein the molar ratio of water to the free base is 1:1.

In another embodiment, the X-ray powder diffraction pattern of the crystal form III obtained using CuK_{α} radiation includes at least the characteristic peaks located at the following °2θ: 12.4±0.2, 14.2±0.2, 20.0±0.2 and 21.9±0.2. In another embodiment, the X-ray powder diffraction pattern further includes the characteristic peaks located at the following °2θ: 7.0±0.2, 9.3±0.2, 13.9±0.2 and 24.5±0.2. In another embodiment, the X-ray powder diffraction pattern further includes the characteristic peaks located at the following °2θ: 15.3±0.2, 20.6±0.2, 23.4±0.2, 27.5±0.2, 28.3±0.2 and 28.8±0.2.

In another embodiment, the X-ray powder diffraction pattern has the following characteristic peaks:

| Angle °2θ±0.2 °2θ | Relative intensity % |
|---|---|
| 7.0 | 27.5 |
| 9.3 | 39.6 |
| 12.4 | 94.4 |
| 13.9 | 27.3 |
| 14.2 | 91.4 |
| 20.0 | 100 |
| 21.9 | 65.9 |
| 24.5 | 41.9 |

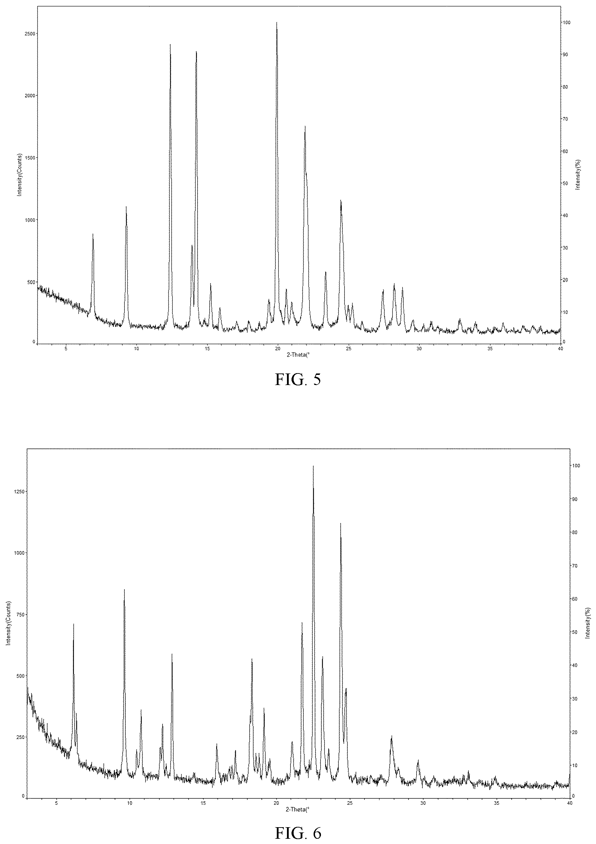

In another embodiment, the X-ray powder diffraction pattern comprises one or more peaks at the 20 value in Table 3.3. In another embodiment, the X-ray powder diffraction pattern is substantially as shown in FIG. 5.

In another embodiment, the crystal form III has an endothermic peak at 173±2°C in differential scanning calorimetry analysis.

In another embodiment, the crystal form III has a weight loss of about 3.5% prior to 200°C in thermogravimetric analysis.

### The crystal form IV of compound A

In one embodiment, the present disclosure provides the crystal form IV of compound A, which is a methanol solvate, wherein the molar ratio of methanol to the free base is 1:2.

In another embodiment, the X-ray powder diffraction pattern of the crystal form IV obtained using CuK_{α} radiation includes at least the characteristic peaks located at the following °2θ: 9.7±0.2, 22.5±0.2 and 24.4±0.2. In another embodiment, the X-ray powder diffraction pattern further includes the characteristic peaks located at the following °2θ: 6.2±0.2, 12.9±0.2, 18.4±0.2, 21.8±0.2, 23.2±0.2 and 24.8±0.2. In another embodiment, the X-ray powder diffraction pattern further includes the characteristic peaks located at the following °2θ: 6.4±0.2, 10.8±0.2, 12.3±0.2, 16.0±0.2, 19.2±0.2, 21.1±0.2 and 27.9±0.2.

In another embodiment, the X-ray powder diffraction pattern has the following characteristic peaks:

| Angle °2θ±0.2 °2θ | Relative intensity % |
|---|---|
| 6.2 | 43.4 |
| 9.7 | 59.9 |
| 12.9 | 40.3 |
| 18.4 | 38.3 |
| 21.8 | 48.2 |
| 22.5 | 100 |
| 23.2 | 38.8 |
| 24.4 | 82.9 |
| 24.8 | 29.7 |

In another embodiment, the X-ray powder diffraction pattern comprises one or more peaks at the 20 value in Table 3.4. In another embodiment, the X-ray powder diffraction pattern is substantially as shown in FIG. 6.

In another embodiment, the crystal form IV has endothermic peaks at 176±2°C and 251±2°C in differential scanning calorimetry analysis.

In another embodiment, the crystal form IV has a weight loss of about 3.7% prior to 200°C in thermogravimetric analysis.

In another embodiment, the crystal form IV has the following unit cell parameters:

| Space group | C2 |
|---|---|
| *a*/Å | 31.9592(11) |
| *b*/Å | 9.58750(10) |
| *c*/Å | 31.2990(8) |
| *α*/° | 90 |
| *β*/° | 116.850(3) |
| *γ*/° | 90 |
| Volume/Å³ | 8556.4(4) |

### The crystal form V of compound A

In one embodiment, the present disclosure provides the crystal form V of compound A, which is an isobutanol solvate, wherein the molar ratio of isobutanol to the free base is 1:6.

In another embodiment, the X-ray powder diffraction pattern of the crystal form V obtained using CuK_{α} radiation includes at least the characteristic peaks located at the following °2θ: 9.4±0.2, 18.1±0.2, 18.9±0.2, 21.3±0.2 and 23.6±0.2. In another embodiment, the X-ray powder diffraction pattern further includes the characteristic peaks located at the following °2θ: 24.3±0.2 and 26.8±0.2. In another embodiment, the X-ray powder diffraction pattern further includes the characteristic peaks located at the following °2θ: 9.9±0.2, 11.8±0.2, 15.6±0.2, 15.9±0.2, 17.6±0.2, 18.4±0.2, 19.1±0.2, 19.8±0.2, 21.8±0.2 and 23.2±0.2.

In another embodiment, the X-ray powder diffraction pattern has the following characteristic peaks:

| Angle °2θ±0.2 °2θ | Relative intensity % |
|---|---|
| 9.4 | 81.6 |
| 18.1 | 90.8 |
| 18.9 | 51.7 |
| 21.3 | 61.6 |
| 23.6 | 100 |
| 24.3 | 45.7 |
| 26.8 | 34.3 |

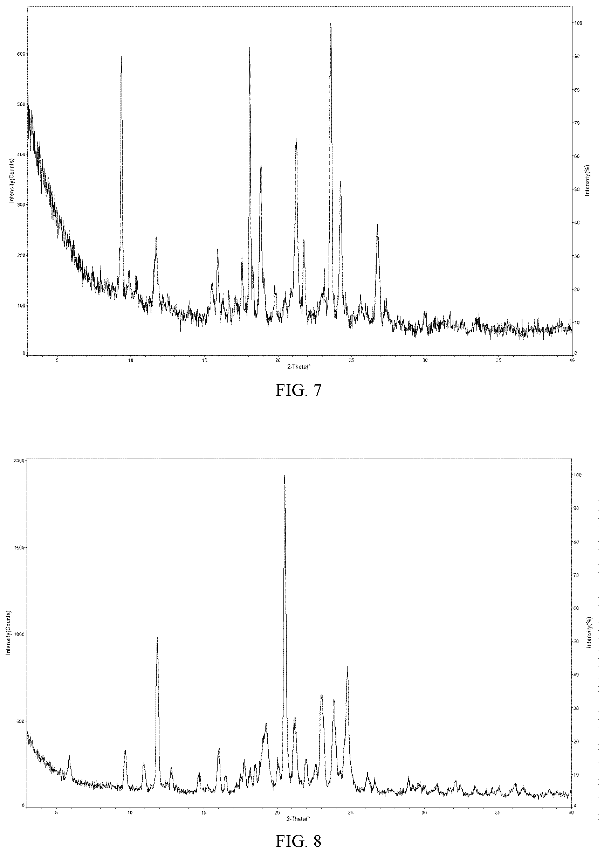

In another embodiment, the X-ray powder diffraction pattern comprises one or more peaks at the 2θ value in Table 3.5. In another embodiment, the X-ray powder diffraction pattern is substantially as shown in FIG. 7.

In another embodiment, the crystal form V has an endothermic peak at 173±2°C in differential scanning calorimetry analysis.

In another embodiment, the crystal form V has a weight loss of about 6.5% prior to 200°C in thermogravimetric analysis.

### The crystal form VI of compound A

In one embodiment, the present disclosure provides the crystal form VI of compound A, which is an anhydrate.

In another embodiment, the X-ray powder diffraction pattern of the crystal form VI obtained using CuK_{α} radiation includes at least the characteristic peaks located at the following °2θ: 11.9±0.2, 20.5±0.2, 23.1±0.2, 23.9±0.2 and 24.8±0.2. In another embodiment, the X-ray powder diffraction pattern further includes the characteristic peaks located at the following °2θ: 9.7±0.2, 16.1±0.2, 19.3±0.2 and 21.2±0.2. In another embodiment, the X-ray powder diffraction pattern further includes the characteristic peaks located at the following °2θ: 5.9±0.2, 11.0±0.2, 12.8±0.2, 14.7±0.2, 16.6±0.2, 17.8±0.2, 18.2±0.2, 18.6±0.2, 20.1±0.2, 22.0±0.2, 22.6±0.2, 26.2±0.2 and 29.0±0.2.

In another embodiment, the X-ray powder diffraction pattern has the following characteristic peaks:

| Angle °2θ±0.2 °2θ | Relative intensity % |
|---|---|
| 9.7 | 12.3 |
| 11.9 | 48.7 |
| 16.1 | 14 |
| 19.3 | 20 |
| 20.5 | 100 |
| 21.2 | 20.3 |
| 23.1 | 29.3 |
| 23.9 | 26.3 |
| 24.8 | 36.8 |

In another embodiment, the X-ray powder diffraction pattern comprises one or more peaks at the 20 value in Table 3.6. In another embodiment, the X-ray powder diffraction pattern is substantially as shown in FIG. 8.

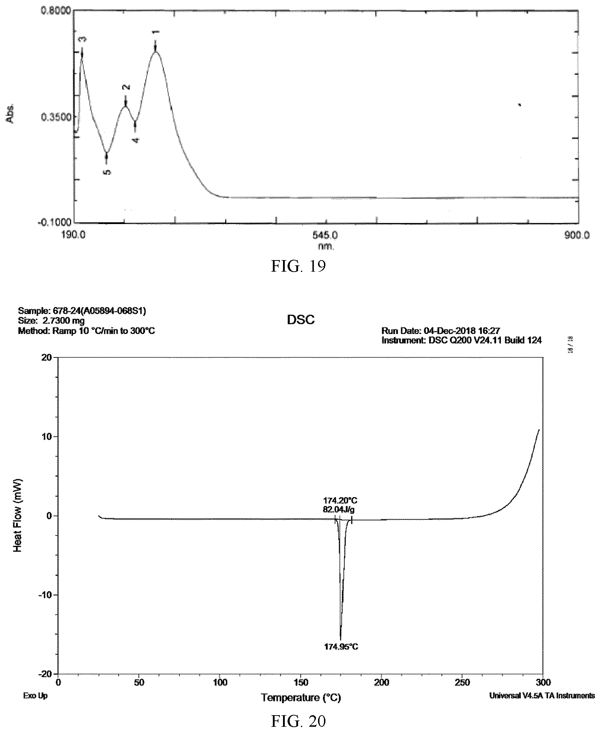

In another embodiment, the crystal form VI has an endothermic peak at 175±2°C in differential scanning calorimetry analysis. In another embodiment, the crystal form VI has a DSC curve substantially as shown in FIG. 20.

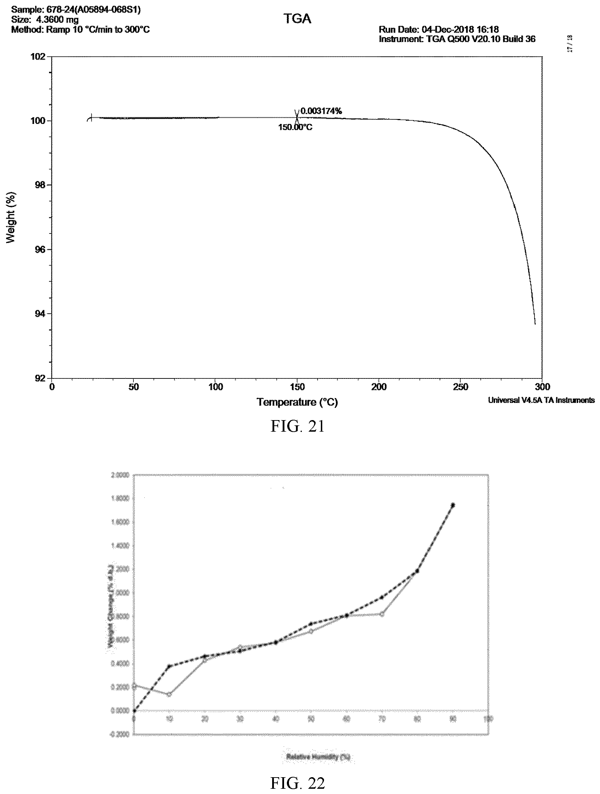

In another embodiment, the crystal form VI exhibits substantially no weight loss prior to 200°C in thermogravimetric analysis. In another embodiment, the crystal form VI has a TGA curve substantially as shown in FIG. 21.

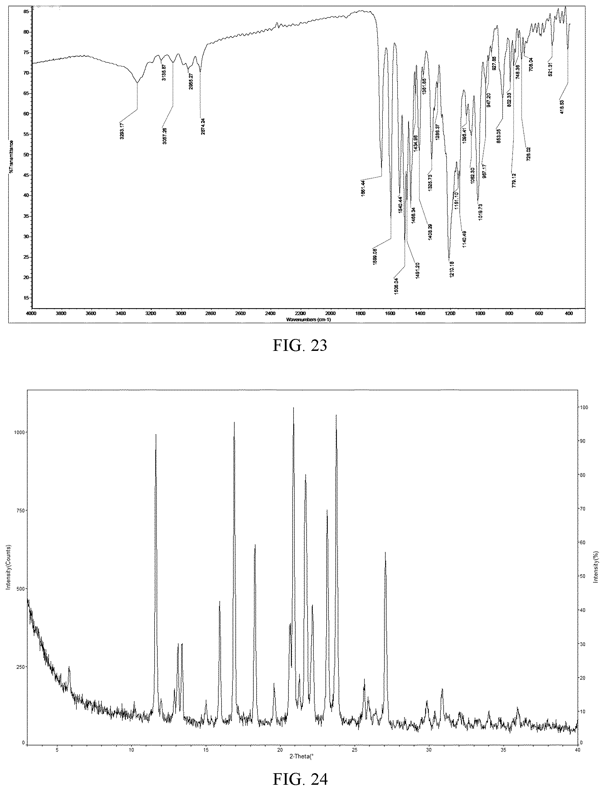

In another embodiment, the crystal form VI has absorption peaks in the infrared absorption spectrum at the following cm⁻¹: 853±2, 1020±2, 1062±2, 1210±2, 1408±2, 1466±2, 1491±2, 1599±2, 1661±2, 3293±2. In another embodiment, the crystal form VI has an infrared absorption spectrum substantially as shown in FIG. 23.

In another embodiment, the crystal form VI has absorption peaks in the UV spectrum at the following nm: 201±2, 263±2 and 306±2. In another embodiment, the crystal form VI has a UV spectrum substantially as shown in FIG. 19.

### The crystal form VII of compound A

In one embodiment, the present disclosure provides the crystal form VII of compound A, which is a hydrate wherein the molar ratio of water to the free base is 1:1.

In another embodiment, the X-ray powder diffraction pattern of the crystal form VII obtained using CuK_{α} radiation includes at least the characteristic peaks located at the following °2θ: 11.7±0.2, 16.9±0.2, 18.3±0.2, 20.9±0.2, 21.7±0.2, 23.2±0.2, 23.8±0.2 and 27.1±0.2. In another embodiment, the X-ray powder diffraction pattern further includes the characteristic peaks located at the following °2θ: 13.4±0.2, 16.0±0.2, 20.7±0.2 and 22.2±0.2. In another embodiment, the X-ray powder diffraction pattern further includes the characteristic peaks located at the following °2θ: 13.2±0.2, 19.6±0.2, 21.3±0.2, 25.7±0.2 and 30.9±0.2.

In another embodiment, the X-ray powder diffraction pattern has the following characteristic peaks:

| Angle °2θ±0.2 °2θ | Relative intensity % |
|---|---|
| 11.7 | 92.9 |
| 13.4 | 25.2 |
| 16.0 | 39.8 |
| 16.9 | 97.3 |
| 18.3 | 58 |
| 20.7 | 29.4 |
| 20.9 | 99.3 |
| 21.7 | 76.5 |
| 22.2 | 35.4 |
| 23.2 | 68.5 |
| 23.8 | 100 |
| 27.1 | 56.3 |

In another embodiment, the X-ray powder diffraction pattern comprises one or more peaks at the 20 value in Table 3.7. In another embodiment, the X-ray powder diffraction pattern is substantially as shown in FIG. 24.

In another embodiment, the crystal form VII has an endothermic peak at 174±2°C in differential scanning calorimetry analysis.

In another embodiment, the crystal form VII has a weight loss of about 3.4% prior to 200°C in thermogravimetric analysis.

### The crystal form VIII of compound A

In one embodiment, the present disclosure provides the crystal form VIII of compound A, which is an ethanol solvate, wherein the molar ratio of ethanol to the free base is 2:5.

In another embodiment, the X-ray powder diffraction pattern of the crystal form VIII obtained using CuK_{α} radiation includes at least the characteristic peaks located at the following °2θ: 9.6±0.2, 12.7±0.2, 18.3±0.2, 19.1±0.2, 22.8±0.2 and 24.3±0.2. In another embodiment, the X-ray powder diffraction pattern further includes the characteristic peaks located at the following °2θ: 10.7±0.2, 12.1±0.2, 19.4±0.2, 21.8±0.2, 22.5±0.2, 24.7±0.2 and 27.6±0.2.

In another embodiment, the X-ray powder diffraction pattern has the following characteristic peaks:

| Angle °2θ±0.2 °2θ | Relative intensity % |
|---|---|
| 9.6 | 100 |
| 12.7 | 19.2 |
| 18.3 | 82.8 |
| 19.1 | 12.6 |
| 22.8 | 13.6 |
| 24.3 | 22.7 |

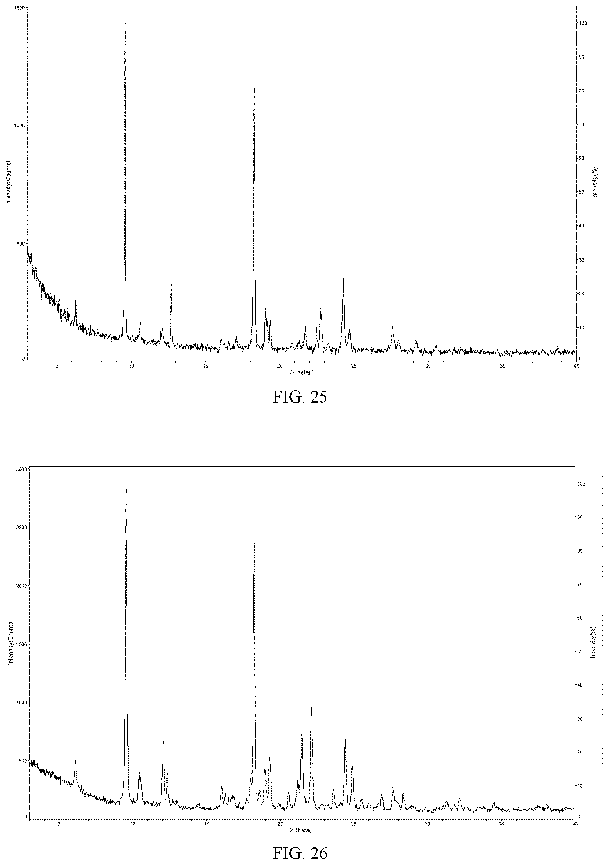

In another embodiment, the X-ray powder diffraction pattern comprises one or more peaks at the 20 value in Table 3.8. In another embodiment, the X-ray powder diffraction pattern is substantially as shown in FIG. 25.

### The crystal form IX of compound A

In one embodiment, the present disclosure provides the crystal form IX of compound A, which is a solvate of ethanol, isopropanol and tetrahydrofuran, wherein the molar ratio of ethanol to the free base is 1:2, the molar ratio of isopropanol to the free base is 1:3, and the molar ratio of tetrahydrofuran to the free base is 0.06: 1.

In another embodiment, the X-ray powder diffraction pattern of the crystal form IX obtained using CuK_{α} radiation includes at least the characteristic peaks located at the following °2θ: 9.6±0.2, 18.2±0.2 and 22.1±0.2. In another embodiment, the X-ray powder diffraction pattern further includes the characteristic peaks located at the following °2θ: 6.1±0.2, 12.1±0.2, 12.4±0.2, 19.0±0.2, 19.3±0.2, 21.5±0.2, 24.4±0.2 and 24.9±0.2. In another embodiment, the X-ray powder diffraction pattern further includes the characteristic peaks located at the following °2θ: 10.5±0.2, 16.1±0.2, 20.6±0.2, 21.2±0.2, 23.6±0.2, 26.9±0.2, 27.7±0.2 and 28.4±0.2.

In another embodiment, the X-ray powder diffraction pattern has the following characteristic peaks:

| Angle °2θ±0.2 °2θ | Relative intensity % |
|---|---|
| 6.1 | 10 |
| 9.6 | 100 |
| 12.1 | 20 |
| 12.4 | 10.3 |
| 18.2 | 85.5 |
| 19.0 | 11.1 |
| 19.3 | 16.3 |
| 21.5 | 23.5 |
| 22.1 | 30 |
| 24.4 | 21.5 |
| 24.9 | 13.2 |

In another embodiment, the X-ray powder diffraction pattern comprises one or more peaks at the 20 value in Table 3.9. In another embodiment, the X-ray powder diffraction pattern is substantially as shown in FIG. 26.

In another embodiment, the crystal form IX has an endothermic peak at 174±2°C in differential scanning calorimetry analysis.

In another embodiment, the crystal form IX has a weight loss of about 5.8% prior to 200°C in thermogravimetric analysis.

### The crystal form X of compound A

In one embodiment, the present disclosure provides the crystal form X of compound A, which is a tetrahydrofuran solvate, wherein the molar ratio of tetrahydrofuran to the free base is 1:12.

In another embodiment, the X-ray powder diffraction pattern of the crystal form X obtained using CuK_{α} radiation includes at least the characteristic peaks located at the following °2θ: 7.0±0.2, 9.3±0.2, 12.3±0.2, 14.2±0.2, 16.3±0.2, 18.8±0.2, 19.9±0.2, 21.4±0.2 and 23.9±0.2. In another embodiment, the X-ray powder diffraction pattern further includes the characteristic peaks located at the following °2θ: 15.4±0.2, 19.2±0.2, 19.5±0.2, 22.2±0.2, 23.4±0.2, 24.8±0.2, 25.8±0.2, 26.2±0.2, 26.7±0.2, 28.8±0.2 and 29.2±0.2.

In another embodiment, the X-ray powder diffraction pattern has the following characteristic peaks:

| Angle °2θ±0.2 °2θ | Relative intensity % |
|---|---|
| 7.0 | 70.1 |
| 9.3 | 63.1 |
| 12.3 | 100 |
| 14.2 | 80.4 |
| 16.3 | 54.8 |
| 18.8 | 60.4 |
| 19.9 | 93.8 |
| 21.4 | 63.3 |
| 23.9 | 80.1 |

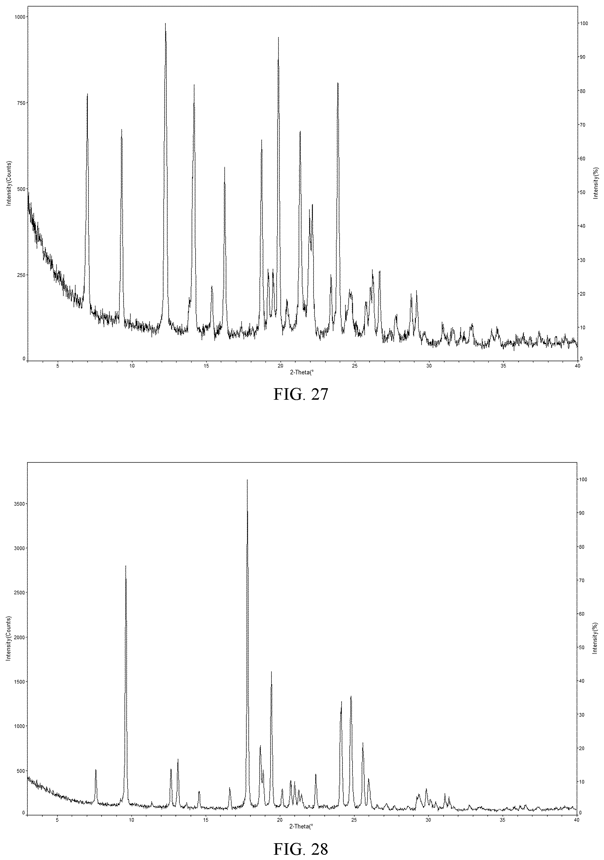

In another embodiment, the X-ray powder diffraction pattern comprises one or more peaks at the 2θ value in Table 3.10. In another embodiment, the X-ray powder diffraction pattern is substantially as shown in FIG. 27.

In another embodiment, the crystal form X has an endothermic peak at 173±2°C in differential scanning calorimetry analysis.

In another embodiment, the crystal form X has a weight loss of about 3.6% prior to 200°C in thermogravimetric analysis.

### The crystal form XI of compound A

In one embodiment, the present disclosure provides the crystal form XI of compound A, which is an acetonitrile solvate, wherein the molar ratio of acetonitrile to the free base is 2:5.

In another embodiment, the X-ray powder diffraction pattern of the crystal form XI obtained using CuK_{α} radiation includes at least the characteristic peaks located at the following °2θ: 9.7±0.2, 17.9±0.2, 19.5±0.2, 24.2±0.2 and 24.8±0.2. In another embodiment, the X-ray powder diffraction pattern further includes the characteristic peaks located at the following °2θ: 7.6±0.2, 12.7±0.2, 13.2±0.2, 18.7±0.2, 18.9±0.2, 22.4±0.2 and 25.6±0.2. In another embodiment, the X-ray powder diffraction pattern further includes the characteristic peaks located at the following °2θ: 14.6±0.2, 16.7±0.2, 20.2±0.2, 20.7±0.2, 21.0±0.2, 21.3±0.2, 26.0±0.2 and 29.9±0.2.

In another embodiment, the X-ray powder diffraction pattern has the following characteristic peaks:

| Angle °2θ±0.2 °2θ | Relative intensity % |
|---|---|
| 7.6 | 10.4 |
| 9.7 | 73.3 |
| 12.7 | 11.7 |
| 13.2 | 14.7 |
| 17.9 | 100 |
| 18.7 | 18.4 |
| 18.9 | 11 |
| 19.5 | 41.4 |
| 22.4 | 10.5 |
| 24.2 | 32.4 |
| 24.8 | 33.8 |
| 25.6 | 19.8 |

In another embodiment, the X-ray powder diffraction pattern comprises one or more peaks at the 2θ value in Table 3.11. In another embodiment, the X-ray powder diffraction pattern is substantially as shown in FIG. 28.

In another embodiment, the crystal form XI has an endothermic peak at 174±2°C in differential scanning calorimetry analysis.

In another embodiment, the crystal form XI has a weight loss of about 3.9% prior to 200°C in thermogravimetric analysis.

### Salts of compound A and crystal forms thereof

The present disclosure relates to various salts of compound A, such as hydrochloride, benzenesulfonate, p-toluenesulfonate, mesylate, and hydrobromide.

The present disclosure also relates to crystalline forms of various salts of compound A, such as "the crystal form I of compound A hydrochloride", "the crystal form I of compound Abenzenesulfonate", "the crystal form I of compound A p-toluenesulfonate", "the crystal form II of compound A p-toluenesulfonate", "the crystal form I of compound A mesylate", and "the crystal form I of compound A hydrobromide".

### The crystal form I of compound A hydrochloride

In one embodiment, the present disclosure provides the crystal form I of compound A hydrochloride. In another embodiment, the crystal form I of compound A hydrochloride is an anhydrate.

In another embodiment, the X-ray powder diffraction pattern of the crystal form I of the hydrochloride obtained using CuK_{α} radiation includes at least the characteristic peaks located at the following °2θ: 6.5±0.2, 13.8±0.2, 18.7±0.2 and 23.3±0.2. In another embodiment, the X-ray powder diffraction pattern further includes the characteristic peaks located at the following °2θ: 11.3±0.2, 15.7±0.2, 16.4±0.2, 21.9±0.2, 22.7±0.2, 24.2±0.2 and 28.6±0.2. In another embodiment, the X-ray powder diffraction pattern further includes the characteristic peaks located at the following °2θ: 16.8±0.2, 19.7±0.2, 20.8±0.2, 21.3±0.2, 25.2±0.2 and 35.0±0.2.

In another embodiment, the X-ray powder diffraction pattern has the following characteristic peaks:

| Angle °2θ±0.2 °2θ | Relative intensity % |
|---|---|
| 6.5 | 86.2 |
| 11.3 | 25.4 |
| 13.8 | 90.2 |
| 15.7 | 29.8 |
| 16.4 | 32.7 |
| 18.7 | 100 |
| 21.9 | 31.6 |
| 22.7 | 33.5 |
| 23.3 | 57.7 |
| 24.2 | 37.9 |
| 28.6 | 32.7 |

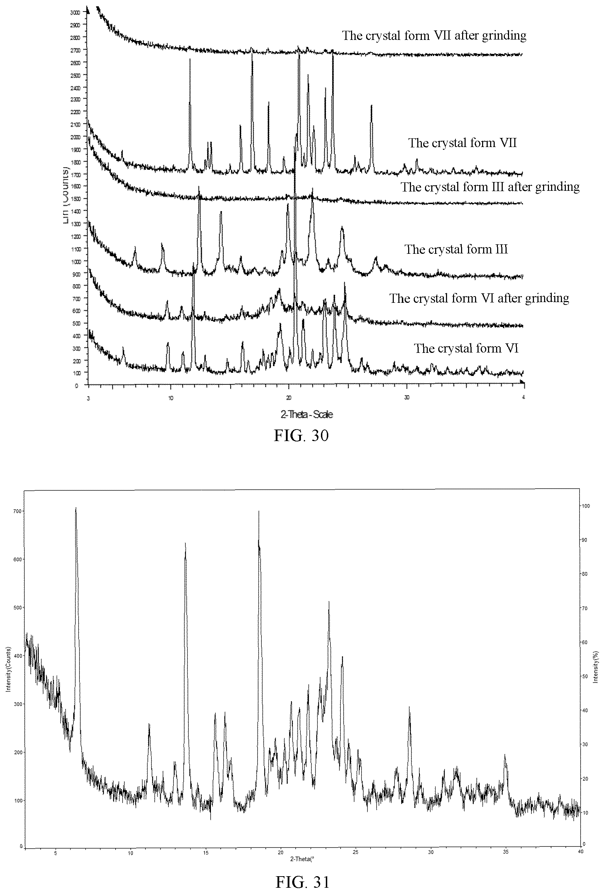

In another embodiment, the X-ray powder diffraction pattern comprises one or more peaks at the 2θ value in Table 3.1. In another embodiment, the X-ray powder diffraction pattern is substantially as shown in FIG. 31.

In another embodiment, the crystal form I of the hydrochloride has an endothermic peak at 229±2°C in differential scanning calorimetry analysis.

In another embodiment, the crystal form I of the hydrochloride has a weight loss of about 0.7% at 175°C in thermogravimetric analysis.

### The crystal form I of compound A benzenesulfonate

In one embodiment, the present disclosure provides the crystal form I of compound A benzenesulfonate. In another embodiment, the crystal form I of the benzenesulfonate is an anhydrate.

In another embodiment, the X-ray powder diffraction pattern of the crystal form I of the benzenesulfonate obtained using CuK_{α} radiation includes at least the characteristic peaks located at the following °2θ: 7.6±0.2, 10.2±0.2 and 22.8±0.2. In another embodiment, the X-ray powder diffraction pattern further includes the characteristic peaks located at the following °2θ: 16.4±0.2, 19.1±0.2, 19.9±0.2, 21.3±0.2, 21.9±0.2 and 23.3±0.2. In another embodiment, the X-ray powder diffraction pattern further includes the characteristic peaks located at the following °2θ: 20.5±0.2, 21.6±0.2 and 25.0±0.2.

In another embodiment, the X-ray powder diffraction pattern has the following characteristic peaks:

| Angle °2θ±0.2 °2θ | Relative intensity % |
|---|---|
| 7.6 | 50.1 |
| 10.2 | 100 |
| 16.4 | 46.3 |
| 19.1 | 27.7 |
| 19.9 | 26.9 |
| 21.3 | 25.9 |
| 21.9 | 25.6 |
| 22.8 | 69.2 |
| 23.3 | 43.1 |

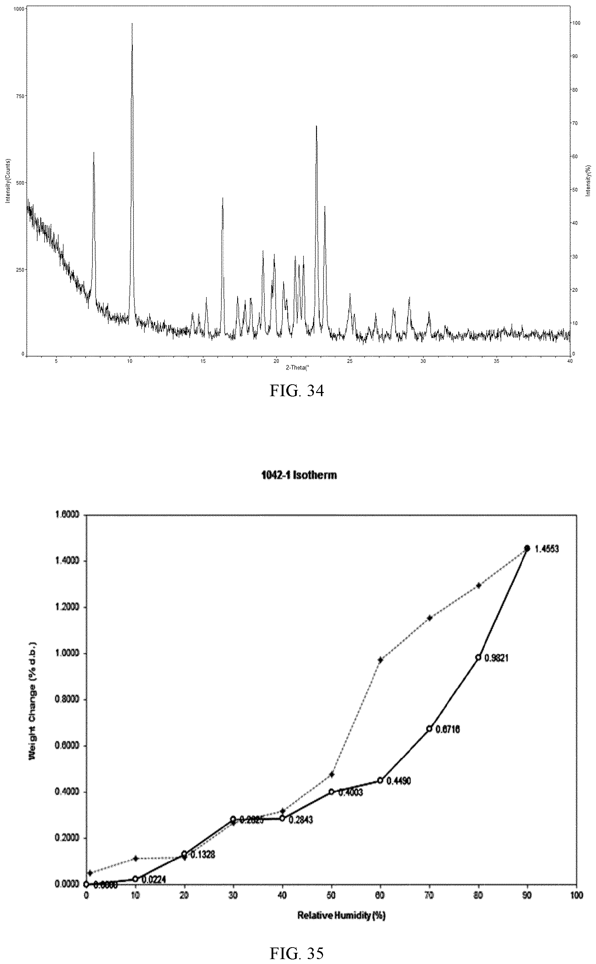

In another embodiment, the X-ray powder diffraction pattern comprises one or more peaks at the 20 value in Table 3.2. In another embodiment, the X-ray powder diffraction pattern is substantially as shown in FIG. 34.

In another embodiment, the crystal form I of the benzenesulfonate has an endothermic peak at 253±2°C in differential scanning calorimetry analysis.

In another embodiment, the crystal form I of the benzenesulfonate has a weight loss of about 0.5% at 200°C in thermogravimetric analysis.

### The crystal form I of compound A p-toluenesulfonate

In one embodiment, the present disclosure provides the crystal form I of compound A p-toluenesulfonate. In another embodiment, the crystal form I of the p-toluenesulfonate is an anhydrate.

In another embodiment, the X-ray powder diffraction pattern of the crystal form I of the p-toluenesulfonate obtained using CuK_{α} radiation includes at least the characteristic peaks located at the following °2θ: 7.4±0.2, 10.2±0.2, 21.3±0.2 and 21.9±0.2. In another embodiment, the X-ray powder diffraction pattern further includes the characteristic peaks located at the following °2θ: 27.9±0.2. In another embodiment, the X-ray powder diffraction pattern further includes the characteristic peaks located at the following °2θ: 14.9±0.2, 16.3±0.2, 19.2±0.2 and 23.0±0.2.

In another embodiment, the X-ray powder diffraction pattern has the following characteristic peaks:

| Angle °2θ±0.2 °2θ | Relative intensity % |
|---|---|
| 7.4 | 63.7 |
| 10.2 | 100 |
| 21.3 | 54.5 |
| 21.9 | 92.2 |
| 27.9 | 34.3 |

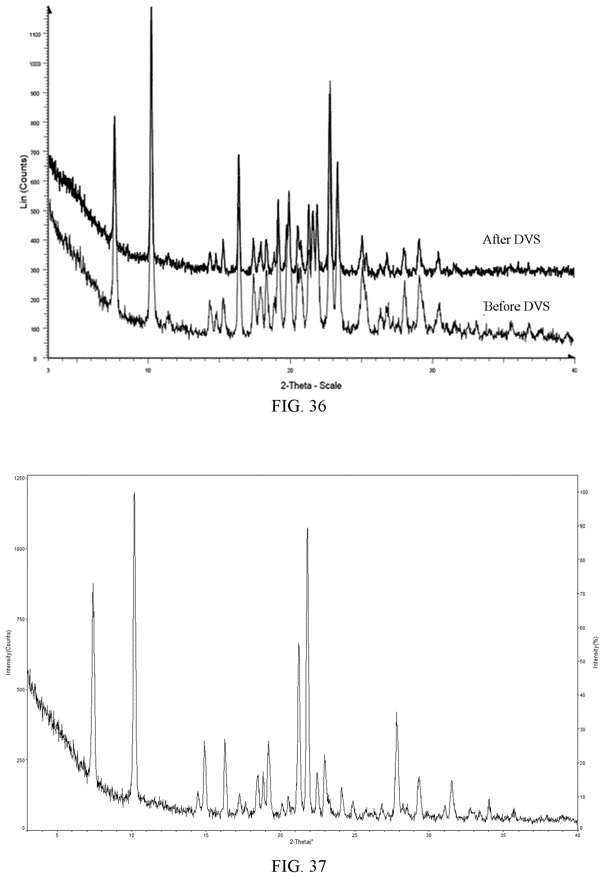

In another embodiment, the X-ray powder diffraction pattern comprises one or more peaks at the 2θ value in Table 3.3. In another embodiment, the X-ray powder diffraction pattern is substantially as shown in FIG. 37.

In another embodiment, the crystal form I of the p-toluenesulfonate has an endothermic peak at 236±2°C in differential scanning calorimetry analysis.

In another embodiment, the crystal form I of the p-toluenesulfonate has a weight loss of about 0.1% at 200°C in thermogravimetric analysis.

### The crystal form II of compound A p-toluenesulfonate

In one embodiment, the present disclosure provides the crystal form II of compound A p-toluenesulfonate. In another embodiment, the crystal form II of the p-toluenesulfonate is an anhydrate.

In another embodiment, the X-ray powder diffraction pattern of the crystal form II of the p-toluenesulfonate obtained using CuK_{α} radiation includes at least the characteristic peaks located at the following °2θ: 6.4±0.2, 7.4±0.2, 10.2±0.2, 16.3±0.2, 21.3±0.2 and 21.8±0.2.

In another embodiment, the X-ray powder diffraction pattern has the following characteristic peaks:

| Angle °2θ±0.2 °2θ | Relative intensity % |
|---|---|
| 6.4 | 100 |
| 7.4 | 10.1 |
| 10.2 | 25.6 |
| 16.3 | 5.6 |
| 21.3 | 12.1 |
| 21.8 | 5.2 |

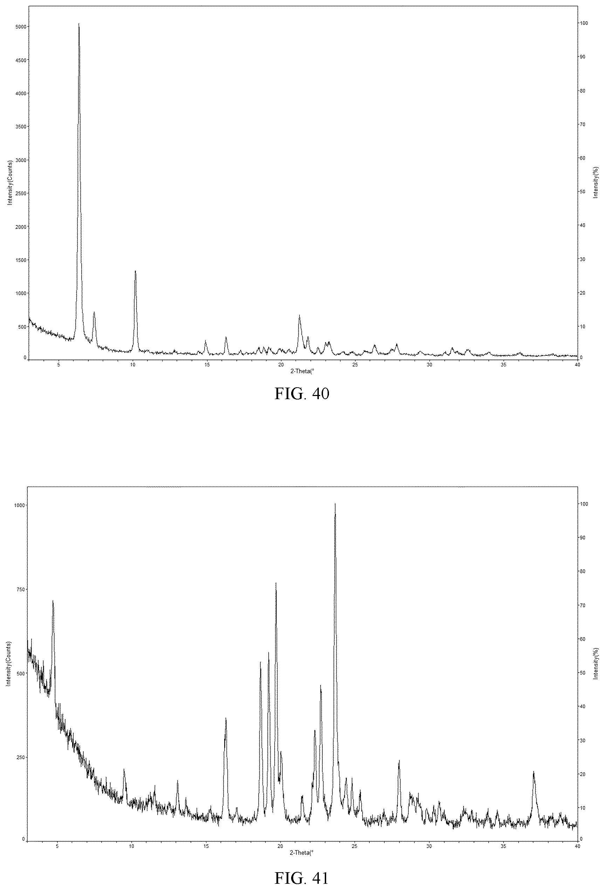

In another embodiment, the X-ray powder diffraction pattern comprises one or more peaks at the 20 value in Table 3.4. In another embodiment, the X-ray powder diffraction pattern is substantially as shown in FIG. 40.

In another embodiment, the crystal form II of the p-toluenesulfonate has an endothermic peak at 234±2°C in differential scanning calorimetry analysis.

In another embodiment, the crystal form II of the p-toluenesulfonate has a weight loss of about 0.2% at 200°C in thermogravimetric analysis.

### The crystal form I of compound A mesylate

In one embodiment, the present disclosure provides the crystal form I of compound A mesylate. In another embodiment, the crystal form I of the mesylate is an anhydrate.

In another embodiment, the X-ray powder diffraction pattern of the crystal form I of the mesylate obtained using CuK_{α} radiation includes at least the characteristic peaks located at the following °2θ: 19.7±0.2 and 23.7±0.2. In another embodiment, the X-ray powder diffraction pattern further includes the characteristic peaks located at the following °2θ: 4.7±0.2, 16.4±0.2, 18.7±0.2, 19.2±0.2, 22.3±0.2 and 22.7±0.2. In another embodiment, the X-ray powder diffraction pattern further includes the characteristic peaks located at the following °2θ: 20.1±0.2, 28.1±0.2 and 37.1±0.2.

In another embodiment, the X-ray powder diffraction pattern has the following characteristic peaks:

| Angle °2θ±0.2 °2θ | Relative intensity % |
|---|---|
| 4.7 | 32.6 |
| 16.4 | 32.5 |
| 18.7 | 48.5 |
| 19.2 | 49.8 |
| 19.7 | 74.8 |
| 22.3 | 28.1 |
| 22.7 | 42.3 |
| 23.7 | 100 |

In another embodiment, the X-ray powder diffraction pattern comprises one or more peaks at the 20 value in Table 3.5. In another embodiment, the X-ray powder diffraction pattern is substantially as shown in FIG. 41.

In another embodiment, the crystal form I of the mesylate has an endothermic peak at 206±2°C in differential scanning calorimetry analysis.

In another embodiment, the crystal form I of the mesylate has a weight loss of about 0.7% at 200°C in thermogravimetric analysis.

### The crystal form I of compound A hydrobromide

In one embodiment, the present disclosure provides the crystal form I of compound A hydrobromide. In another embodiment, the crystal form I of the hydrobromide is an anhydrate.

In another embodiment, the X-ray powder diffraction pattern of the crystal form I of the hydrobromide obtained using CuK_{α} radiation includes at least the characteristic peaks located at the following °2θ: 6.4±0.2, 13.5±0.2, 20.6±0.2, 21.1±0.2, 23.8±0.2, 24.0±0.2 and 26.1±0.2. In another embodiment, the X-ray powder diffraction pattern further includes the characteristic peaks located at the following °2θ: 13.0±0.2 and 28.5±0.2.

In another embodiment, the X-ray powder diffraction pattern has the following characteristic peaks:

| Angle °2θ±0.2 °2θ | Relative intensity % |
|---|---|
| 6.4 | 100 |
| 13.5 | 30.7 |
| 20.6 | 34.9 |
| 21.1 | 25.5 |
| 23.8 | 31.1 |
| 24.0 | 40.2 |
| 26.1 | 31.2 |

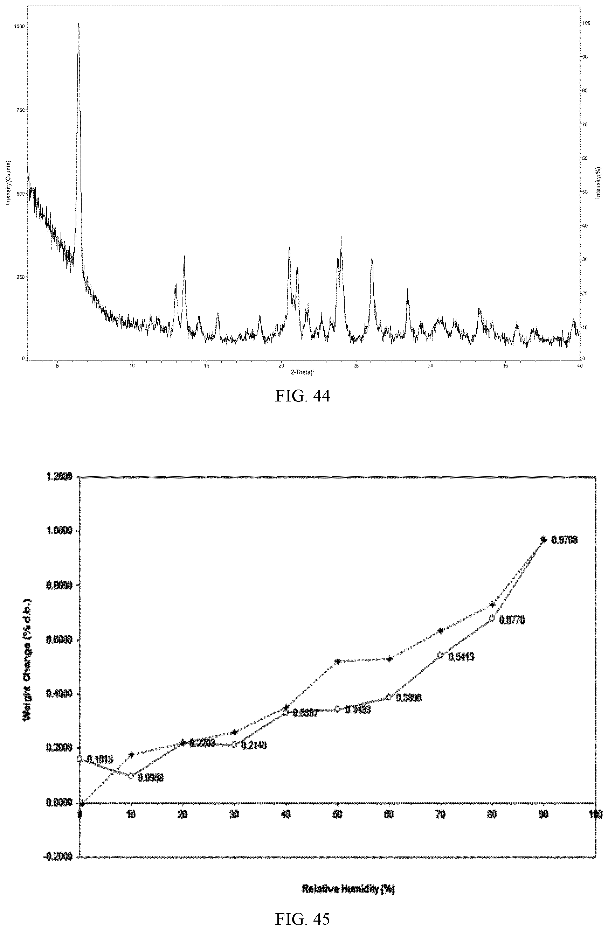

In another embodiment, the X-ray powder diffraction pattern comprises one or more peaks at the 2θ value in Table 3.6. In another embodiment, the X-ray powder diffraction pattern is substantially as shown in FIG. 44.

In another embodiment, the crystal form I of the hydrobromide has an endothermic peak at 251±2°C in differential scanning calorimetry analysis.

In another embodiment, the crystal form I of the hydrobromide has a weight loss of about 0.6% at 200°C in thermogravimetric analysis.

### Method for preparing compound A and the crystal form VI thereof

The present disclosure relates to a method for the preparation of kilogram-scale high-purity compound A and the crystal form VI thereof, see Scheme 1.

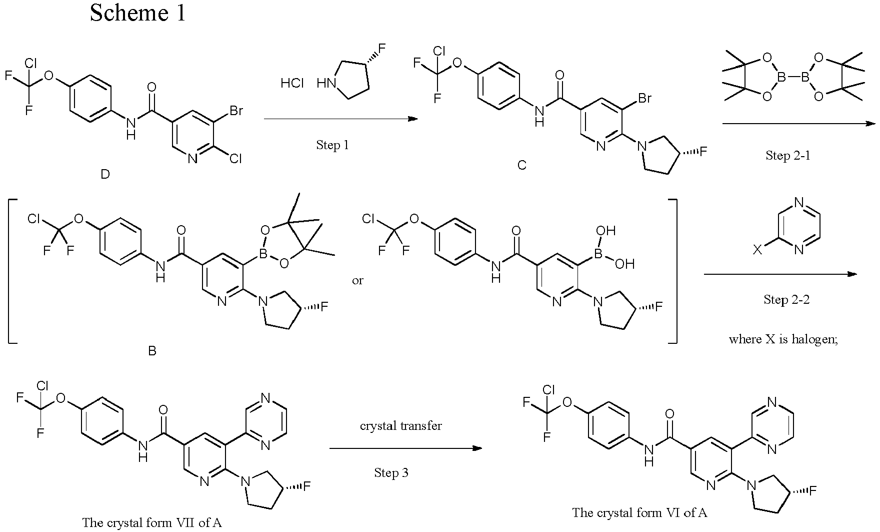

Step 1: Substitution reaction of compound D with 3-(R)-fluoropyrrolidine hydrochloride is carried out in the presence of a base, alternatively inorganic base. Step 2: Compound C is reacted with bis(pinacolato)diboron in the presence of a specific palladium catalyst and an acetate to form intermediate compound B or its boronic acid hydrolysis product or a mixture thereof, which is then reacted with 2-halopyrazine in the presence of a palladium catalyst and a base to form compound A. Alternatively, the product is recrystallized to obtain the crystal form VII of compound A. Step 3: The crystal form VII of compound A is converted into the crystal form VI of compound A.

### Step 1

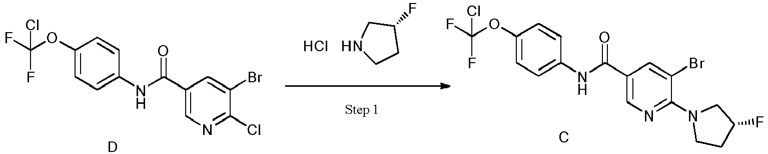

In Step 1, compound D is reacted with 3-(R)-fluoropyrrolidine hydrochloride in the presence of a base to generate compound C.

In a specific embodiment, the reaction is carried out in an aprotic solvent in the presence of a base. In another specific embodiment, the base is an inorganic base, alternatively sodium hydroxide, potassium hydroxide, lithium hydroxide, cesium hydroxide, sodium carbonate, potassium carbonate, cesium carbonate, sodium bicarbonate or potassium bicarbonate; alternatively, sodium carbonate, potassium carbonate or cesium carbonate; alternatively, sodium carbonate.

In another specific embodiment, the aprotic solvent is selected from DCM, DCE, ethyl acetate, methyl acetate, isopropyl acetate, n-hexane, n-heptane, petroleum ether, acetone, acetonitrile, toluene, methyl tert-butyl ether, tetrahydrofuran, 2-methyltetrahydrofuran, ethylene glycol monomethyl ether, ethylene glycol dimethyl ether, DMF, DMA or DMSO; alternatively, acetonitrile, tetrahydrofuran, 2-methyltetrahydrofuran, DMF or DMSO; alternatively, DMSO.

In another specific embodiment, the reaction is carried out at a temperature of 50°C to the temperature of solvent reflux, alternatively, 50-90 °C, or still alternatively, 70±10°C for at least 1 hour, alternatively, at least 20 hours.

In another specific embodiment, the compound D has a molar ratio of 0.8-1.2:1 with 3-(R)-fluoropyrrolidine hydrochloride, alternatively, 1:1.

In another specific embodiment, the amount of the base is 2-3 times that of the compound D, alternatively, 2.2 times.

### Step 2-1

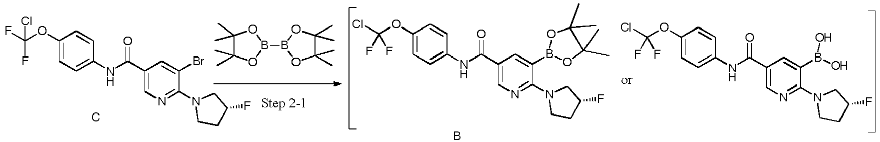

### Step 2-2

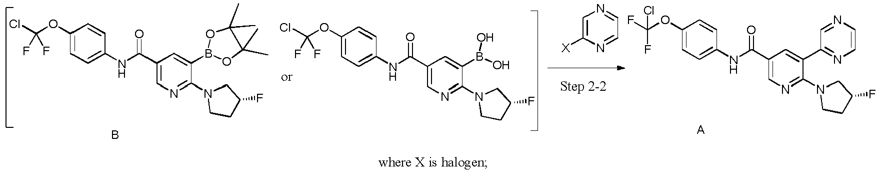

In Step 2, compound C is reacted with bis(pinacolato)diboron in the presence of a palladium catalyst Pd(dppf)Cl₂ and an acetate to form intermediate compound B and its boronic acid hydrolysis product or a mixture thereof (step 2-1), which is then reacted with 2-halopyrazine in the presence of a palladium catalyst and a base to form compound A (step 2-2).

In a specific embodiment of step 2-1, the reaction is as follows.

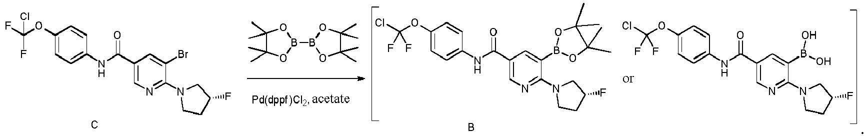

In another specific embodiment of step 2-1, the reaction comprises reacting a compound of formula (C) with bis(pinacolato)diboron in a solvent in the presence of a palladium catalyst Pd(dppf)Cl₂ and an acetate, wherein the solvent is selected from DMSO, DCM, DCE, ethyl acetate, methyl acetate, isopropyl acetate, acetone, acetonitrile, methyl tert-butyl ether, ethylene glycol monomethyl ether, ethylene glycol dimethyl ether, DMF or DMA; alternatively, DMSO, 2-methyltetrahydrofuran, ethylene glycol monomethyl ether or DMF; alternatively, DMSO.

In a specific embodiment of step 2-1, the acetate is selected from potassium acetate, sodium acetate, or cesium acetate; alternatively, potassium acetate.

In a specific embodiment of step 2-1, the amount of the acetate is 2-5 times that of compound C; alternatively, 2.5 times.

In a specific embodiment of step 2-1, the amount of the bis(pinacolato)diboron is 3-6 times that of compound C; alternatively, 3 times.

In a specific embodiment of step 2-1, the amount of the catalyst is 0.01-0.1 times that of compound C, alternatively, 0.05 times.

In a specific embodiment of step 2-1, the reaction is carried out at a temperature of 60°C to the temperature of solvent reflux, alternatively, 60-100°C, or still alternatively, 80±10°C; alternatively, for a reaction period of at least 1 hour, alternatively, at least 2 hours.

In a specific embodiment of step 2-2, the reaction is as follows. 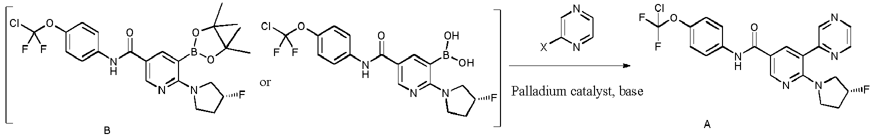 wherein X is halogen; alternatively, I or Br; still alternatively, I.

In another specific embodiment of step 2-2, the reaction comprises reacting the compound of formula (B), boronic acid hydrolysis product thereof, or a mixture thereof with 2-halopyrazine in DMSO or DMF in the presence of a palladium catalyst and a base.

In another specific embodiment of step 2-2, the DMSO or DMF contains water in a volume ratio of 0.01-0.5:1; alternatively, 0.05-0.2:1, alternatively, 0.067: 1, 0.1: 1, or 0.2:1.

In another specific embodiment of step 2-2, the base is selected from sodium phosphate, potassium phosphate, sodium carbonate, potassium carbonate, cesium carbonate, quaternary ammonium salt, NaF, KF, CsF, sodium bicarbonate, potassium bicarbonate, dibasic sodium phosphate, or dibasic potassium phosphate.

In another specific embodiment of step 2-2, the quaternary ammonium salt is selected from organic quaternary ammonium salts including tetrabutylammonium fluoride, tetrabutylammonium bromide, tetraethylammonium fluoride, tetraethylammonium bromide, tetramethylammonium fluoride, tetramethylammonium bromide, or tetramethylammonium chloride; alternatively, tetrabutylammonium fluoride.

In another specific embodiment of step 2-2, the base is selected from sodium phosphate, potassium phosphate, sodium carbonate, potassium carbonate, cesium carbonate, tetrabutylammonium fluoride, NaF, KF, and CsF.

In another specific embodiment of step 2-2, the palladium catalyst is selected from 1,1'-bis(diphenylphosphino)ferrocene palladium dichloride, palladium acetate, tetrakis(triphenylphosphine)palladium, tris(dibenzylideneacetone)dipalladium, palladium dichloride or bis(triphenylphosphine)palladium dichloride; alternatively, 1,1'-bis(diphenylphosphino)ferrocene palladium dichloride, tetrakis(triphenylphosphine)palladium, tris(dibenzylideneacetone)dipalladium or bis(triphenylphosphine)palladium dichloride; alternatively, 1,1'-bis(diphenylphosphino)ferrocene palladium dichloride.

In another specific embodiment of step 2-2, the molar ratio of 2-halopyrazine to compound B or its boronic acid hydrolysis product or a mixture thereof is 0.8-1.5:1; alternatively, 1.2:1.

In another specific embodiment of step 2-2, the amount of the base is 1-3.5 times that of compound B or its boronic acid hydrolysis product or a mixture thereof; alternatively, 1.2-2.7 times; alternatively, 1.2, 1.5, 1.8, 2.0, 2.1, 2.2, 2.4 or 2.7 times.

In another specific embodiment of step 2-2, the amount of the palladium catalyst is 0.005-0.1 times that of compound B or its boronic acid hydrolysis product or a mixture thereof; alternatively, 0.01-0.05 times; alternatively, 0.01, 0.02, 0.03, 0.04 or 0.05 times.

In another specific embodiment of step 2-2, the reaction is carried out at a temperature of room temperature to the temperature of solvent reflux, alternatively, 30-80°C, or still alternatively, 30±5°C, 50±5°C, 65±5°C or 80±5°C; alternatively, for a reaction period of at least 1 hour, alternatively, at least 3 hours.

In a specific embodiment of step 2-2, the reaction is as follows. 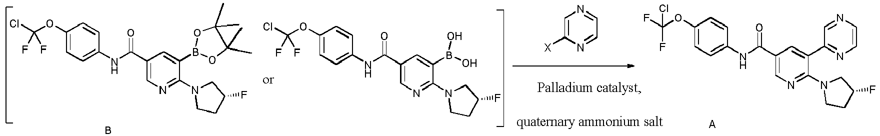 wherein X is halogen; alternatively, I or Br; still alternatively, I.

In another specific embodiment of step 2-2, the reaction comprises reacting the compound of formula (B), boronic acid hydrolysis product thereof, or a mixture thereof with 2-halopyrazine in the presence of a palladium catalyst and a quaternary ammonium salt.

In another specific embodiment of step 2-2, the quaternary ammonium salt is selected from organic quaternary ammonium salts including tetrabutylammonium fluoride, tetrabutylammonium bromide, tetraethylammonium fluoride, tetraethylammonium bromide, tetramethylammonium fluoride, tetramethylammonium bromide, or tetramethylammonium chloride; alternatively, tetrabutylammonium fluoride.

In another specific embodiment of step 2-2, the palladium catalyst is selected from 1,1'-bis(diphenylphosphino)ferrocene palladium dichloride, palladium acetate, tetrakis(triphenylphosphine)palladium, tris(dibenzylideneacetone)dipalladium, palladium dichloride or bis(triphenylphosphine)palladium dichloride; alternatively, 1,1'-bis(diphenylphosphino)ferrocene palladium dichloride, tetrakis(triphenylphosphine)palladium, tris(dibenzylideneacetone)dipalladium or bis(triphenylphosphine)palladium dichloride; alternatively, 1,1'-bis(diphenylphosphino)ferrocene palladium dichloride.

In another specific embodiment of step 2-2, the molar ratio of 2-halopyrazine to compound B or its boronic acid hydrolysis product or a mixture thereof is 0.8-1.5:1; alternatively, 1.2:1.

In another specific embodiment of step 2-2, the amount of the base is 1-3.5 times that of compound B or its boronic acid hydrolysis product or a mixture thereof; alternatively, 1.2-2.7 times; alternatively, 1.2, 1.5, 1.8, 2.0, 2.1, 2.2, 2.4 or 2.7 times.

In another specific embodiment of step 2-2, the amount of the palladium catalyst is 0.005-0.1 times that of compound B or its boronic acid hydrolysis product or a mixture thereof; alternatively, 0.01-0.05 times; alternatively, 0.01, 0.02, 0.03, 0.04 or 0.05 times.

In another specific embodiment of step 2-2, the reaction is carried out in a solvent selected from DCM, DCE, ethyl acetate, methyl acetate, isopropyl acetate, n-hexane, n-heptane, petroleum ether, acetone, acetonitrile, toluene, methyl tert-butyl ether, tetrahydrofuran, 2-methyltetrahydrofuran, isopropanol, water, ethylene glycol monomethyl ether, ethylene glycol dimethyl ether, DMF, DMA, DMSO, or a mixture thereof; alternatively, the solvent is selected from tetrahydrofuran, 2-methyltetrahydrofuran, ethylene glycol monomethyl ether, isopropanol, water, toluene, DMF, DMSO or a mixture thereof; alternatively, the solvent is tetrahydrofuran, DMF, isopropanol, water, toluene or a mixture thereof.

In another specific embodiment of step 2-2, the reaction is carried out at a temperature of room temperature to the temperature of solvent reflux, alternatively, 30-80°C, or still alternatively, 30±5°C, 50±5°C, 65±5°C or 80±5°C; alternatively, for a reaction period of at least 1 hour, alternatively, at least 3 hours.

### Step 3

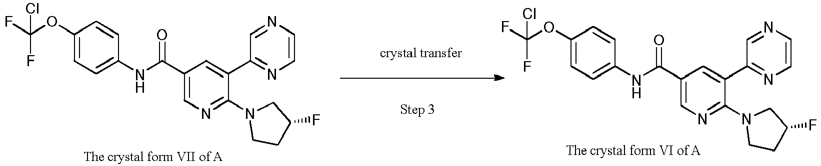

In Step 3, the crystal form VII of compound A is converted into the crystal form VI of compound A. In one embodiment, step 3 comprises steps of dissolving the crystal form VII of compound A in acetone solution, concentrating at atmospheric pressure, then adding n-heptane in two batches, and then distilling at atmospheric pressure. In one embodiment, the temperature is 75±5 °C. This crystalline conversion yields substantially pure crystal form VI of compound A.

The method of the present application is an improvement over the methods disclosed in the prior art. The preparation of compound A has been disclosed in International Publication No. WO 2018/133827 A1 (also referred to herein as "the '827 publication").

The method for preparing compound A in the '827 publication is described in Scheme 2 below:

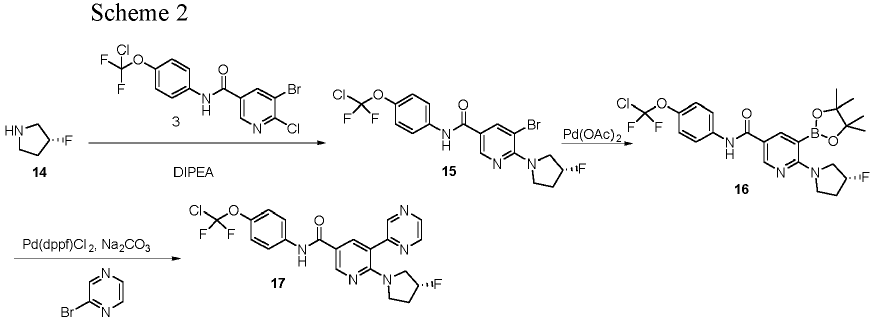

There are a number of differences between the method of '827 and the method of the present disclosure. At least some of the differences between the two methods are described in Table A below:

**Table A: Differences between the method of '827 and the method of the present disclosure**

| Synthesis steps | Change | | The advantages of change |
|---|---|---|---|
| | The method of '827 | The method of the present disclosure | |
| Step 1 | DIPEA | Na₂CO₃ | Switching to inorganic alkali to make post-treatment simpler |
| | 140°C | 70°C | Lowering the reaction temperature to reduce safety issues that exist at high temperatures |
| Step 2-1 | Dioxane | DMSO | Changing the reaction solvent system to avoid the risk of genotoxicity of dioxane |
| | K₃PO₄ | KOAc | Avoiding the use of potassium phosphate to avoid the risk of possible self-coupling by-product |
| Step 2-2 | 2-Bromopyrazine | 2-Iodopyrazine | Switching from expensive 2-bromopyrazine to relatively inexpensive 2-iodopyrazine |
| | Na₂CO₃ | TBAF | Replacing alkali with TBAF to reduce the risk of instability of the substrate boron ester in an alkaline reaction system |
| Step 3 | N/A | Dissolved in acetone; concentrated at atmospheric pressure, then adding n-heptane in batches, and then concentrated at atmospheric pressure until the temperature reaches 75±5 °C | Developping crystal transformation process |

The differences between the method of the present disclosure and the method of '827 lead to significant improvements of the '827 method, including those involving scale optimization, safety, increased yield, improved purity, and overall method. The method of '827 also has disadvantages in separation, that is, the reaction product is purified on a column, i.e. a very expensive separation method that is not available on an industrial scale, in all steps. In contrast, the method of the present disclosure uses centrifugation, filtration, and recrystallization for separation, and is more suitable for large-scale production.

### Substantially pure crystal form VI of compound A

The present disclosure provides a method for synthesizing the crystal form VI of compound A in high purity and high chiral purity, which is safe and suitable for large-scale production and can be used in a composition comprising the crystal form VI of compound A. On the one hand, the crystal form VI of compound A is produced by a commercial scale method. The term "commercial scale method" refers to a method that is run in a single batch of at least about 100 g. On the another hand, the method of the present application produces the crystal form VI of compound A in an improved yield (>90%) with limited impurities.

The term "purity" as used herein refers to the percentage content of the crystal form VI of compound A based on HPLC. Purity is based on the "organic" purity of the compound. Purity does not include water, solvents, metals, inorganic salts, etc. The purity of the crystal form VI of compound A is compared to the purity of the reference standard by comparing the area under the peak.

In one embodiment, the crystal form VI of compound A has a purity of not less than about 96%. In another embodiment, the crystal form VI of compound A has a purity of not less than about 98%. In yet another embodiment, the crystal form VI of compound A has a purity of not less than about 98.5%. In yet another embodiment, the crystal form VI of compound A has a purity of not less than about 99%. In yet another embodiment, the crystal form VI of compound A has a purity of not less than about 99.5%. In yet another embodiment, the crystal form VI of compound A has a purity of 96.0%, 96.1%, 96.2%, 96.3%, 96.4%, 96.5%, 96.6%, 96.7%, 96.8%, 96.9%, 97.0%, 97.1%, 97.2%, 97.3%, 97.4%, 97.5%, 97.6%, 97.7%, 97.8%, 97.9%, 98.0%, 98.1%, 98.2%, 98.3%, 98.4%, 98.5%, 98.6%, 98.7%, 98.8%, 98.9%, 99.0%, 99.1%, 99.2%, 99.3%, 99.4%, 99.5%, 99.6%, 99.7%, 99.8% or 99.9%.

The crystal form VI of compound A prepared in the present disclosure contains one chiral carbon atom and is R-configuration. The chiral center of the crystal form VI of compound A is introduced from the starting material and is not involved in the subsequent steps. Moreover, no racemization is observed.

The term "chiral purity" as used herein refers to the chiral purity of the crystal form VI of compound A as determined by chiral HPLC. Chiral purity is based on the "organic" purity of the compound. Chiral purity is not associated with water, solvents, metals, inorganic salts, etc. The chiral purity of the crystal form VI of compound A is compared to the chiral purity of the reference standard by comparing the area under the peak.

In one embodiment, the crystal form VI of compound A has a chiral purity of not less than about 96%. In another embodiment, the crystal form VI of compound A has a chiral purity of not less than about 98%. In yet another embodiment, the crystal form VI of compound A has a chiral purity of not less than about 99%. In yet another embodiment, the crystal form VI of compound A has a chiral purity of not less than about 99.4%. In another embodiment, the crystal form VI of compound A has a chiral purity of 96.0%, 96.1%, 96.2%, 96.3%, 96.4%, 96.5%, 96.6%, 96.7%, 96.8%, 96.9%, 97.0%, 97.1%, 97.2%, 97.3%, 97.4%, 97.5%, 97.6%, 97.7%, 97.8%, 97.9%, 98.0%, 98.1%, 98.2%, 98.3%, 98.4%, 98.5%, 98.6%, 98.7%, 98.8%, 98.9%, 99.0%, 99.1%, 99.2%, 99.3%, 99.4%, 99.5%, 99.6%, 99.7%, 99.8% or 99.9%.

In one embodiment, the present disclosure relates to the crystal form VI of compound A containing less than about 0.8% total impurities. In another embodiment, the total impurities are less than about 0.5%. In yet another embodiment, the total impurities are less than about 0.3%. In yet another embodiment, the total impurities are less than about 0.2%.

In one embodiment, the present disclosure relates to the crystal form VI of compound A containing not more than about 1% of water, not more than about 0.8% of water, not more than about 0.7% of water, not more than about 0.6% of water, not more than about 0.5% of water, not more than about 0.4% of water, not more than about 0.3% of water, not more than about 0.2% of water, not more than about 0.1% of water, not more than about 0.09% of water, not more than about 0.08% of water, not more than about 0.07% of water, not more than about 0.06% of water, not more than about 0.05% of water. In another embodiment, the present disclosure relates to the crystal form VI of compound A containing not more than about 0.11% of water. In yet another embodiment, the present disclosure relates to the crystal form VI of compound A containing not more than about 0.1% of water. In yet another embodiment, the present disclosure relates to the crystal form VI of compound A containing not more than about 0.09% of water.

### Pharmaceutical composition

In another aspect, the present disclosure provides a pharmaceutical composition comprising (i) a pharmaceutically active ingredient: a crystal form of the free base of compound A or pharmaceutically acceptable salts thereof, (ii) a diluent, (iii) a disintegrant, (iv) a glidant, and (v) a lubricant.

In a specific embodiment of the above aspect, the active pharmaceutical ingredient is the crystal form of the free base of compound A; alternatively, the crystal form is selected from the crystal form I of compound A, the crystal form II of compound A, the crystal form III of compound A, the crystal form IV of compound A, the crystal form V of compound A, the crystal form VI of compound A, the crystal form VII of compound A, the crystal form VIII of compound A, the crystal form IX of compound A, the crystal form X of compound A and the crystal form XI of compound A; alternatively, the crystal form is selected from the crystal form I of compound A, the crystal form IV of compound A, the crystal form VI of compound A and the crystal form XI of compound A. In another specific embodiment, the pharmaceutically active ingredient is the crystal form I of compound A hydrochloride, the crystal form I of compound A benzenesulfonate, the crystal form I of compound A p-toluenesulfonate, the crystal form II of compound A p-toluenesulfonate, the crystal form I of compound A mesylate or the crystal form I of compound A hydrobromide; alternatively, the crystal form is selected from the crystal form I of compound A benzenesulfonate, the crystal form I of compound A p-toluenesulfonate, the crystal form II of compound A p-toluenesulfonate or the crystal form I of compound A mesylate.

In another aspect, the present disclosure provides the pharmaceutical composition described above, wherein the pharmaceutically active ingredient accounts for 1-30%, alternatively, 5-20%, alternatively, 8-15%, or still alternatively, about 10% by weight of the total weight of the pharmaceutical composition, based on the weight of the free base of the compound; alternatively, wherein the amount of the pharmaceutically active ingredient in a unit dose is 1-100 mg, alternatively, 5-50 mg, alternatively, 8-40 mg, alternatively, about 10, 20, 30 or 40 mg.

In another aspect, the present disclosure provides the pharmaceutical composition described above, wherein the diluent accounts for 65-95%, alternatively, 70-90%, alternatively, about 80%, 81%, 82%, 83%, 84% or 85% by weight of the total weight of the pharmaceutical composition; alternatively, wherein the amount of the diluent in a unit dose is 65-380 mg, alternatively, 70-360 mg, alternatively, 80-350 mg, such as, about 83 mg or 332 mg.

In another aspect, the present disclosure provides the pharmaceutical composition described above, wherein the diluent is selected from lactose monohydrate, microcrystalline cellulose, anhydrous calcium hydrogen phosphate, mannitol and pregelatinized starch, and mixtures thereof; alternatively, the microcrystalline cellulose is microcrystalline cellulose 102; the lactose monohydrate is selected from lactose monohydrate Granulac 200, lactose monohydrate Tablettose 80 and lactose monohydrate FLOWLAC^{®}100; alternatively, where both of lactose monohydrate and microcrystalline cellulose are present, the weight ratio of lactose monohydrate to microcrystalline cellulose is 5:1 to 1:5, alternatively, 2:1 to 1:3, alternatively, about 1:2, e.g. 1:1.96.

In another aspect, the present disclosure provides the pharmaceutical composition described above, wherein the disintegrant accounts for 1-5%, alternatively, 2-4%, alternatively, about 3% by weight of the total weight of the pharmaceutical composition; alternatively, wherein the amount of the disintegrant in a unit dose is 1-20 mg, alternatively, 2-16 mg, alternatively, about 3, 6, 9 or 12 mg.

In another aspect, the present disclosure provides the pharmaceutical composition described above, wherein the disintegrant is croscarmellose sodium.

In another aspect, the present disclosure provides the pharmaceutical composition described above, wherein the glidant accounts for 1-5%, alternatively, 2-4%, alternatively, about 3% by weight of the total weight of the pharmaceutical composition; alternatively, wherein the amount of (iv) glidant in a unit dose is 1-20 mg, alternatively, 2-16 mg, alternatively, about 3, 6, 9 or 12 mg.

In another aspect, the present disclosure provides the pharmaceutical composition described above, wherein the glidant is colloidal silica.

In another aspect, the present disclosure provides the pharmaceutical composition described above, wherein the lubricant accounts for 0.1-5%, alternatively, 0.5-2%, alternatively, about 1% by weight of the total weight of the pharmaceutical composition; alternatively, wherein the amount of the lubricant in a unit dose is 0.1-20 mg, alternatively, 0.5-8 mg, alternatively, about 1, 2, 3 or 4 mg.

In another aspect, the present disclosure provides the pharmaceutical composition described above, wherein the lubricant is magnesium stearate or sodium stearyl fumarate.

In another aspect, the present disclosure provides the pharmaceutical composition described above, comprising the following ingredients:
(i) 1-30% of the crystal form VI of compound A, by weight,
(ii) 70-90% of lactose monohydrate and microcrystalline cellulose (1:2 by weight), by weight,
(iii) 2-4% of croscarmellose sodium, by weight,
(iv) 2-4% of colloidal silica, by weight, and
(v) 0.1-5% of magnesium stearate, by weight.

In one embodiment, the present disclosure provides the pharmaceutical composition described above, wherein a unit dose comprises the following components:
(i) about 10 mg of the crystal form VI of compound A,
(ii) about 28 mg of lactose monohydrate and about 55 mg of microcrystalline cellulose,
(iii) about 3 mg of croscarmellose sodium,
(iv) about 3 mg of colloidal silica, and
(v) about 1 mg of magnesium stearate.

In one embodiment, the present disclosure provides the pharmaceutical composition described above, wherein a unit dose comprises the following components:
(i) about 40 mg of the crystal form VI of compound A,
(ii) about 112 mg of lactose monohydrate and about 220 mg of microcrystalline cellulose,
(iii) about 12 mg of croscarmellose sodium,
(iv) about 12 mg of colloidal silica, and
(v) about 4 mg of magnesium stearate.

In another aspect, the present disclosure provides a pharmaceutical composition comprising:
(i) a compound of formula (A),
(ii) a diluent,
(iii) a disintegrant,
(iv) a glidant, and
(v) a lubricant,
wherein the diluent accounts for 1-5%, alternatively, 2-4%, alternatively, about 3% by weight of the total weight of the pharmaceutical composition.

In another aspect, the present disclosure provides the pharmaceutical composition described above, wherein the glidant is colloidal silica.

In another aspect, the present disclosure provides the pharmaceutical composition described above, wherein the content of the glidant in a unit dose is 1-20 mg, alternatively, 2-16 mg, alternatively, about 3, 6, 9 or 12 mg.

In another aspect, the present disclosure provides the pharmaceutical composition described above, wherein the diluent accounts for 65-95%, alternatively, 70-90%, alternatively, about 80%, 81%, 82%, 83%, 84% or 85% by weight of the total weight of the pharmaceutical composition; alternatively, wherein the content of the diluent in a unit dose is 65-380 mg, alternatively, 70-360 mg, alternatively, 80-350 mg, such as about 83 mg or 332 mg.

In another aspect, the present disclosure provides the pharmaceutical composition described above, wherein the diluent is selected from lactose monohydrate, microcrystalline cellulose, anhydrous calcium hydrogen phosphate, mannitol and pregelatinized starch, and mixtures thereof; alternatively, where both of lactose monohydrate and microcrystalline cellulose are present, the weight ratio of lactose monohydrate to microcrystalline cellulose is 5:1 to 1:5, alternatively, 2:1 to 1:3, alternatively, about 1:2, such as 1:1.96.

In another aspect, the present disclosure provides the pharmaceutical composition described above, wherein the disintegrant accounts for 1-5%, alternatively, 2-4%, alternatively, about 3% by weight of the total weight of the pharmaceutical composition; alternatively, wherein the content of the disintegrant in a unit dose is 1-20 mg, alternatively, 2-16 mg, alternatively, about 3, 6, 9 or 12 mg.

In another aspect, the present disclosure provides the pharmaceutical composition described above, wherein the disintegrant is croscarmellose sodium.

In another aspect, the present disclosure provides the pharmaceutical composition described above, wherein the lubricant accounts for 0.1-5%, alternatively, 0.5-2%, alternatively, about 1% by weight of the total weight of the pharmaceutical composition; alternatively, wherein the amount of the lubricant in a unit dose is 0.1-20 mg, alternatively, 0.5-8 mg, alternatively, about 1, 2, 3 or 4 mg.

In another aspect, the present disclosure provides the pharmaceutical composition described above, wherein the lubricant is magnesium stearate or sodium stearyl fumarate.

The present disclosure also provides a method for preparing a tablet, the method comprising a) mixing a crystal form of the free base of compound A or a salt thereof, a diluent, a disintegrant and a glidant to form a mixture; and b) adding a lubricant to the mixture described in a).

### Pharmacology and efficacy

The present disclosure provides a method of treating a disease or disorder caused by Bcr-Abl in a subject, comprising administering to the subject an effective amount of various crystal forms of the free base of compound A. In some embodiments, the subject is a human subj ect.

The compounds disclosed herein show therapeutic efficacy especially on diseases or disorders that are dependent on the activity of Bcr-Abll. In particular, the compounds disclosed herein inhibit the ATP binding site of Bcr-Abl1 (including wild-type Bcr-Abl1 and/or its mutations (including T315I mutations)).

Carcinoma cells utilize invapodia to degrade the extra cellular matrix during tumor invasion and metastasis. Abl kinase activity is required for Src-induced invapodia formation, regulating distinct stages of invapodia assembly and function. The compounds disclosed herein, therefore, as inhibitors of Abl, have the potential to be used as therapies for the treatment of metastatic invasive carcinomas.

An inhibitor of c-Abl kinase can be used to treat brain cancers: including Glioblastoma which is the most common and most aggressive malignant primary brain tumor in which the expression of c-Abl is immunohistochemically detectable in a subset of patients. Therefore, a new c-Abl inhibitor with high brain exposure represents a solid therapeutic approach for glioblastoma and other brain cancers.

Compounds disclosed herein can be useful in the treatment of viruses. For example, viral infections can be mediated by Abl1 kinase activity, as in the case of pox viruses and the Ebola virus. Imatinib and nilotinib have been shown to stop the release of Ebola viral particles from infected cells, in vitro. Compounds disclosed herein that inhibit c-Abl kinase, therefore, can be expected to reduce the pathogen's ability to replicate.

Parkinson's disease is the second most prevalent chronic neurodegenerative disease with the most common familial autosomal-recessive form being caused by mutations in the E3 ubiquitin ligase, parkin. Recent studies showed that activated c-ABL was found in the striatum of patients with sporadic Parkinson's disease. Concomitantly, parkin was tyrosine-phosphorylated, causing loss of its ubiquitin ligase and cytoprotective activities as indicated by the accumulation of parkin substrates.

The compounds or compositions disclosed herein are also useful in the treatment of diseases, disorders or conditions mediated by Bcr-Abl kinase: respiratory diseases, allergies, rheumatoid arthritis, osteoarthritis, rheumatic disorders, psoriasis, ulcerative colitis, Crohn's disease, septic shock, proliferative disorders, atherosclerosis, allograft rejection after transplantation, diabetes, stroke, obesity or restenosis, leukemia, stromal tumor, thyroid cancer, systemic mastocytosis, eosinophilia syndrome, fibrosis, rheumatoid arthritis, polyarthritis, scleroderma, lupus erythematosus, graft versus host disease, neurofibromatosis, pulmonary hypertension, Alzheimer's disease, seminoma, dysgerminoma, mast cell tumor, lung cancer, bronchial carcinoma, dysgerminoma, testicular intraepithelial neoplasia, melanoma, breast cancer, neuroblastoma, papillary/follicular parathyroid hyperplasia/adenoma, colon cancer, colorectal adenoma, ovarian cancer, prostate cancer, glioblastoma, brain tumor, malignant glioma, pancreatic cancer, malignant pleura tumor, hemangioblastoma, hemangioma, kidney cancer, liver cancer, adrenal cancer, bladder cancer, stomach cancer, rectal cancer, vaginal cancer, cervical cancer, endometrial cancer, multiple myeloma, neck and head tumors, neoplasia and other proliferative disease or proliferative diseases, or the combination thereof.

### Examples

### General method 1. X-ray powder diffraction (XRPD)

The solid samples obtained from the experiments were analyzed with a D8 advance powder X-ray diffraction analyzer (Bruker) equipped with a LynxEye detector with a 2θ scanning angle from 3° to 40° and a scanning step length of 0.02 °. The light tube voltage and light tube current were 40 KV and 40 mA, respectively, when the samples were measured.

### General method 2. Polarizing microscope analysis (PLM)

The instrument model used for PLM analysis was ECLIPSE LV100POL polarizing microscope (Nikon, Japan).

### General method 3. Nuclear magnetic resonance hydrogen spectroscopy analysis (¹H NMR)

The chemical structure of solid samples was confirmed by ¹H NMR. ¹H NMR analysis was performed using a Bruker Advance 300 equipped with a B-ACS 120 autosampling system.

### General method 4. Differential scanning calorimetry analysis (DSC)

The instrument model for differential scanning calorimetry analysis was DSC Q200 or Discovery DSC 250 (TA, USA). Samples were accurately weighed and placed in a punctured sample pot for DSC, and the exact mass of the sample was recorded. The samples were heated to the final temperature (e.g. 300°C or 350°C) at a ramp rate of 10°C/min.

### General method 5. Thermogravimetric analysis (TGA)

The thermogravimetric analyzer model was TGA Q500 or Discovery TGA 55 (TA, USA). The sample was placed in an open aluminum sample pot that has been equilibrated and the mass was weighed automatically in the TGA heating oven. The sample was heated to the final temperature (e.g. 300°C or 350°C) at a ramp rate of 10°C/min.

### General method 6. Dynamic moisture adsorption and desorption analysis (DVS)

The instrument model used for dynamic moisture adsorption and desorption analysis was IGA Sorp (Hidentity Isochema). The samples were measured in a gradient mode with a moisture range of 0% to 90%, wherein the humidity increment of each gradient was 10%.

### Example 1 Preparation of compound A

Compound A was prepared using the method in Example 7 of WO 2018/133827 A1 as follows:

### Preparation of (R)-N-(4-(chlorodifluoromethoxy)phenyl)-6-(3-fluoropyrrolidin-1-yl)-5-(pyrazin-2-yl)nicotinamide (Compound 17)

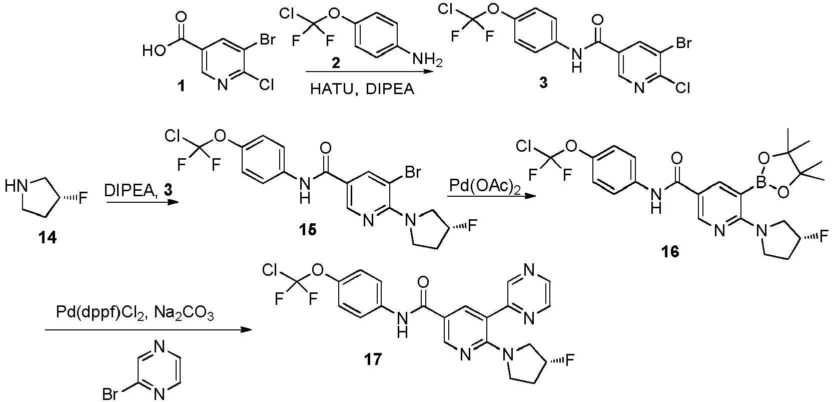

### Step 1: Synthesis of 6-chloro-5-bromo-N-(4-(chlorodifluoromethoxy)phenyl)nicotinamide (compound 3)

To a reaction flask were added 6-chloro-5-bromonicotinic acid (1.17 g, 4.97 mmol) and 4-(chlorodifluoromethoxy)aniline (0.8 g, 4.15 mmol), and dissolved in 20 mL of anhydrous DMF. 2-(7-Aza-1H-benzotriazol-1-yl)-1,1,3,3-tetramethyluronium hexafluorophosphate (HATU, 2.1 g, 5.39 mmol) and N,N-diisopropylethylamine (DIPEA, 534 mg, 4.15 mmol) were added, and the reaction was stirred under nitrogen protection at room temperature for 18 hours. The reaction mixture was diluted with a large amount of water, and extracted 3-4 times with ethyl acetate. The organic phases were combined, washed with saturated brine, concentrated, purified by column chromatography, and dried in vacuum to afford 1.18 g of a product, yield: 69.5%.

### Step 2: Synthesis of (R)-6-(3-fluoropyrrolidin-1-yl)-5-bromo-N-(4-(chlorodifluoromethoxy)phenyl)nicotinamide (compound 15)

To a reaction flask were added compound 3 (0.90 g, 2.19 mmol) and (R)-3-fluoropyrrolidine hydrochloride (330 mg, 2.63 mmol). 15 mL of isopropyl alcohol and DIPEA (621 mg, 4.82 mmol) were added. The mixture was heated to 140 °C and reacted with stirring for 2 hours. After cooling to room temperature, the reaction mixture was concentrated to remove the solvent. The residue was purified by silica gel column chromatography to afford 841 mg of a product, yield: 83%.

### Step 3: Synthesis of (R)-6-(3-fluoropyrrolidin-1-yl)-5-(4,4,5,5-tetramethyl-1,3,2-dioxaborolan-2-yl)-N-(4-(chlorodifluoromethoxy)phenyl)nicotinamide (compound 16)

In a reaction flask were added compound 15 (628 mg, 1.35 mmol), bis(pinacolato)diboron (1.03 g, 4.06 mmol), palladium acetate (10 mg, 0.041 mmol), Xphos (50 mg, 0.101 mmol) and potassium phosphate (861 mg, 4.06 mmol). The mixture was dissolved by adding 20 mL of anhydrous dioxane. The solution was heated to 60 °C in microwave and reacted for 4 hours. TLC showed that the starting material was not completely comsumed. Additional bis(pinacolato)diboron (1.03 g, 4.06 mmol) was added, and then the mixture was reacted at 60 °C overnight. TLC showed that the reaction was completed. The reaction mixture was concentrated, and purified by silica gel column chromatography to afford 514.3 mg of a product, yield: 75%.

### Step 4: Synthesis of (R)-N-(4-(chlorodifluoromethoxy)phenyl)-6-(3-fluoropyrrolidin-1-yl)-5-(pyrazin-2-yl)nicotinamide (compound 17).

To a reaction flask were added compound 16 (200 mg, 0.372 mmol), 2-bromopyrazine (50 mg, 0.27 mmol), Pd(dppf)Cl₂ (10 mg, 0.01 mmol) and sodium carbonate (60 mg, 0.558 mmol). 5 mL of glycol dimethyl ether and 0.9 mL of water were added, and the mixture was bubbled with nitrogen gas for 10 minutes. The mixture was heated to 100 °C in microwave and reacted for half an hour. TLC showed that the reaction was completed. The reaction mixture was concentrated, and purified by silica gel column chromatography to afford 35 mg of a product, yield: 20.3%. LC-MS(APCI): m/z = 464.5 (M+1)⁺. ¹H NMR (400 MHz, CDCl₃) δ 8.78 (d, *J* = 2.0 Hz, 1H), 8.73 (s, 1H), 8.64 (s, 1H), 8.54 (d, *J* = 2.1 Hz, 1H), 8.11 (d, *J* = 2.0 Hz, 1H), 7.95 (s, 1H), 7.67 (d, *J=* 8.9 Hz, 2H), 7.23 (d, *J=* 8.6 Hz, 2H), 5.28 (s, 1H), 5.14 (s, 1H), 3.70 - 3.57 (m, 1H), 3.52 (d, *J=* 10.8 Hz, 1H), 3.42 (t, *J=* 11.6 Hz, 1H), 3.30 (t, *J=* 9.5 Hz, 1H), 2.26 - 2.17 (m, 1H), 2.02 (dd, *J* = 9.5, 3.8 Hz, 1H).

Compound A was recrystallized with anhydrous ethanol to obtain a crystal form, which was named as crystal form I.

### Example 2 Large-scale production of the crystal form VI of compound A

Three batches of the crystal form VI of compound A were prepared in kilogram-scale using the process of Scheme 1.

### Example 2.1 Preparation of starting material 5-bromo-6-chloro-N-(4-(chlorodifluoromethoxy)phenyl)nicotinamide (compound D)

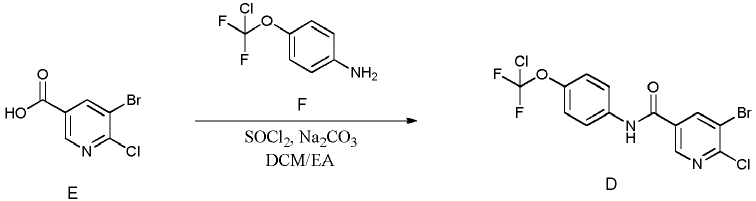

Dichloromethane (4 volumes), 5-bromo-6-chloronicotinic acid (1.0 eq.) and DMF (0.05 eq.) were added to a reactor, and the mixture was stirred. The temperature was controlled not to exceed 5 °C. Dichlorosulfoxide (2.0 eq.) was slowly added dropwise. After the addition was completed, the mixture was heated to 40 °C for reflux. The mixture was stirred for at least 20 hours. Then a sample was taken and sent to HPLC for detection until 5-bromo-6-chloronicotinic acid was less than 2.0%. The mixture was cooled to room temperature, and concentrated under reduced pressure. Additional ethyl acetate was added, and the mixture was concentrated. The operation was repeated. Ethyl acetate was added (to 5 volumes) for dilution and dissolution for later use (a solution of acyl chloride intermediate in ethyl acetate).

Water (4 volumes) and sodium carbonate (2.0 eq.) were added sequentially to a reactor with stirring under nitrogen. The mixture was stirred until clear. Ethyl acetate (2 volumes) and 4-(chlorodifluoromethoxy)aniline (0.95 eq. - 1.0 eq.) were added. The solution of acyl chloride intermediate in ethyl acetate was slowly added dropwise while the temperature was controlled below 5 °C. The mixture was stirred at room temperature for 1 hour, and a sample was taken and sent to HPLC for monitoring. The mixture was concentrated at 45 °C under reduced pressure to remove ethyl acetate, and filtered by suction. The filter cake was rinsed with water. The filter cake was collected and dried in a vacuum oven at 45 °C. The purity of the starting material, 5-bromo-6-chloro-N-(4-(chlorodifluoromethoxy)phenyl)nicotinamide, was detected by an appropriate HPLC method.

According to the above synthetic method, three batches of 5-bromo-6-chloro-N-(4-(chlorodifluoromethoxy)phenyl)nicotinamide (compound D) have been prepared in pilot kilogram-scale, and the results are listed in Table 2.1.

**Table 2.1. Batch production results of compound D**

| Batch No. | Amount of materials | | Compound D | | |
|---|---|---|---|---|---|
| | | | Yield (weight) | Yield (%) | Purity |
| 1 | Compound E (1.0 eq.) | 4.0 kg | 6.6 kg | 94.6% | 95.4 % |
| | Compound F (0.95 eq.) | 3.1 kg | | | |
| | Thionyl chloride (2.0 eq.) | 4.0 kg | | | |
| | Sodium carbonate (2.0 eq.) | 3.6 kg | | | |
| | DMF (0.05 eq.) | 62 g | | | |
| | Solvent (dichloromethane/ethyl acetate/water) (per volume) | 4 L | | | |
| 2 | Compound E (1.0 eq.) | 4.2 kg | 6.742 kg | 92.0% | 95.7% |
| | Compound F (0.97 eq.) | 3.3 kg | | | |
| | Thionyl chloride (2.0 eq.) | 4.2 kg | | | |
| | Sodium carbonate (2.0 eq.) | 3.8 kg | | | |
| | DMF (0.05 eq.) | 65 g | | | |
| | Solvent (dichloromethane/ethyl acetate/water) (per volume) | 4.2 L | | | |
| 3-1 (First pot) | Compound E (1.0 eq.) | 3.6 kg | 11.0 kg | 88.4% | 97.8% |
| | Compound F (0.97 eq.) | 2.8 kg | | | |
| | Thionyl chloride (2.0 eq.) | 3.6 kg | | | |
| | Sodium carbonate (2.0 eq.) | 3.2 kg | | | |
| | DMF (0.05 eq.) | 55 g | | | |
| | Solvent (dichloromethane/ethyl acetate/water) (per volume) | 3.6 L | | | |
| 3-2 (Second pot) | Compound E (1.0 eq.) | 3.6 kg | | | |
| | Compound F (1.0 eq.) | 2.9 kg | | | |
| | Thionyl chloride (2.0 eq.) | 3.6 kg | | | |
| | Sodium carbonate (2.0 eq.) | 3.2 kg | | | |
| | DMF (0.05 eq.) | 55 g | | | |
| | Solvent (dichloromethane/ethyl acetate/water) (per volume) | 3.6 L | | | |

### Example 2.2 Preparation of the crystal form VI of compound A

### Step 1: Preparation of (R)-5-bromo-N-(4-(chlorodifluoromethoxy)phenyl)-6-(3-fluoropyrrol-1-yl)nicotinamide (compound C)

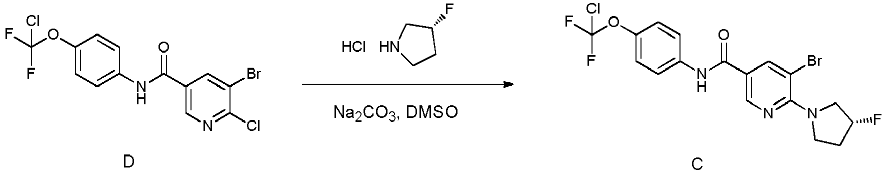

DMSO (3 volumes) was added to reactor A under nitrogen, and 5-bromo-6-chloro-N-(4-(chlorodifluoromethoxy)phenyl)nicotinamide (1.0 eq.), 3-(R)-fluoropyrrolidine hydrochloride (1.0 eq.), and anhydrous sodium carbonate (2.2 eq.) were added under stirring. The mixture was heated to 70±10°C and reacted at this temperature with stirring for at least 22 hours. A sample was taken and sent to HPLC for detection until the reaction was completed *(standard: 5-bromo-6-chloro-N-(4-(chlorodifluoromethoxy)phenyl)nicotinamide* ≤ *2.0%*). The mixture was cooled to 20-30 °C for later use. Demineralized water (18 vol) was added to reactor B under nitrogen. The reaction solution in the reactor A was added dropwise to the reactor B, while the temperature was controlled at 20-30 °C. After the addition was completed, the mixture was stirred at the controlled temperature for at least 15 minutes. The mixture was centrifuged in a centrifugal machine under nitrogen, and the precipitate was rinsed with demineralized water. The precipitate was collected.

The precipitate was dried under vacuum at 55±5 °C for at least 10 hours. A trace stream of nitrogen gas was sent to the precipitate during drying, and a sample was taken for KF detection (*standard: KF* ≤ *4.0%*). The baking was stopped, and the precipitate was cooled to below 30 °C. A sample was taken and sent to HPLC for detection, and the precipitate was collected. The product was put into a double-layer LDPE bag and the bag was tied tightly. A desiccant accounting for about 10% of the product weight was added between the second layer and the third layer of the LDPE bag. The mixture was weighed, labeled as intermediate, and then stored temporarily at room temperature.

According to the above synthetic method, three batches of (R)-5-bromo-N-(4-(chlorodifluoromethoxy)phenyl)-6-(3-fluoropyrrol-1-yl)nicotinamide in pilot kilogram-scale have been prepared, and the results are listed in Table 2.2-1.

**Table 2.2-1. Batch production results of compound C**

| Batch No. | Amount of materials | | Compound C | | |
|---|---|---|---|---|---|
| | | | Yield (weight) | Yield (%) | Purity |
| 1 | Compound D (1.0 eq.) | 3.1 kg | 3.195 kg | 91.5% | 98.3% |
| | 3-(R)-Fluoropyrrolidine hydrochloride (1.0 eq.) | 0.93 kg | | | |
| | Anhydrous sodium carbonate (2.2 eq.) | 1.77 kg | | | |
| | Solvent (DMSO/water) (per volume) | 3.1 L | | | |
| 2 | Compound D (1.0 eq.) | 3.1 kg | 3.215 kg | 92.1% | 98.1% |
| | 3-(R)-Fluoropyrrolidine hydrochloride (1.0 eq.) | 0.93 kg | | | |
| | Anhydrous sodium carbonate (2.2 eq.) | 1.77 kg | | | |
| | Solvent (DMSO/water) (per volume) | 3.1 L | | | |
| 3 | Compound D (1.0 eq.) | 3.1 kg | 3.146 kg | 93.1% | 97.9% |
| | 3-(R)-Fluoropyrrolidine hydrochloride (1.0 eq.) | 0.93 kg | | | |
| | Anhydrous sodium carbonate (2.2 eq.) | 1.77 kg | | | |
| | Solvent (DMSO/water) (per volume) | 3.1 L | | | |

### Step 2: Preparation of the crystal form VII of compound A

### Step 2-1:

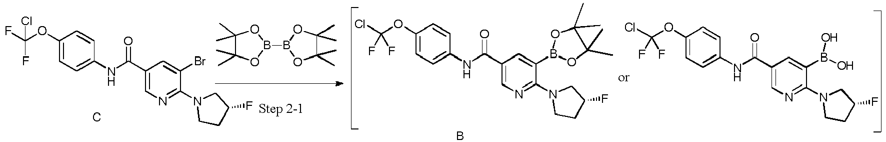

### Step 2-2:

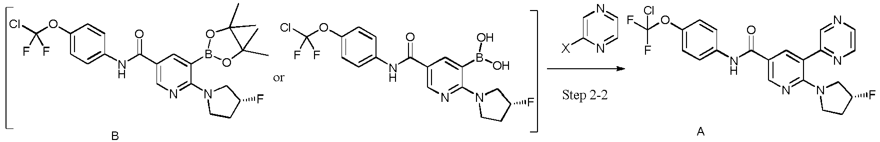

where X is halogen;

### Step 2-1: Preparation of intermediate (R)-(5-((4-(chlorodifluoromethoxy)phenyl)carbamoyl)-2-(3-fluoropyrrolidin-1-yl)pyridin-3-yl)boronic acid (compound B) or its boronic acid hydrolysis product or their mixture

The applicant has carried out in-depth research on the reaction for obtaining compound B, and has carried out step-by-step optimization of various conditions in the reaction, as detailed below:

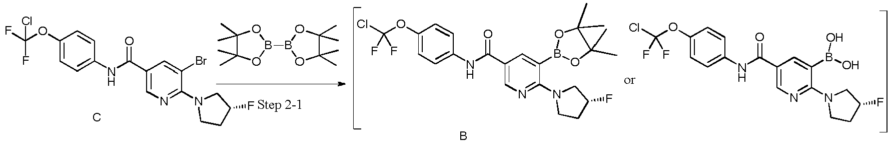

In the following tables, the following abbreviations were used:

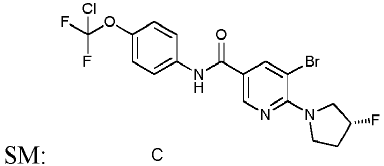

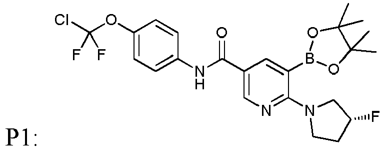

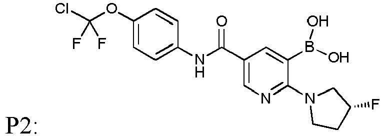

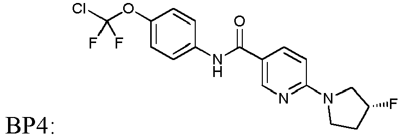

### 1)Optimization of the amount of B₂Pin₂

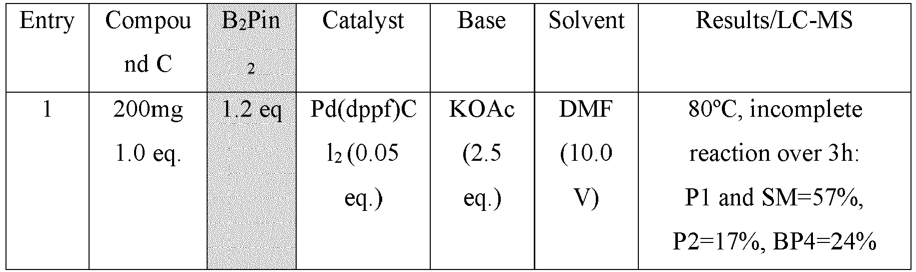

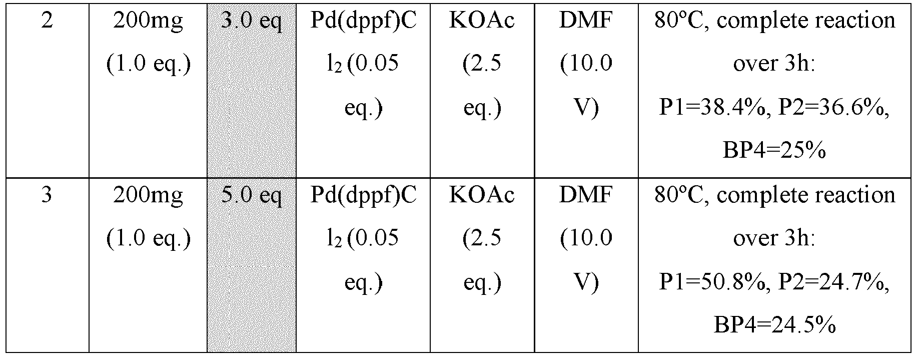

### 2)Optimization of solvent types

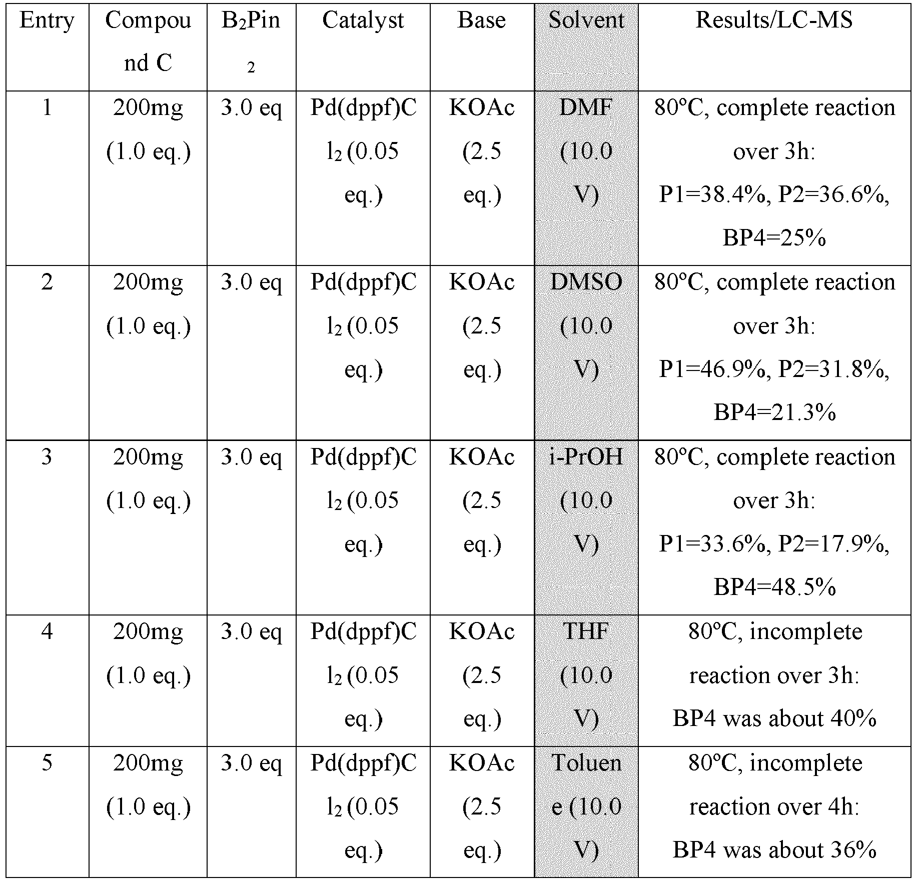

### 3)Optimization of the amount of solvent

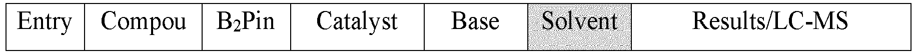

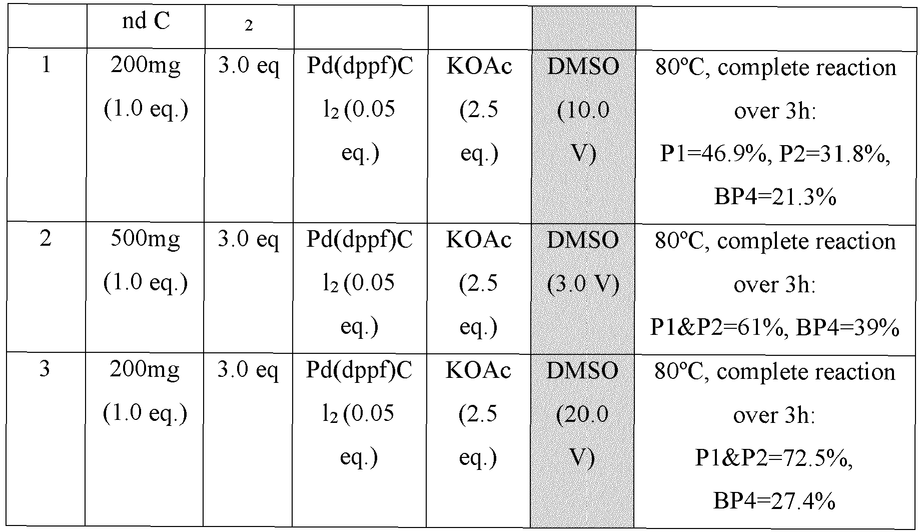

### 4)Optimization of base types

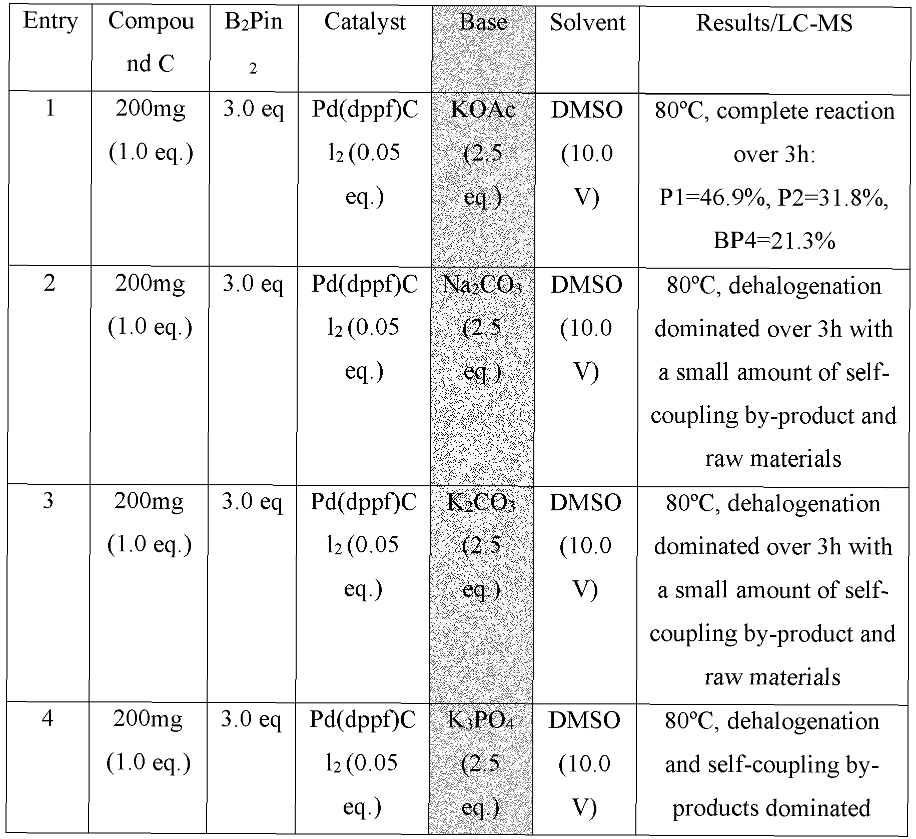

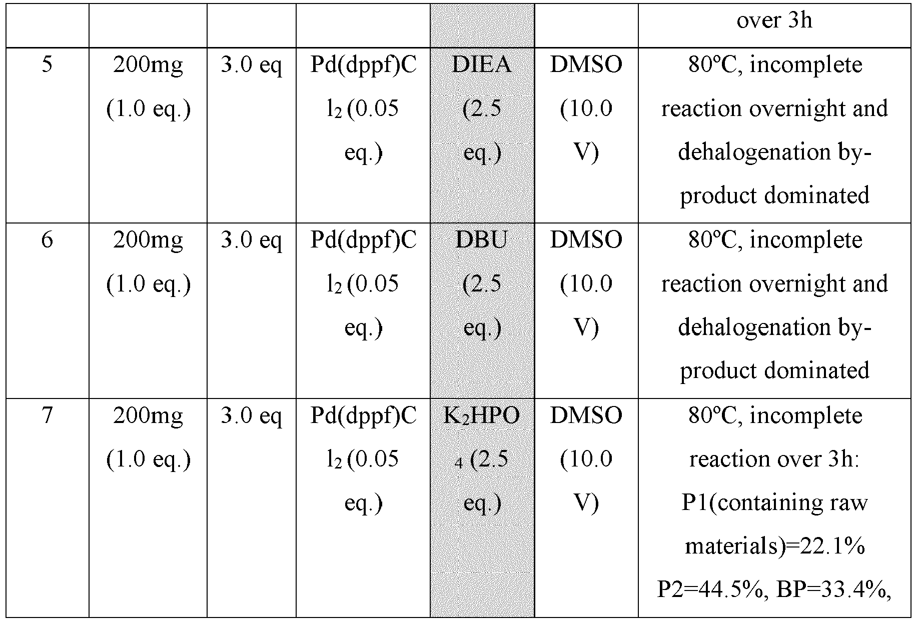

### 5)Optimization of catalysts and ligands

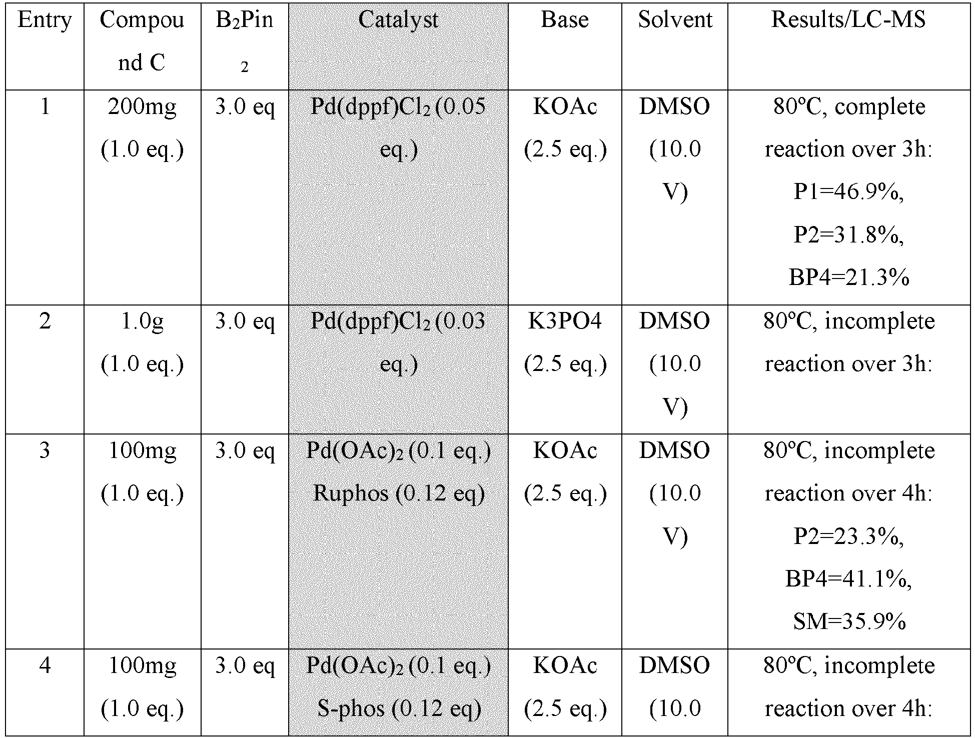

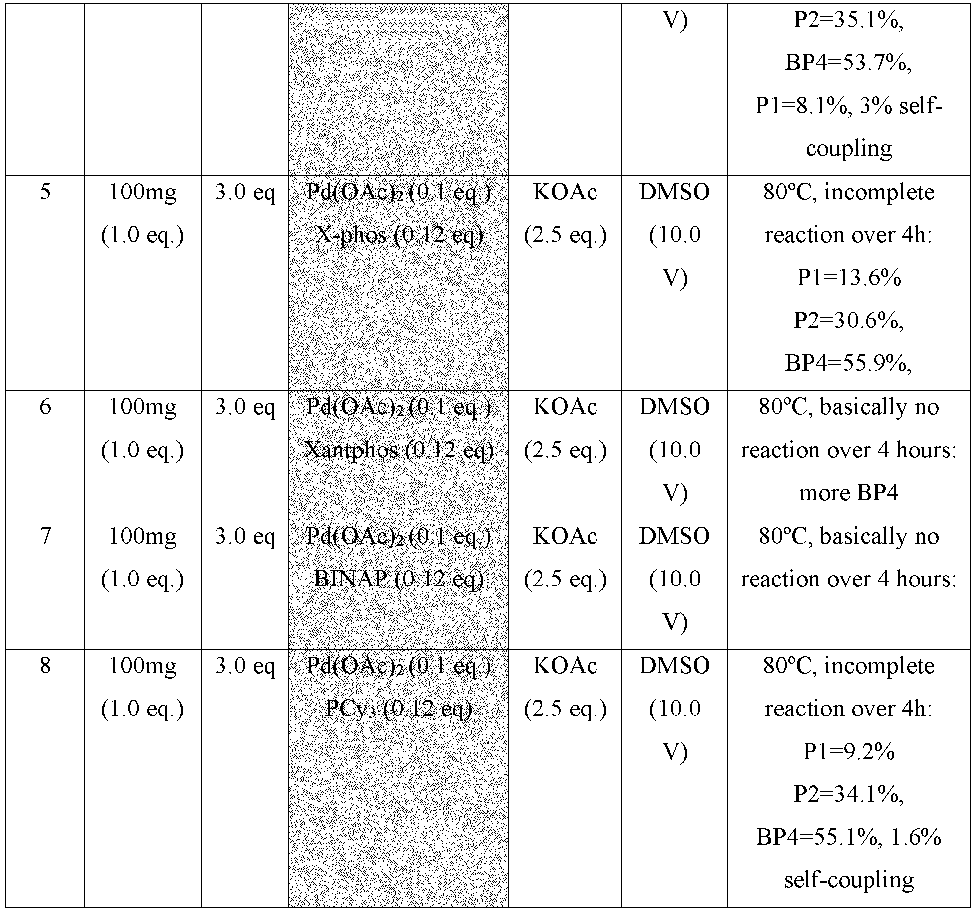

### 6)Optimization of the amount of base and B₂Pin₂

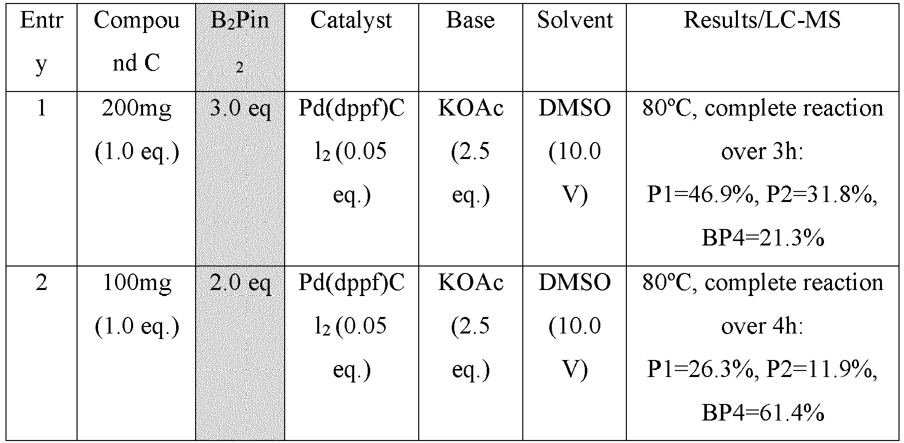

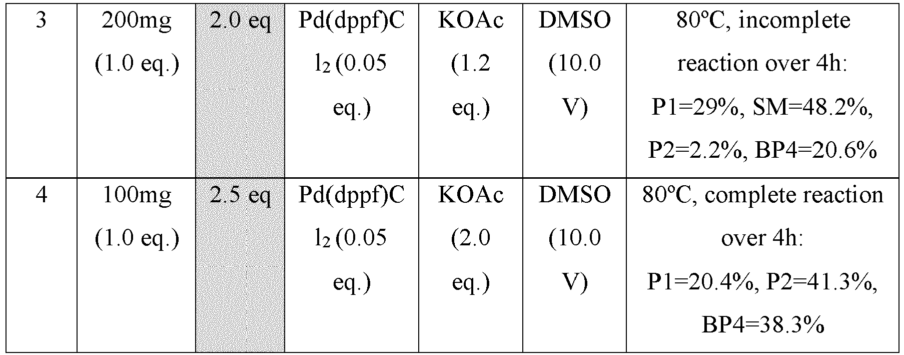

After the above optimizations, three batches of optimized preparations for scale-up were provided below:
DMSO (10 vol.) was added to reactor A under nitrogen. Compound C (1.0 eq.), bis(pinacolato)diboron (3.0 eq.), potassium acetate (2.5 eq.), 1,1'-bis(diphenylphosphino)ferrocene palladium dichloride (0.05 eq.), and purified water^{#} ((3.7%-KF)*m/2, kg)) were added sequentially under stirring. The mixture was heated to 80±10°C and stirred for at least 2 h at the controlled temperature. Additional purified water^{#} ((3.7%-KF)*m/2, kg)) was added under nitrogen, and the mixture was reacted for at least 2 hours while maintaining the temperature. A sample was taken and sent to HPLC for monitoring the reaction process (*standard: compound C* ≤ *2.0%*) (#: m is the mass of compound C and KF is the KF of compound C). If the reaction was not completed, the mixture was cooled to 20-30 °C, and additional 1,1'-bis(diphenylphosphino)ferrocene palladium dichloride (0.01 eq.) was added under nitrogen. The mixture was heated to 80±10 °C and stirred for at least 2 h while maintaining the temperature. A sample was taken and sent to HPLC for monitoring until the reaction was completed. The mixture was cooled to room temperature, and filtered by suction. The filtrate was collected, and concentrated under reduced pressure to 1.0 - 1.5 volume, and n-heptane was added. The mixture was stirred, and the precipitate was collected.

The precipitate was dried under vacuum at 40±5°C for at least 12 hours, and then the baking was stopped. The precipitate was cooled to below 30 °C, and then kept at the temperature for at least 1 hour. The precipitate was then collected.

### Step 2-2: Preparation of compound A and the crystal form VII thereof

The applicant has carried out in-depth research on the reaction for obtaining compound A, and has carried out step-by-step optimization of various conditions in the reaction, as detailed below:

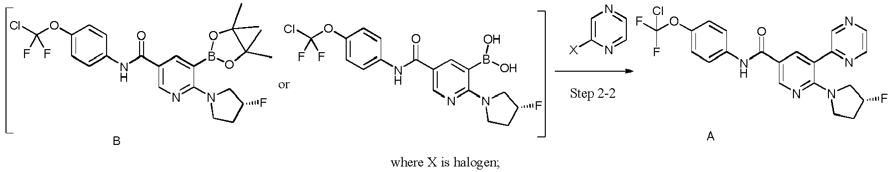

In the following tables, the following abbreviations were used:

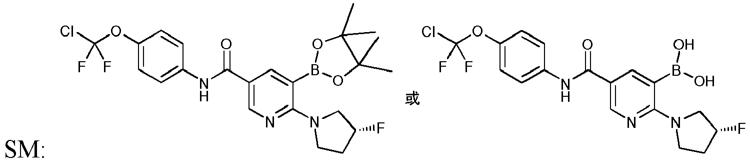

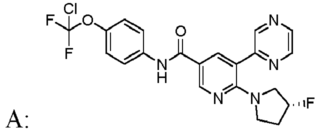

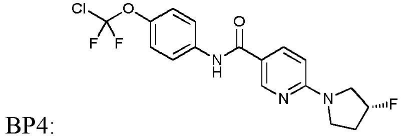

### 1 Optimization of halopyrazines

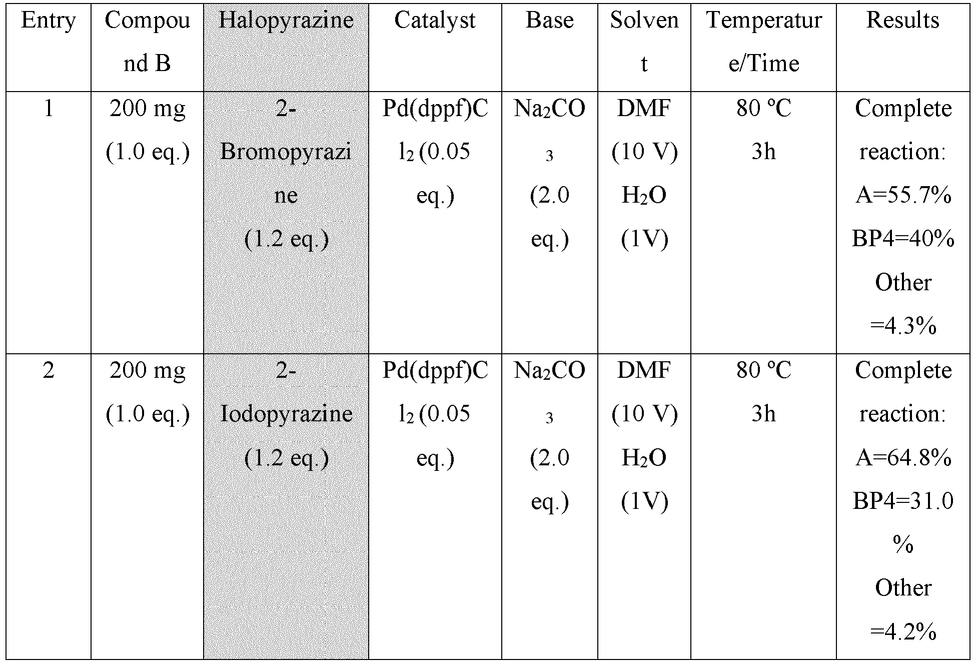

### 2)Optimization of base types

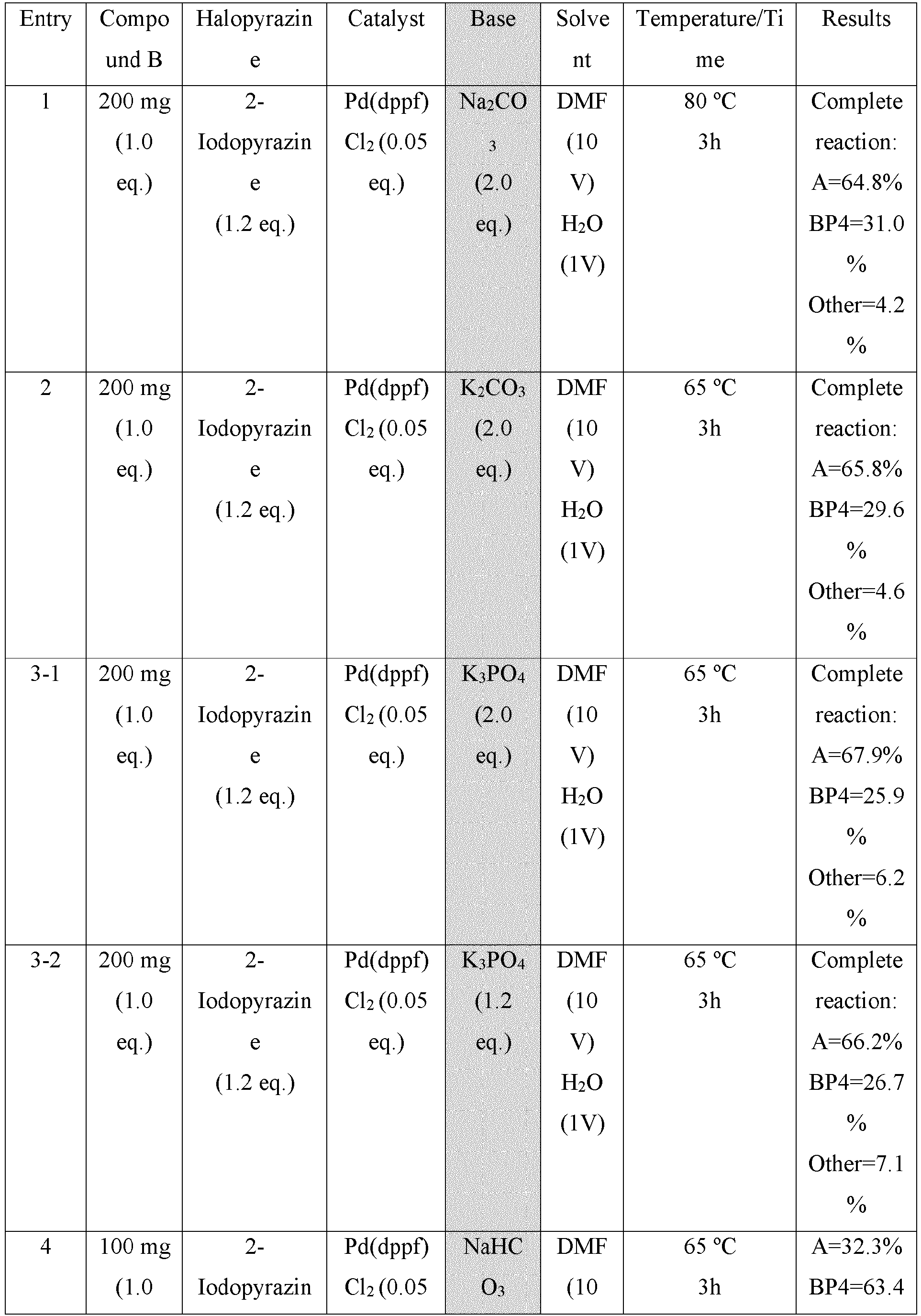

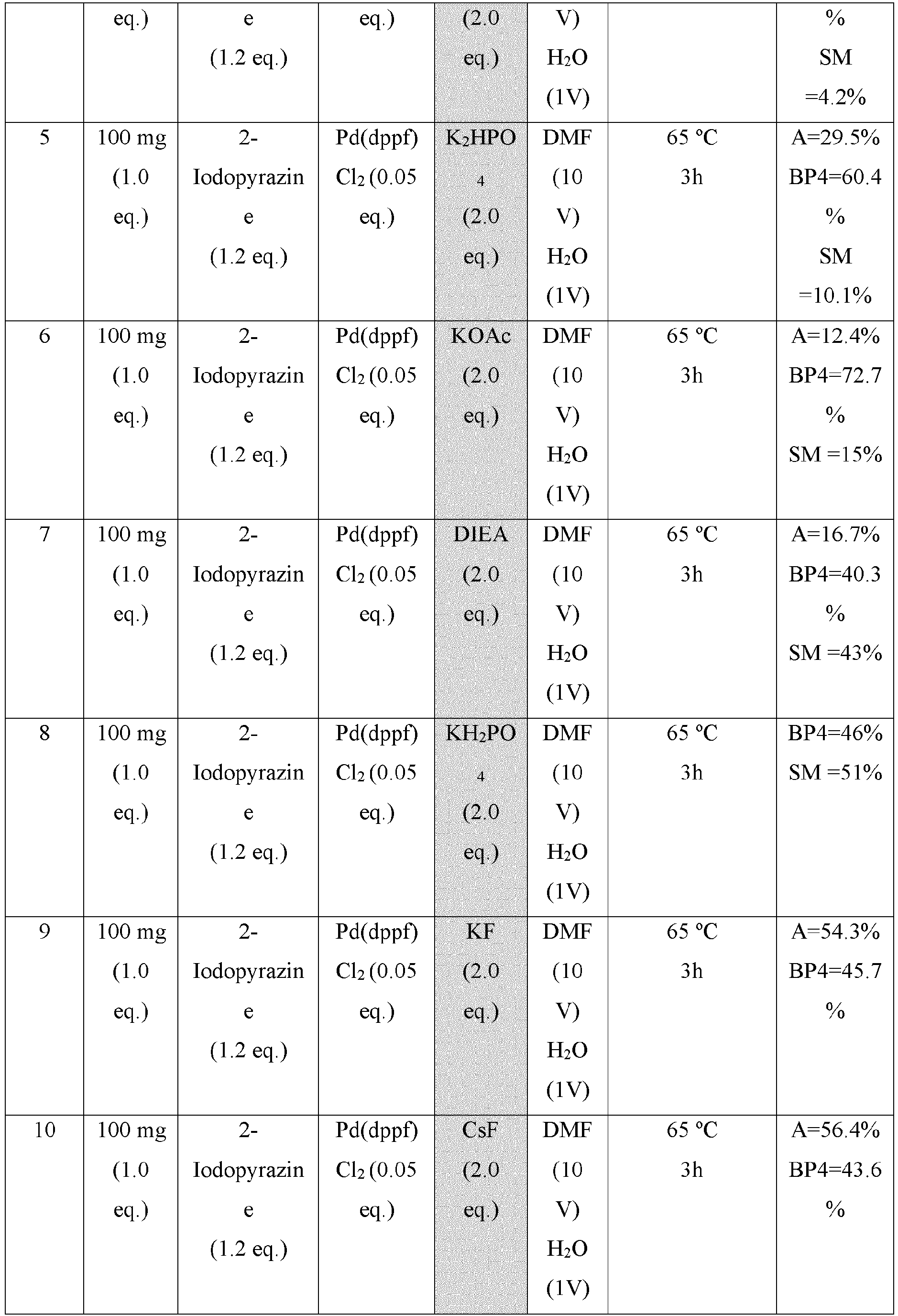

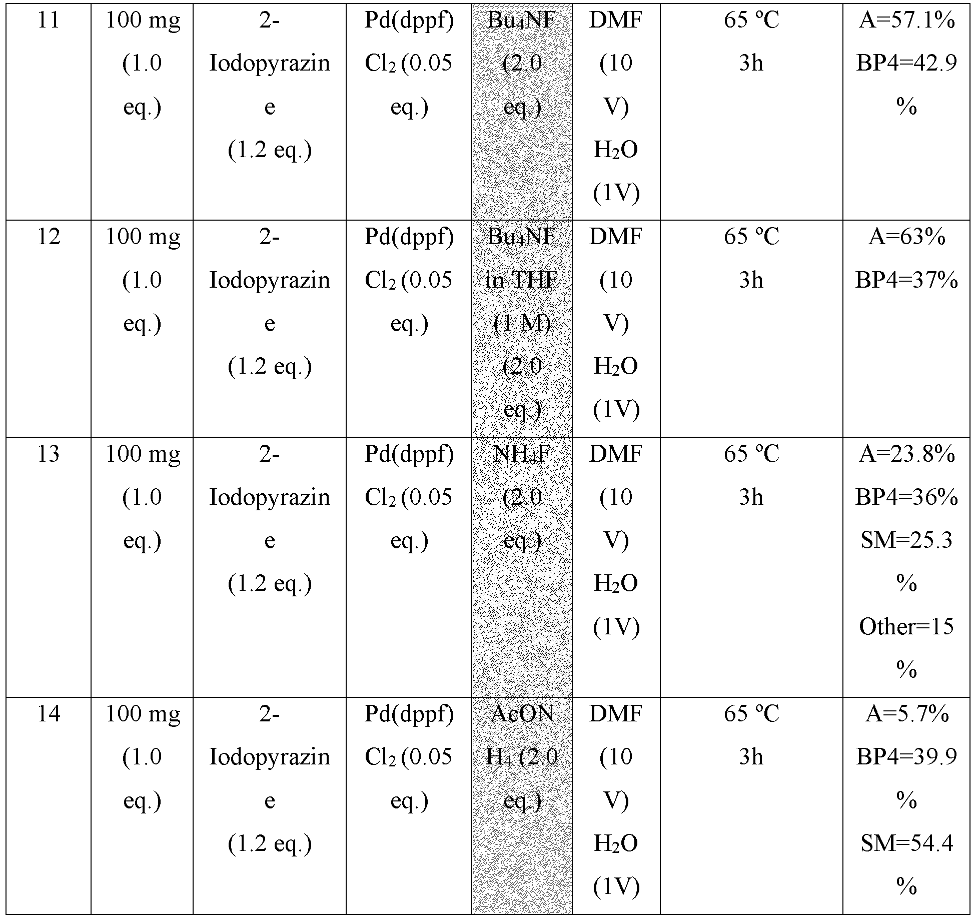

### 3)Optimization of the amount of catalyst using 1M solution of Bu₄NF in THF as base

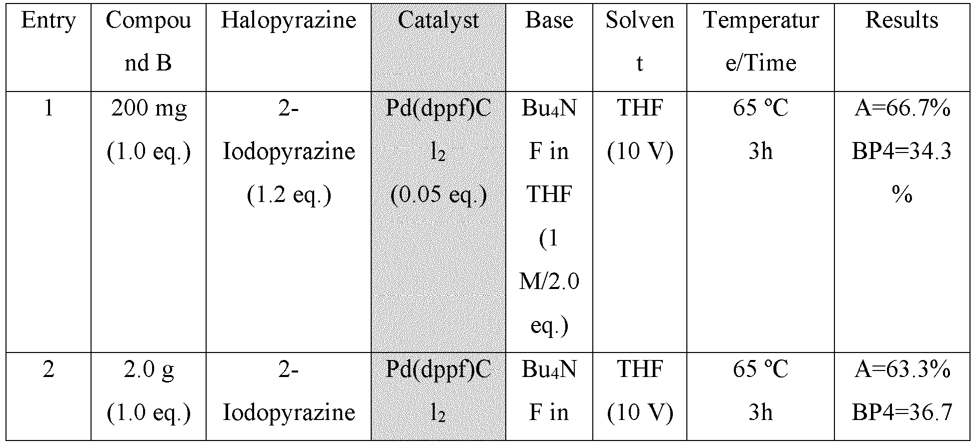

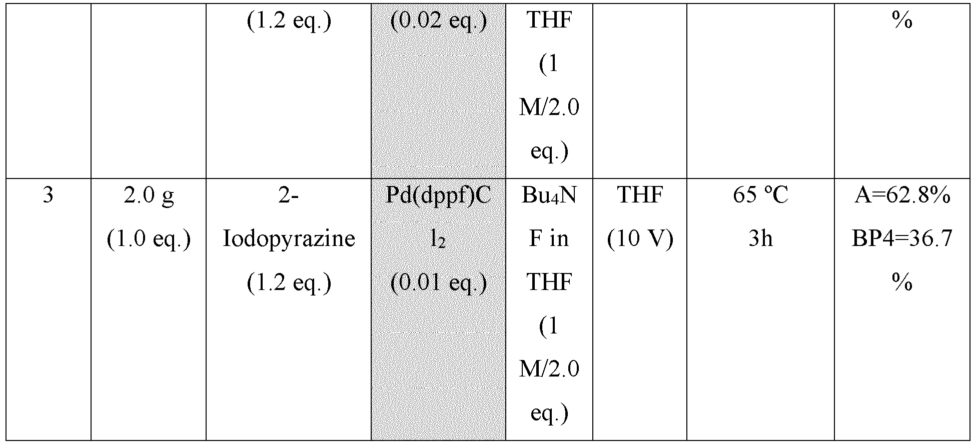

### 4)Optimization of type and amount of catalyst using K₃PO₄ as base

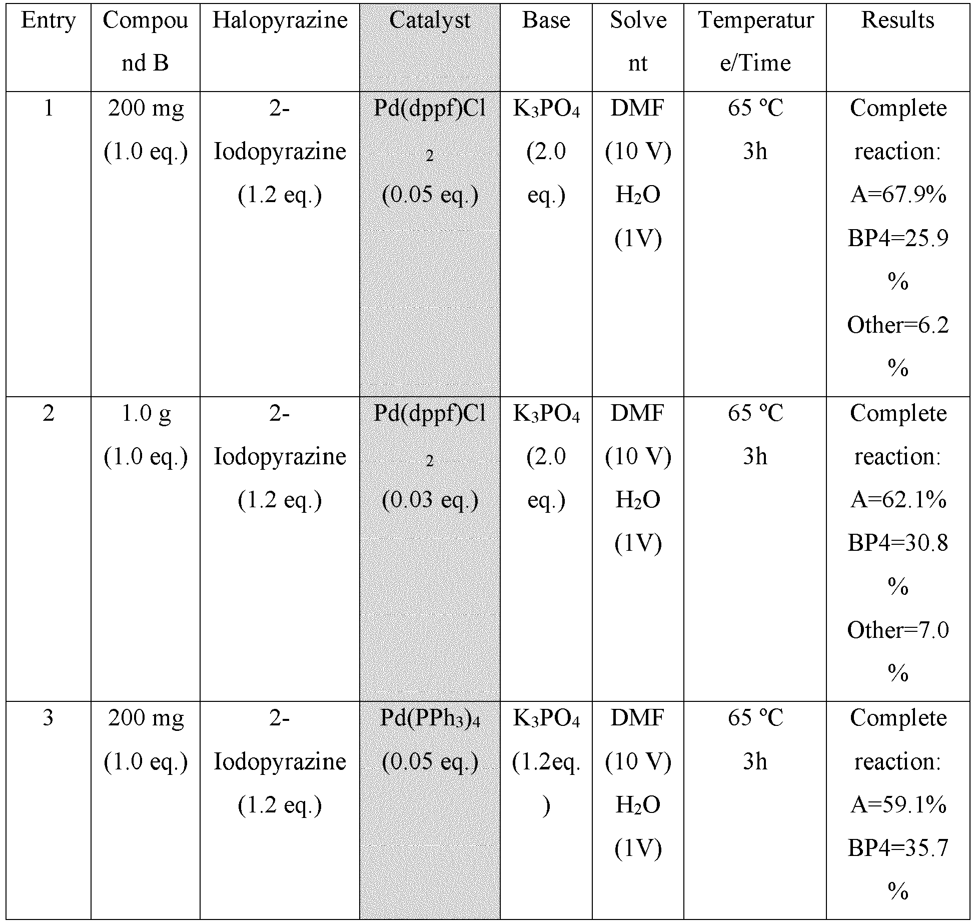

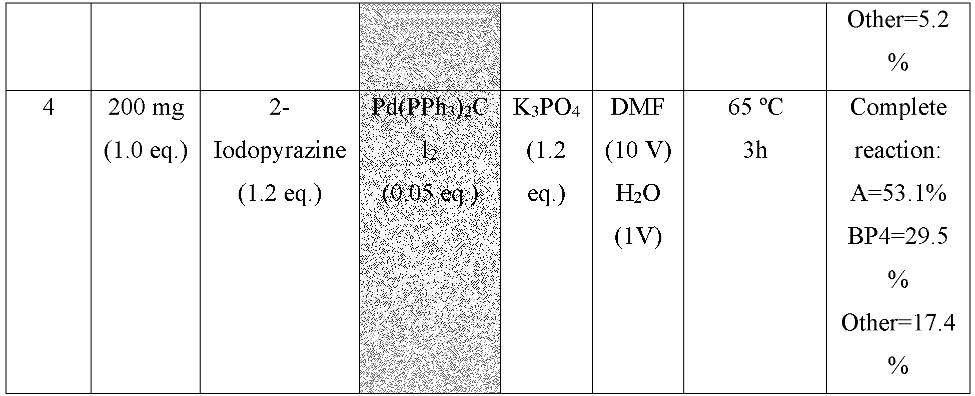

### 5)Optimization of solvents using 1M solution of Bu₄NF in THF as base

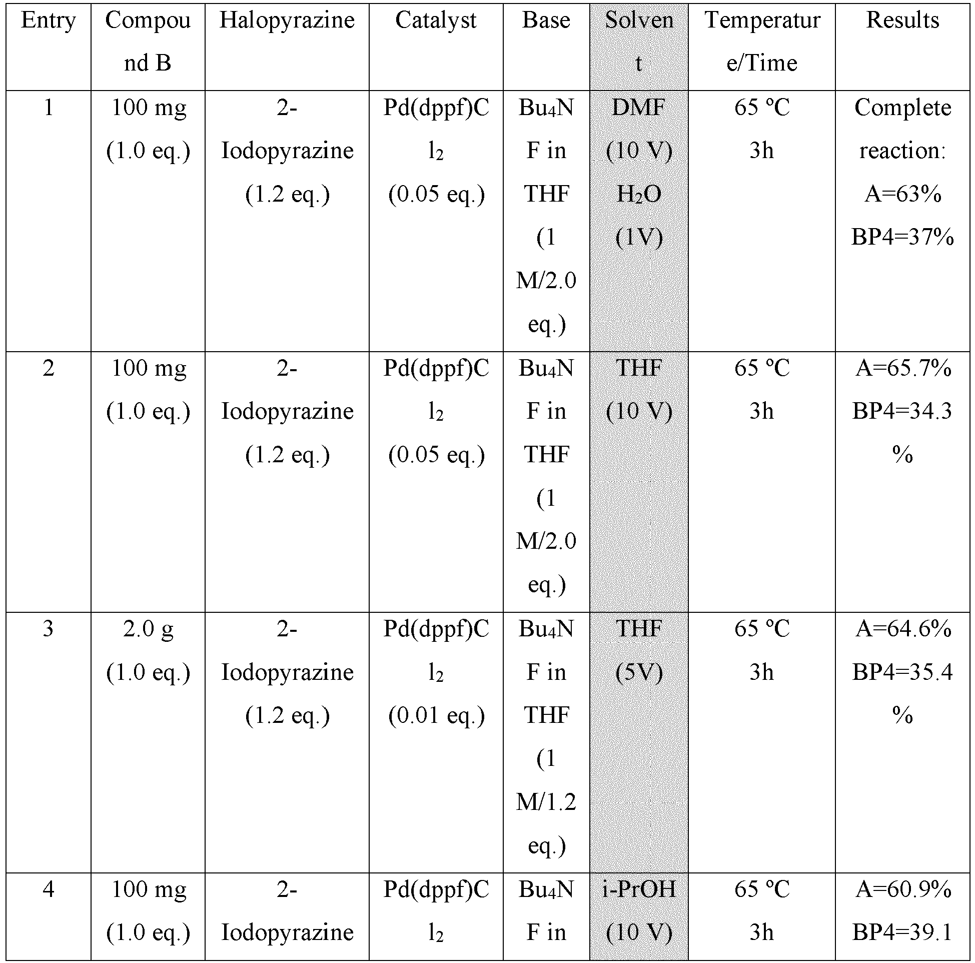

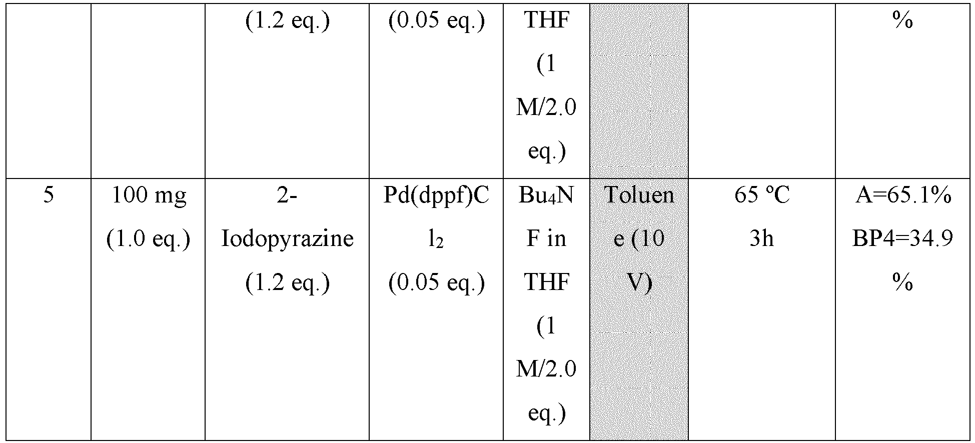

### 6)Optimization of temperature using 1M solution of Bu₄NF in THF as base

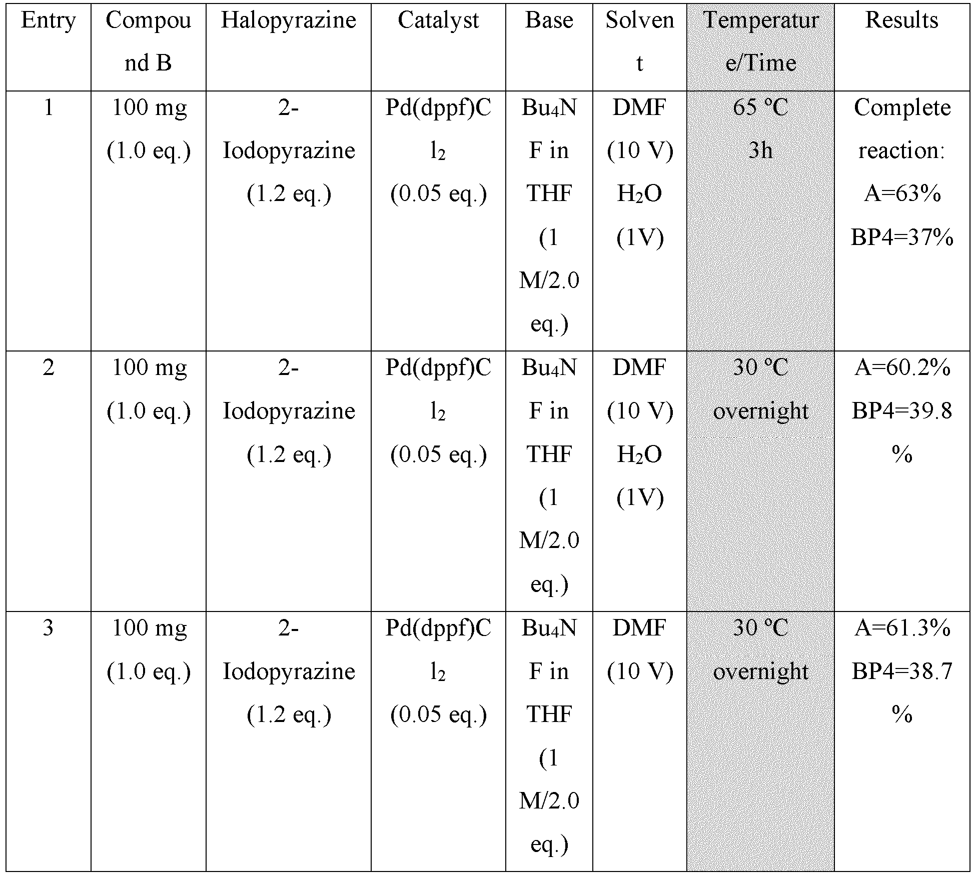

### 7)Optimization of the amount of solvent using K₃PO₄ as base

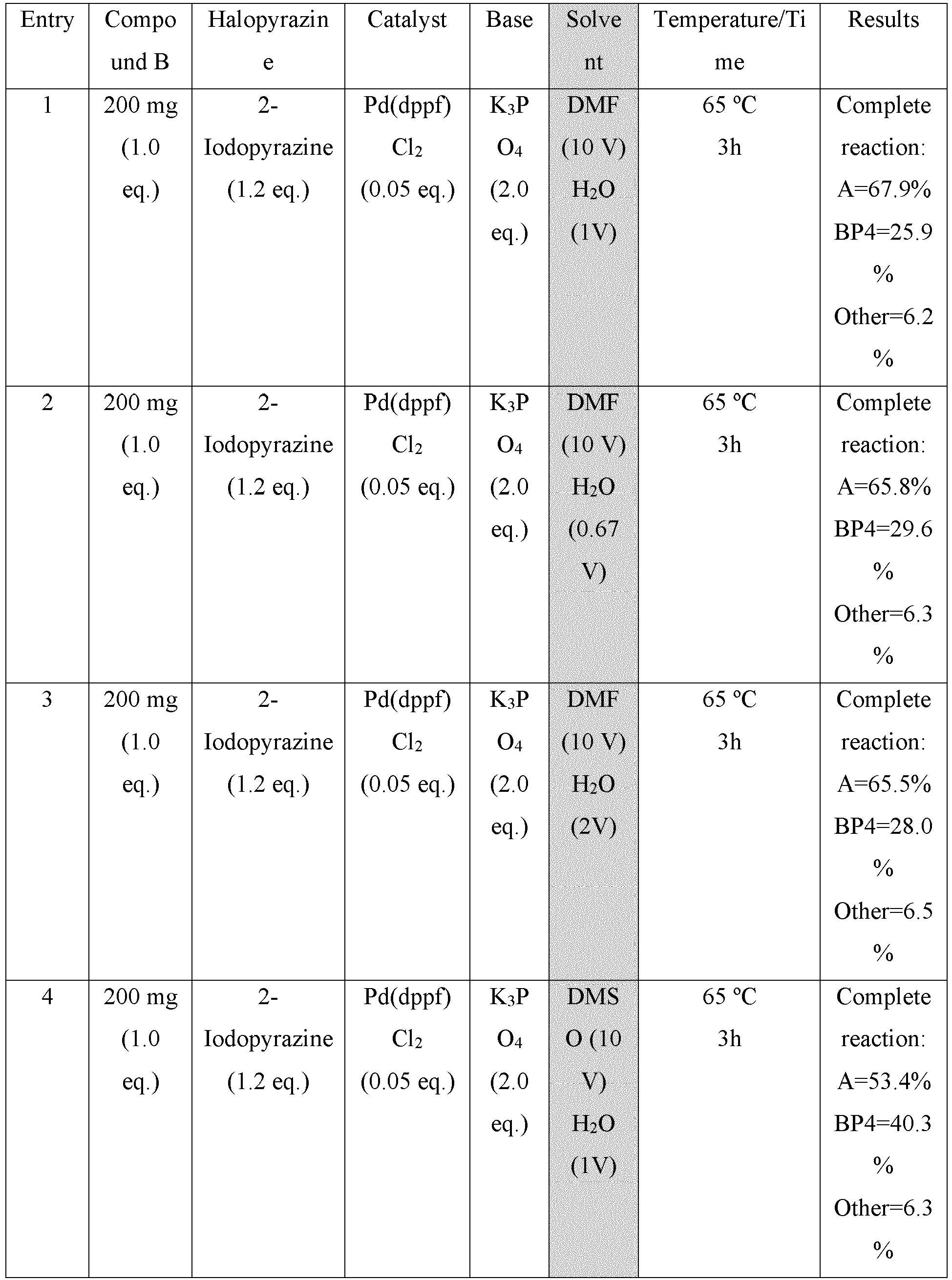

### 8)Optimization of the temperature using K₃PO₄ as base

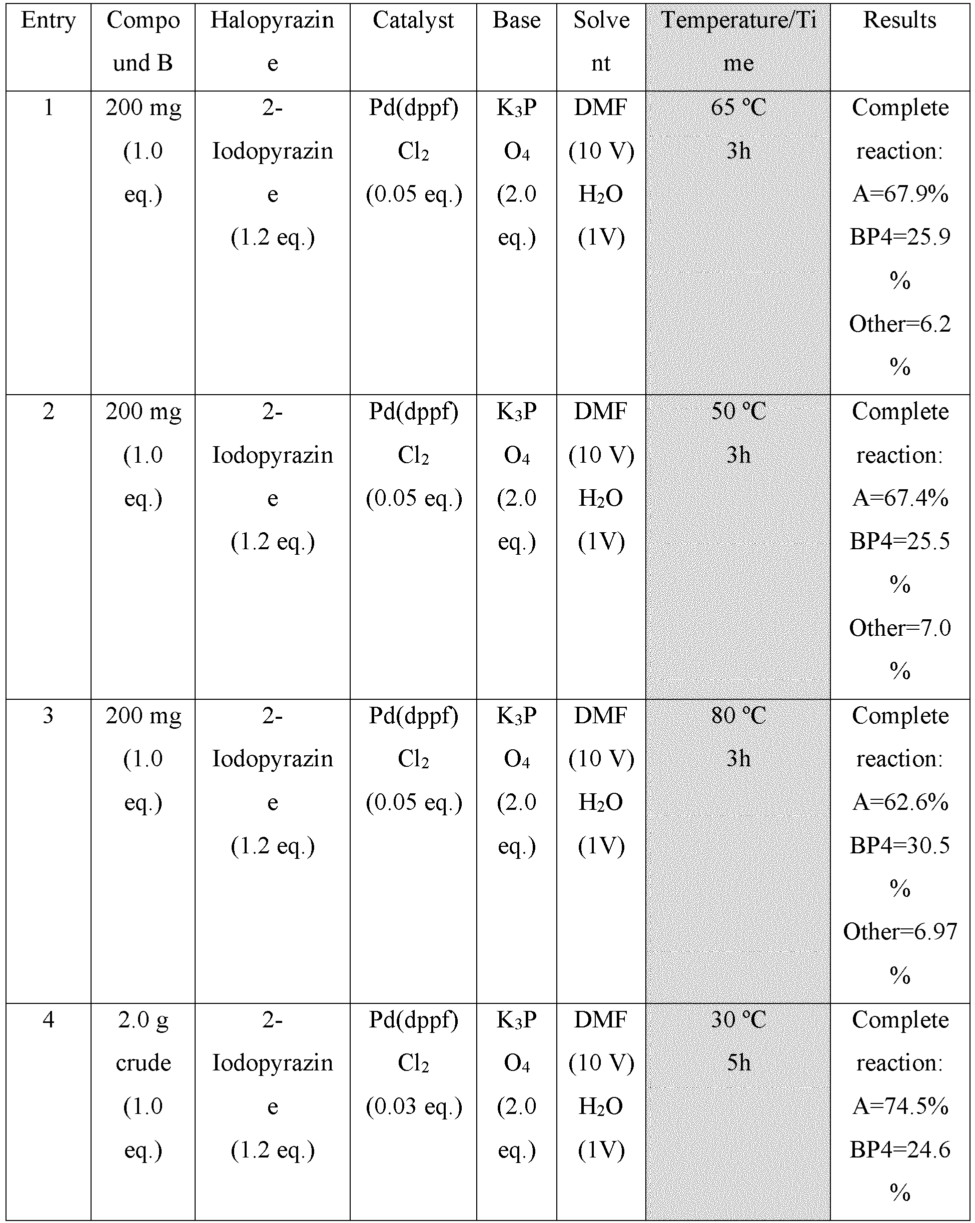

After the above optimizations, three batches of optimized preparations for scale-up were provided below:
Tetrahydrofuran (5 vol.) was added to reactor A under nitrogen. Compound B (1.0 eq.), 2-iodopyrazine (1.2 eq.), tetrabutylammonium fluoride (1 M solution in tetrahydrofuran) (2.7 eq.), and 1,1'-bis(diphenylphosphino)ferrocene palladium dichloride (0.01 eq.) were added sequentially under stirring. The mixture was heated to 65±5 °C, and reacted for at least 3 hours while maintaining the temperature. A sample was taken and sent to HPLC for monitoring until the reaction was completed. The mixture was cooled to room temperature, and filtered by suction. The filtrate was collected, washed with water, concentrated, and then recrystallizated.

Recrystallization (I): Anhydrous ethanol (7 vol.) and p-toluenesulfonic acid monohydrate (1.5 eq.) were added to a reactor under nitrogen. The mixture was heated to 75±5 °C, and stirred for at least 1 hour while maintaining the temperature. The mixture was cooled to 20-30 °C, and stirred for at least 3 hours while maintaining the temperature. The mixture was centrifuged, and rinsed with anhydrous ethanol. The precipitate was collected. Acetone (6 vol.), demineralized water (6 vol.), the filter cake (1.0 eq.) and anhydrous sodium carbonate (1.5 eq.) were added sequentially to a reactor under nitrogen, and the mixture was stirred at 20-30 °C for at least 1 hour. The external temperature was controlled at 45±5 °C. The mixture was concentrated under reduced pressure to 6 - 8 volumes. The mixture was cooled to 20 - 30 °C, centrifuged, and rinsed with demineralized water. The precipitate was collected.

Slurrying: Demineralized water (10 volumes) was added under nitrogen, and the precipitate was added with stirring. The mixture was stirred for at least 1 hour while maintaining the temperature at 20-30 °C. The mixture was centrifuged, and rinsed with demineralized water, and the precipitate was collected.

Recrystallization (II): Anhydrous ethanol (10 vol.) was added to a reactor under nitrogen. The filter cake and p-toluenesulfonic acid monohydrate (1.5 eq.) were added with stirring. The mixture was heated to 75±5 °C, and stirred for at least 1 hour while maintaining the temperature. The mixture was cooled to 20-30 °C, and stirred for at least 3 hours while maintaining the temperature. The mixture was centrifuged, and rinsed with anhydrous ethanol, and the precipitate was collected. Acetone (6 vol.), demineralized water (6 vol.), the filter cake (1.0 eq.) and anhydrous sodium carbonate (1.5 eq.) were added sequentially to a reactor under nitrogen, and the mixture was stirred at 20-30 °C for at least 1 hour. The external temperature was controlled at 45±5 °C. The mixture was concentrated under reduced pressure to 6 - 8 volumes. The mixture was cooled to 20 - 30 °C, centrifuged, and rinsed with demineralized water, and the precipitate was collected. A sample was taken for HPLC detection. If the standard was not met, the operations of "slurrying of recrystallization (I)" and "recrystallization (II)" were repeated until the standard is met by HPLC detection. If the standard was met, the operation of "slurrying of recrystallization (I)" was performed and the precipitate was collected and sent to HPLC detection until the standard is met (*standard: the purity of the crystal form VII of compound A* ≥ *99.2%, impurity RRT0.37 <0.10%*)

The precipitate was dried under vacuum at 55±5°C for at least 12 hours, and then the baking was stopped. The precipitate was cooled to below 30 °C, and then kept at the temperature for at least 1 hour. A sample was taken to check the appearance, identified, and subjected to HPLC, and then the precipitate was collected. The product was put into a three-layer LDPE bag, and the bag was tied tightly. A desiccant was added between the second layer and the third layer of the LDPE bag. The mixture was weighed, labeled as intermediate, and then stored temporarily at room temperature.

According to the above synthetic method, three batches of the crystal form VII of compound A in pilot kilogram-scale have been prepared, and the results are listed in Table 2.2-2:

**Table 2.2-2: Batch production results of the crystal form VII of compound A**

| Batch No. | Material ratio in step 2-1 | | Material ratio in step 2-2 | | the crystal form VII of compound A | | |
|---|---|---|---|---|---|---|---|
| | | | | | Yield (weight) | Yield (%) | Purity |
| 1 | Compound C (1.0 eq.) | 3.145 kg | Compound B (1.0 eq.) | 2.846 kg | 1.54 kg | 49.0% | 99.7% |
| | B₂Pin₂ (3.0 eq.) | 5.16 kg | 2-Iodopyrazine (1.2 eq.) | 1.37 kg | | | |
| | Pd(dppf)Cl₂ (0.05 eq.) | 248 g | Pd(dppf)Cl₂ (0.01 eq.) | 39.8 g | | | |
| | | | | | | | *RRT0.58*=*0.06%* |
| | Potassium acetate (2.5 eq.) | 1.67 kg | TBAF(1 M) (2.7 eq.) | 14.5 L | | | |
| | | | | | | | *RRT0.74*=*0.08%* |
| | | | | | | | *RRT1.19*=*0.18%* |
| | DMSO (per volume) | 3.15 L | Solvent (THF/anhydrous ethanol/acetone/water) (per volume) | 2.8 L | | | |
| 2 | Compound C (1.0 eq.) | 3.155 kg | Compound B (1.0 eq.) | 2.778 kg | 1.436 kg | 45.5% | 99.7% |
| | | | | | | | *RRT0.37*=*0.06%* |
| | B₂Pin₂ (3.0 eq.) | 5.17 kg | 2-Iodopyrazine (1.2 eq.) | 1.33 kg | | | |
| | | | | | | | *RRT0.58*=*0.05%* |
| | | | | | | | *RRT0.74*=*0.07%* |
| | Pd(dppf)Cl₂ (0.05 eq.) | 249 g | Pd(dppf)Cl₂ (0.01 eq.) | 39.8 g | | | |
| | | | | | | | *RRT1.19*=*0.16%* |
| | Potassium acetate (2.5 eq.) | 1.67 kg | TBAF(1 M) (2.7 eq.) | 14.6 L | | | |
| | DMSO (per volume) | 3.16 L | Solvent (THF/anhydrous ethanol/acetone/water) (per volume) | 2.8 L | | | |
| 3 | Compound C (1.0 eq.) | 3.096 kg | Compound B (1.0 eq.) | 2.694 kg | 1.355 kg | 43.8% | 99.8% |
| | B₂Pin₂ (3.0 eq.) | 5.08 kg | 2-Iodopyrazine (1.2 eq.) | 1.29 kg | | | |
| | Pd(dppf)Cl₂ (0.05 eq.) | 245 g | Pd(dppf)Cl₂ (0.01 eq.) | 37.7 g | | | |
| | Potassium acetate (2.5 eq.) | 1.64 kg | TBAF(1 M) (2.7 eq.) | 14.2 L | | | *RRT0.58*=*0.08%* |
| | | | | | | | *RRT1.19*=*0.16%* |
| | DMSO (per volume) | 3.1 L | Solvent (THF/anhydrous ethanol/acetone/water) (per volume) | 2.7 L | | | |

### Step 3: Preparation of the crystal form VI of compound A

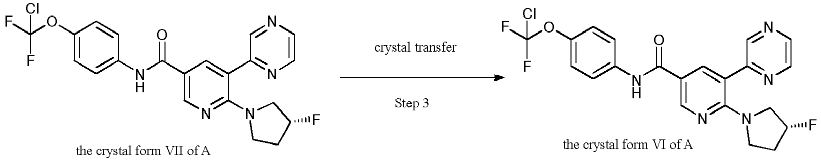

A solution of the crystal form VII of compound A in acetone (1.0 eq. dissolved in 15 volumes of acetone) that had passed through a microporous cartridge was added to a reactor under nitrogen. The temperature was controlled at 50-80 °C, and the mixture was evaporated to 7 - 9 volumes at atmospheric pressure. n-Heptane (7.5 volumes) that had passed through a microporous cartridge was added, and the mixture was evaporated to 7 - 9 volumes at atmospheric pressure. n-Heptane (7.5 volumes) that had passed through a microporous cartridge was added, and the mixture was evaporated to 7 - 9 volumes at atmospheric pressure. The internal temperature was increased to 75±5 °C. The mixture was cooled to 20-30 °C, and stirred for at least 3 hours while maintaining the temperature. The mixture was centrifuged, and rinsed with n-heptane that had passed through a microporous cartridge. The precipitate was collected and dried under vacuum at 55±5°C for at least 12 hours. A small stream of nitrogen gas was introduced during the drying process. The precipitate was cooled to below 30°C for at least 1 hour and sampled for KF detection (*standard: KF* ≤ *0.5%*)*.* A sample was taken for solvent residue detection (*standard: acetone* ≤ *5000 ppm, n-heptane* ≤ *5000 ppm, tetrahydrofuran* ≤ *720 ppm*)*.* A sample was sent for HPLC analysis (*standard: the crystal form VI of compound A has a purity of* ≥ *99.2%*)*.* The product was put into a double-layer low-density polyethylene bag. The bag was tied tightly, and weighed. The outer aluminum foil bag was heat-sealed and put in a fiber drum. The product was stored in an airtight container at room temperature.

According to the above synthetic method, three batches of the crystal form VI of compound A in pilot kilogram-scale have been prepared, and the results are listed in Table 2.2-3:

| Batch No. | Material ratio | | The crystal form VI of compound A | | | |
|---|---|---|---|---|---|---|
| | | | Yield (weight) | Yield (%) | Purity | Chiral purity |
| 1 | The crystal form VII of compound A (1.0 eq.) | 1.5 kg | 1.414 kg | 95.7% | 99.7% | 99.6% |
| | | | | | *RRT0.74*=*0.08%* | |
| | Solvent (acetone/n-heptane) (per volume) | 1.5 L | | | *RRT1.19*=*0.17%* | |
| 2 | The crystal form VII of compound A (1.0 eq.) | 1.396 kg | 1.298 kg | 93.0% | 99.7% | 99.5% |
| | | | | | *RRT0.74*=*0.08%* | |
| | Solvent (acetone/n-heptane) (per volume) | 1.4 L | | | *RRT1.19*=*0.17%* | |
| 3 | The crystal form VII of compound A (1.0 eq.) | 1.314 kg | 1.234 kg | 93.9% | 99.8% | 99.6% |
| | Solvent (acetone/n-heptane) (per volume) | 1.3 L | | | *RRT1.19*=*0.15%* | |

### Example 3 Preparation of various crystal forms of compound A

In the screening of various crystal forms of the free base of compound A, compound A was used as the starting material, and several methods of cooling crystallization, evaporating crystallization, suspension crystal transformation, dissolution-precipitation crystallization, heat treatment and grinding crystallization were used to screen various crystal forms of the free base of compound A. A total of 11 crystal forms were found: one anhydrous crystal form (the crystal form VI of compound A), two hydrate crystal forms (the crystal form III of compound A and the crystal form VII of compound A), and eight solvate crystal forms (the crystal form I of compound A, the crystal form II of compound A, the crystal form IV of compound A, the crystal form V of compound A, the crystal form VIII of compound A, the crystal form IX of compound A, the crystal form X of compound A and the crystal form XI of compound A).

### Example 3.1 Screening of 13×13 (1: 1) binary solvents in a 96-well plate

A certain amount of the crystal form I of compound A was added to 13 vials, and the corresponding solvent (methanol, ethanol, isopropanol, isobutanol, 2-butanone, tetrahydrofuran, acetonitrile, methyl tert-butyl ether, acetone, water, toluene, ethyl acetate, isopropyl acetate, respectively) was added gradually to 3 mL, where the crystal form I of compound A formed a suspension in methanol, ethanol, isopropanol, isobutanol, toluene, methyl tert-butyl ether and water, and formed a solution in other solvents. 2 mL of the suspension/solution was filtered, and the resulting filtrate was used for the screening of binary solvents in a 96-well plate. The above corresponding filtrates were distributed two by two in a 96-well plate in a volume of 100 µL for each filtrate. The 96-well plate was sealed with a sealing film, and allowed to evaporate to dryness in a fume hood at room temperature. Information of the 13 solvents in the 96-well plate is given in Table 3.1.

**Table 3.1: Information of 13×13 solvents in the 96-well plate**

| | Me tha nol | Eth ano l | Iso pro pan ol | Isob utan ol | 2-Buta none | TH F | AC N | MT BE | Ac eto ne | Wa ter | To lu en e | Eth yl ace tate | Isopro pyl acetat e |
|---|---|---|---|---|---|---|---|---|---|---|---|---|---|
| Methano l | A1 | A2 | A3 | A4 | A5 | A6 | A7 | A8 | A9 | A1 0 | A 11 | A1 2 | B1 |
| Ethanol | / | B2 | B3 | B4 | B5 | B6 | B7 | B8 | B9 | B1 0 | B1 1 | B1 2 | C1 |
| Isopropa nol | / | / | C2 | C3 | C4 | C5 | C6 | C7 | C8 | C9 | C1 0 | C1 1 | C12 |
| Isobutan ol | / | / | / | D1 | D2 | D3 | D4 | D5 | D6 | D7 | D 8 | D9 | D10 |
| 2-Butanon e | / | / | / | / | D11 | D1 2 | E1 | E2 | E3 | E4 | E5 | E6 | E7 |
| THF | / | / | / | / | / | E8 | E9 | E10 | E1 1 | E1 2 | F1 | F2 | F3 |
| ACN | / | / | / | / | / | / | F4 | F5 | F6 | F7 | F8 | F9 | F10 |
| MTBE | / | / | / | / | / | / | / | F11 | F1 2 | G1 | G 2 | G3 | G4 |
| Acetone | / | / | / | / | / | / | / | / | G5 | G6 | G 7 | G8 | G9 |
| Water | / | / | / | / | / | / | / | / | / | G1 0 | G 11 | G1 2 | H1 |
| Toluene | / | / | / | / | / | / | / | / | / | / | H 2 | H3 | H4 |
| Ethyl acetate | / | / | / | / | / | / | / | / | / | / | / | H5 | H6 |
| Isopropy l acetate | / | / | / | / | / | / | / | / | / | / | / | / | H7 |

PLM and XRPD tests were performed on the solid precipitated in the 96-well plate, and two new crystal forms were obtained, named as the crystal form II and the crystal form III, wherein the crystal form II was obtained in acetonitrile/methyl tert-butyl ether; and the crystal form III was obtained in acetone/water.

### Example 3.2 Research of suspension crystal transformation in 13 single solvents

The remaining suspension of the crystal form I of compound A in methanol, ethanol, isopropanol, isobutanol, toluene, methyl tert-butyl ether and water from Example 3.1 was slurried and stirred at room temperature for 3 days. Solids were obtained in methanol, ethanol, isopropanol, isobutanol, water, toluene and methyl tert-butyl ether, and subjected to PLM and XRPD tests. The results showed that four new crystal forms were obtained in methanol, isobutanol, methyl tert-butyl ether and toluene, in addition to the crystal form I and the crystal form III. Specifically, the crystal form I was obtained in the suspension of ethanol; the crystal form III was obtained in the suspension of water; a new crystal form, named the crystal form IV, was obtained in the suspension of methanol, which was a solvate of methanol; a new crystal form, named the crystal form V, was obtained in the suspension of isobutanol, which was a solvate of isobutanol; a new crystal form, named the crystal form VI, was obtained in the suspension of methyl tert-butyl ether, which was a anhydrous crystal form; a new crystal form, named the crystal form VII, was obtained in the suspension of toluene, which was a hydrate.

### Example 3.3 Research of evaporating crystallization in 13 single solvents

The remaining 13 single filtrates for spreading the 96-well plate from Example 3.1 were dried in a fume hood. No solid was obtained in water, isopropanol and isobutanol, but solids were found in all other solvents. The solids were tested for PLM and XRPD. The results showed that in addition to the crystal form II, the crystal form III, the crystal form IV and the crystal form VII, a new crystal form, named VIII, was also obtained. Specifically, the crystal form II was obtained as a solvate of acetonitrile by evaporation in a single solvent of acetonitrile; the crystal form III was obtained as a hydrate by evaporation in a single solvent of acetone; the crystal form IV was obtained as a solvate of methanol by evaporation in methanol; the crystal form VII was obtained as a hydrate by evaporation in toluene; and a new crystal form, named the crystal form VIII, was obtained as an solvate of ethanol by evaporation in ethanol.

### Example 3.4 Research of dissolution-precipitation crystallization

A certain amount of compound A was added to the corresponding solvent, and a certain amount of anti-solvent was added after the compound A was dissolved. The test conditions and results are shown in Table 3.4.

**Table 3.4 Test conditions and results of dissolution-precipitation crystallization**

| No. | Temper ature | Solvent | Anti-solvent | Vₛₒₗᵥₑₙₜ: Vₐₙₜᵢ₋ₛₒₗᵥₑₙₜ | Initial phenomenon | Result |
|---|---|---|---|---|---|---|
| 1 | 50°C | ACN | Water | 1: 4 | Suspension | The crystal form III |
| 2 | 50°C | ACN | MTBE | 1: 4 | Clear liquid | No solid |
| 3 | RT | THF | IPA | 1: 6 | Clear liquid | The crystal formIX |
| 4 | RT | THF | Water | 2: 3 | Oil | The crystal form X |
| 5 | RT | THF | MTBE | 1: 6 | Clear liquid | The crystal form VI |
| 6 | RT | THF | n-Heptane | 2: 5 | Oil | The crystal form VI |
| 7 | 50°C | Acetone | Water | 3: 2 | Suspension | The crystal form III |
| 8 | 50°C | Acetone | MTBE | 1: 4 | Clear liquid | No solid |
| 9 | 50°C | Acetone | n-Heptane | 1: 4 | Clear liquid | The crystal form VI |
| 10 | 50°C | EtOAc | MTBE | 1: 4 | Clear liquid | The crystal form VI |
| 11 | 50°C | EtOAc | n-Heptane | 1: 3 | Suspension | The crystal form VI |

The crystal form I was used as the starting material. Solids were produced when the anti-solvent was added to acetonitrile/water, acetone/water, and ethyl acetate/n-heptane. An oil was produced when the anti-solvent was added to tetrahydrofuran/water, and tetrahydrofuran/n-heptane, and then became solids after 1 hour of stirring. After 15 minutes of stirring, solids were produced in tetrahydrofuran/isopropanol, and acetone/n-heptane. After 3 hours of stirring, solids were produced in tetrahydrofuran/methyl tert-butyl ether, and ethyl acetate/methyl tert-butyl ether. Other solutions were clear after stirring overnight. The resulting solids were tested for PLM and XRPD. XRPD results showed that in addition to the crystal form III (hydrate) and the crystal form VI (anhydrous crystal form), a new crystal form, named the crystal form IX, was obtained as a solvate of isopropanol, ethanol and tetrahydrofuran in tetrahydrofuran/isopropanol, and another new crystal form, named the crystal form X, was obtained as a solvate of tetrahydrofuran in tetrahydrofuran/water.

### Example 3.5 Research of cooling crystallization

About 30 mg of the crystal form I of compound A was added to different solvents and heated until dissolved. The resulting solution was then slowly cooled to room temperature. The test conditions and results are listed in Table 3.5. PLM and XRPD tests were performed on the resulting solid crystal forms.

**Table 3.5: Test conditions and results of cooling crystallization**

| No. | Solvent | Solubility (mg/mL) | Phenomenon of the test | Result |
|---|---|---|---|---|
| 1 | MeOH | 34.1 | The mixture became clear at 50 °C and a solid precipitated after stirring at room temperature for 30 minutes. | The crystal form IV |
| 2 | Isobutanol | 10.1 | The mixture became clear at 70 °C and was stirred at room temperature overnight. | No solid |
| 3 | ACN | 298.0 | The mixture became clear at 50 °C and a solid precipitated during cooling. | The crystal form XI |
| 4 | Acetone | 150.5 | The mixture became clear at 50 °C and was stirred at room temperature overnight. | No solid |

XRPD results showed that in addition to the crystal form IV, a new crystal form, named the crystal form XI, was obtained as a solvate of acetonitrile in acetonitrile solution.

### Example 4 Characterization of various crystal forms of the free base of compound A

The various crystal forms obtained in Example 3 were characterized.

### Example 4.1 Characterization of the crystal form I of compound A

The crystal form I of the compound was prepared according to the method in Example 1, and characterized by XRPD, PLM, ¹H NMR, DSC and TGA. The specific results are as follows:

### XRPD analysis

FIG. 1 shows the XRPD data of the crystal form I of compound A acquired according to General method 1. A list of XRPD peaks at diffraction angles 2θ° (°2θ)±0.2 °2θ and relative intensities thereof are provided in Table 4.1.

**Table 4.1: XRPD peak list (2θ°) of the crystal form I of compound A**

| Peak | Angle °2θ±0.2 °2θ | Relative intensity % |
|---|---|---|
| 1 | 6.3 | 9.0 |
| 2 | 9.6 | 48.1 |
| 3 | 10.7 | 14.7 |
| 4 | 12.2 | 23.6 |
| 5 | 12.7 | 6.0 |
| 6 | 14.5 | 1.9 |
| 7 | 16.2 | 14.7 |
| 8 | 16.7 | 3.3 |
| 9 | 17.1 | 3.6 |
| 10 | 17.4 | 3.5 |
| 11 | 17.8 | 3.1 |
| 12 | 18.3 | 59.6 |
| 13 | 19.3 | 25.8 |
| 14 | 19.8 | 3.3 |
| 15 | 20.9 | 6.7 |
| 16 | 21.4 | 6.7 |
| 17 | 21.8 | 31.2 |
| 18 | 22.5 | 31.3 |
| 19 | 22.8 | 11.9 |
| 20 | 23.4 | 10.2 |
| 21 | 23.7 | 2.3 |
| 22 | 24.4 | 100.0 |
| 23 | 24.9 | 36.3 |
| 24 | 26.2 | 4.2 |
| 25 | 27.8 | 20.3 |
| 26 | 28.2 | 7.5 |
| 27 | 28.9 | 1.8 |
| 28 | 29.3 | 4.9 |
| 29 | 30.8 | 5.6 |
| 30 | 31.5 | 1.8 |
| 31 | 31.9 | 2.3 |
| 32 | 32.9 | 2.5 |
| 33 | 33.8 | 2.8 |
| 34 | 34.4 | 1.7 |

### PLM analysis

The collected crystal form I of compound A was subjected to PLM analysis by General method 2. It was observed that the sample was irregular sheet-like particles with a particle size of less than 50 µm.

### ¹H NMR analysis

The ¹H NMR data of the crystal form I of compound A is as follows: ¹H NMR (300 MHz, DMSO-d₆) δ 10.27 (s, 1H), 8.90 (d, *J=* 1.4 Hz, 1H), 8.84 (d, *J=* 2.3 Hz, 1H), 8.75 (dd, *J* = 2.5, 1.5 Hz, 1H), 8.65 (d, *J* = 2.6 Hz, 1H), 8.25 (d, *J* = 2.3 Hz, 1H), 7.93 - 7.79 (m, 2H), 7.35 (d, *J* = 9.0 Hz, 2H), 5.30 (d, *J* = 53.4 Hz, 1H), 3.63 - 3.45 (m, 1H), 3.41 - 3.34 (m, 2H), 3.27 - 3.18 (m, 1H), 2.20 - 1.87 (m, 2H).

### DSC and TGA thermal analysis

The collected crystal form I of compound A was subjected to DSC analysis by General method 4. DSC analysis showed a melting endothermic peak at 172.95 °C with an onset temperature of 170.67 °C. The crystal form I of compound A was heated to 150 °C by DSC and then the solvent was removed. The crystal form was changed into the crystal form VI, as shown in FIG. 3.

The collected crystal form I of compound A was subjected to TGA analysis by General method 5. TGA showed a weight loss of about 5.1% prior to 200°C mainly due to ethanol.

¹H NMR, DSC and TGA analysis indicated that the crystal form I of compound A was a solvate of ethanol.

### DVS analysis

FIG. 2 shows the DVS curve of the crystal form I of compound A acquired by General method 6. DVS analysis shows that the sample was slightly hygroscopic, wherein the water absorption was about 0.2% under the condition of 0-60%RH, about 0.7% under the condition of 0-80%RH, and about 1.3% under the condition of 0-90%RH.

### Example 4.2 Characterization of the crystal form II of compound A

The crystal form II of compound A was obtained by evaporation in a single acetonitrile solvent according to the method in Example 3.3, and characterized by XRPD, PLM, ¹H NMR, DSC and TGA. The specific results are as follows:

### XRPD analysis

FIG. 4 shows the XRPD data of the crystal form II of compound A acquired according to General method 1. A list of XRPD peaks at diffraction angles 2θ° (°2θ)±0.2 °2θ and relative intensities thereof are provided in Table 4.2.

**Table 4.2: XRPD peak list (2θ°) of the crystal form II of compound A**

| Peak | Angle °2θ±0.2 °2θ | Relative intensity % |
|---|---|---|
| 1 | 5.8 | 42.3 |
| 2 | 6.4 | 48.9 |
| 3 | 10.8 | 33.2 |
| 4 | 11.0 | 90.1 |
| 5 | 11.6 | 60.1 |
| 6 | 11.8 | 16.5 |
| 7 | 13.0 | 52.5 |
| 8 | 14.0 | 20.9 |
| 9 | 14.9 | 43.1 |
| 10 | 15.3 | 33.5 |
| 11 | 16.4 | 47.7 |
| 12 | 17.1 | 100 |
| 13 | 18.1 | 11.8 |
| 14 | 19.0 | 8.7 |
| 15 | 19.3 | 31.9 |
| 16 | 19.6 | 54.4 |
| 17 | 19.8 | 50.6 |
| 18 | 20.6 | 39.2 |
| 19 | 20.8 | 13.8 |
| 20 | 21.7 | 12.2 |
| 21 | 22.3 | 20.7 |
| 22 | 22.9 | 53.7 |
| 23 | 23.3 | 36.6 |
| 24 | 23.9 | 10.8 |
| 25 | 25.1 | 43.2 |
| 26 | 25.8 | 7.9 |
| 27 | 26.4 | 16 |
| 28 | 26.9 | 35.9 |
| 29 | 28.2 | 13.3 |
| 30 | 28.9 | 6.1 |
| 31 | 29.4 | 7 |
| 32 | 30.1 | 8.6 |
| 33 | 30.3 | 9.1 |
| 34 | 31.7 | 5.2 |
| 35 | 32.1 | 9 |
| 36 | 34.1 | 4.8 |
| 37 | 36.3 | 4.7 |
| 38 | 38.3 | 5.4 |

### PLM analysis

The collected crystal form II of compound A was subjected to PLM analysis by General method 2. It was observed that the sample was irregular lumps.

### ¹H NMR analysis

The ¹H NMR data of the crystal form II of compound A are as follows: ¹H NMR (400 MHz, DMSO-d₆) δ 10.27 (s, 1H), 8.90 (d, *J=* 1.5 Hz, 1H), 8.84 (d, *J=* 2.4 Hz, 1H), 8.75 (dd, *J* = 2.5, 1.5 Hz, 1H), 8.65 (d, *J* = 2.6 Hz, 1H), 8.24 (d, *J* = 2.4 Hz, 1H), 7.94 - 7.82 (m, 2H), 7.35 (d, *J* = 9.1 Hz, 2H), 5.30 (d, *J* = 53.6 Hz, 1H), 3.51 (ddd, *J* = 39.8, 13.6, 3.4 Hz, 1H), 3.35 (s, 2H), 3.21 (t, *J* = 9.6 Hz, 1H), 2.18 - 1.91 (m, 2H).

### DSC and TGA thermal analysis

The collected crystal form II of compound A was subjected to DSC analysis by General method 4. DSC analysis showed a melting endothermic peak at 115.17 °C with an onset temperature of 109.08 °C.

The collected crystal form II of compound A was subjected to TGA analysis by General method 5. TGA analysis showed a weight loss of about 1.9% prior to 200°C.

¹H NMR, DSC and TGA analysis indicated that the crystal form II of compound A was a solvate of acetonitrile.

### Example 4.3 Characterization of the crystal form III of compound A

The crystal form III of compound A was obtained by suspension crystal transformation in a single water solvent according to the method in Example 3.2, and characterized by XRPD, PLM, ¹H NMR, DSC and TGA. The specific results are as follows:

### XRPD analysis

FIG. 5 shows the XRPD data of the crystal form III of compound A acquired according to General method 1. A list of XRPD peaks at diffraction angles 2θ° (°2θ)±0.2 °2θ and relative intensities thereof are provided in Table 4.3.

**Table 4.3: XRPD peak list (2θ°) of the crystal form III of compound A**

| Peak | Angle °2θ±0.2 °2θ | Relative intensity % |
|---|---|---|
| 1 | 7.0 | 27.5 |
| 2 | 9.3 | 39.6 |
| 3 | 12.4 | 94.4 |
| 4 | 13.9 | 27.3 |
| 5 | 14.2 | 91.4 |
| 6 | 14.9 | 2.5 |
| 7 | 15.3 | 14.7 |
| 8 | 15.9 | 6.2 |
| 9 | 17.1 | 3.2 |
| 10 | 18.0 | 3.4 |
| 11 | 19.4 | 9.2 |
| 12 | 20.0 | 100 |
| 13 | 20.6 | 10.8 |
| 14 | 21.0 | 6.7 |
| 15 | 21.9 | 65.9 |
| 16 | 23.4 | 18.2 |
| 17 | 24.5 | 41.9 |
| 18 | 25.0 | 6.8 |
| 19 | 25.3 | 9.3 |
| 20 | 27.5 | 13.1 |
| 21 | 28.3 | 15 |
| 22 | 28.8 | 14.1 |
| 23 | 29.6 | 3.6 |
| 24 | 30.4 | 2.4 |
| 25 | 30.8 | 3.2 |
| 26 | 31.3 | 1.8 |
| 27 | 32.9 | 4.3 |
| 28 | 34.0 | 3.6 |
| 29 | 34.9 | 1.6 |
| 30 | 35.4 | 1.6 |
| 31 | 36.0 | 3.3 |
| 32 | 37.3 | 2.3 |
| 33 | 38.1 | 2.3 |
| 34 | 38.6 | 2.2 |

### PLM analysis

The collected crystal form III of compound A was subjected to PLM analysis by General method 2. It was observed that the sample was irregular lumps.

### ¹H NMR analysis

The ¹H NMR data of the crystal form III of compound A are as follows: ¹H NMR (400 MHz, DMSO-d₆) δ 10.27 (s, 1H), 8.90 (d, *J=* 1.5 Hz, 1H), 8.84 (d, *J=* 2.3 Hz, 1H), 8.75 (dd, *J* = 2.5, 1.5 Hz, 1H), 8.65 (d, *J* = 2.6 Hz, 1H), 8.24 (d, *J* = 2.4 Hz, 1H), 7.90 - 7.84 (m, 2H), 7.35 (d, *J*= 9.1 Hz, 2H), 5.30 (d, *J* = 53.8 Hz, 1H), 3.56 (dd, *J* = 13.4, 3.3 Hz, 1H), 3.46 (dd, *J* = 13.2, 3.2 Hz, 2H), 3.21 (t, *J=* 9.5 Hz, 1H), 2.13 - 1.98 (m, 2H).

### DSC and TGA thermal analysis

The collected crystal form III of compound A was subjected to DSC analysis by General method 4. DSC analysis showed a melting endothermic peak at 172.83 °C with an onset temperature of 171.83 °C.

The collected crystal form III of compound A was subjected to TGA analysis by General method 5. TGA analysis showed a weight loss of about 3.5% prior to 200°C.

¹H NMR, DSC and TGA analysis indicated that the crystal form III of compound A was a hydrate.

### Example 4.4 Characterization of the crystal form IV of compound A

The crystal form IV of compound A was obtained by cooling crystallization in methanol solution according to the method in Example 3.5, and characterized by XRPD, PLM, ¹H NMR, DSC and TGA. The specific results are as follows:

### XRPD analysis

FIG. 6 shows the XRPD data of the crystal form IV of compound A acquired according to General method 1. A list of XRPD peaks at diffraction angles 2θ° (°2θ)±0.2 °2θ and relative intensities thereof are provided in Table 4.4.

**Table 4.4: XRPD peak list (2θ°) of the crystal form IV of compound A**

| Peak | Angle °2θ±0.2 °2θ | Relative intensity % |
|---|---|---|
| 1 | 4.4 | 4.8 |
| 2 | 6.2 | 43.4 |
| 3 | 6.4 | 15 |
| 4 | 9.7 | 59.9 |
| 5 | 10.5 | 8.4 |
| 6 | 10.8 | 21.6 |
| 7 | 12.1 | 9.4 |
| 8 | 12.3 | 17.3 |
| 9 | 12.5 | 4.4 |
| 10 | 12.9 | 40.3 |
| 11 | 14.4 | 2.8 |
| 12 | 16.0 | 11.9 |
| 13 | 16.8 | 4.7 |
| 14 | 17.0 | 5.1 |
| 15 | 17.2 | 9.8 |
| 16 | 18.4 | 38.3 |
| 17 | 18.6 | 7.5 |
| 18 | 18.9 | 8.9 |
| 19 | 19.2 | 21.9 |
| 20 | 19.6 | 6.1 |
| 21 | 21.1 | 11.1 |
| 22 | 21.8 | 48.2 |
| 23 | 22.5 | 100 |
| 24 | 23.2 | 38.8 |
| 25 | 23.6 | 9.5 |
| 26 | 24.4 | 82.9 |
| 27 | 24.8 | 29.7 |
| 28 | 27.2 | 2.5 |
| 29 | 27.9 | 15.1 |
| 30 | 28.3 | 5.1 |
| 31 | 29.7 | 7.7 |
| 32 | 32.1 | 2.6 |
| 33 | 32.7 | 3 |
| 34 | 33.1 | 4.5 |
| 35 | 34.7 | 2.5 |
| 36 | 34.9 | 3.3 |

### PLM analysis

The collected crystal form IV of compound A was subjected to PLM analysis by General method 2. It was observed that the sample was microcrystalline.

### ¹H NMR analysis

The ¹H NMR data of the crystal form IV of compound A are as follows: ¹H NMR (400 MHz, DMSO-d₆) δ 10.27 (s, 1H), 8.90 (d, *J=* 1.5 Hz, 1H), 8.84 (d, *J=* 2.4 Hz, 1H), 8.75 (dd, *J* = 2.5, 1.5 Hz, 1H), 8.65 (d, *J* = 2.6 Hz, 1H), 8.24 (d, *J* = 2.4 Hz, 1H), 7.92 - 7.82 (m, 2H), 7.35 (d, *J* = 9.1 Hz, 2H), 5.30 (d, *J* = 53.1 Hz, 1H), 3.59 - 3.44 (m, 1H), 3.36 (d, *J* = 6.5 Hz, 2H), 3.21 (t, *J=* 9.4 Hz, 1H), 2.15 - 1.95 (m, 2H).

### DSC and TGA thermal analysis

The collected the crystal form IV of compound A was subjected to DSC analysis by General method 4. DSC analysis showed a melting endothermic peak at 175.84 °C with an onset temperature of 174.03 °C. In addition, there was also an endothermic peak at a higher temperature with a peak temperature of 250.71 °C and an onset temperature of 250.64 °C.

The collected crystal form IV of compound A was subjected to TGA analysis by General method 5. TGA analysis showed a weight loss of about 3.7% prior to 200°C.

¹H NMR, DSC and TGA analysis indicated that the crystal form IV of compound A was a solvate of methanol.

### Example 4.5 Characterization of the crystal form V of compound A

The crystal form V of compound A was obtained by suspension crystal transformation in a single isobutanol solution according to the method in Example 3.2, and characterized by XRPD, PLM, ¹H NMR, DSC and TGA. The specific results are as follows:

### XRPD analysis

FIG. 7 shows the XRPD data of the crystal form V of compound A acquired according to General method 1. A list of XRPD peaks at diffraction angles 2θ° (°2θ)±0.2 °2θ and relative intensities thereof are provided in Table 4.5.

**Table 4.5: XRPD peak list (2θ°) of the crystal form V of compound A**

| Peak | Angle °2θ±0.2 °2θ | Relative intensity % |
|---|---|---|
| 1 | 8.0 | 8.2 |
| 2 | 9.4 | 81.6 |
| 3 | 9.9 | 10.1 |
| 4 | 10.4 | 8 |
| 5 | 11.8 | 24.3 |
| 6 | 15.6 | 11.5 |
| 7 | 15.9 | 24 |
| 8 | 16.3 | 7.3 |
| 9 | 16.7 | 9 |
| 10 | 17.1 | 6.4 |
| 11 | 17.6 | 20.2 |
| 12 | 18.1 | 90.8 |
| 13 | 18.4 | 15 |
| 14 | 18.9 | 51.7 |
| 15 | 19.1 | 15.1 |
| 16 | 19.8 | 11.1 |
| 17 | 20.5 | 7.1 |
| 18 | 20.9 | 6.8 |
| 19 | 21.3 | 61.6 |
| 20 | 21.8 | 21.7 |
| 21 | 23.0 | 7.3 |
| 22 | 23.2 | 15.7 |
| 23 | 23.6 | 100 |
| 24 | 24.3 | 45.7 |
| 25 | 24.6 | 8.9 |
| 26 | 25.6 | 9.2 |
| 27 | 26.8 | 34.3 |
| 28 | 27.4 | 8.3 |
| 29 | 30.0 | 7.5 |
| 30 | 31.7 | 5.4 |
| 31 | 33.7 | 7 |

### PLM analysis

The collected crystal form V of compound A was subjected to PLM analysis by General method 2. It was observed that the sample was microcrystalline.

### ¹H NMR analysis

The ¹H NMR data of the crystal form V of compound A are as follows: ¹H NMR (400 MHz, DMSO-d₆) δ 10.27 (s, 1H), 8.90 (d, *J=* 1.5 Hz, 1H), 8.84 (d, *J=* 2.3 Hz, 1H), 8.75 (dd, *J* = 2.5, 1.5 Hz, 1H), 8.65 (d, *J* = 2.6 Hz, 1H), 8.24 (d, *J* = 2.4 Hz, 1H), 7.91 - 7.83 (m, 2H), 7.35 (d, *J* = 9.1 Hz, 2H), 5.36 (d, *J* = 53.6 Hz, 1H), 3.59 - 3.54 (m, 1H), 3.50 - 3.42 (m, 2H), 3.27 - 3.16 (m, 1H), 2.14 - 1.96 (m, 2H).

### DSC and TGA thermal analysis

The collected crystal form V of compound A was subjected to DSC analysis by General method 4. DSC analysis showed a melting endothermic peak at 173.42 °C with an onset temperature of 171.38 °C.

The collected crystal form V of compound A was subjected to TGA analysis by General method 5. TGA analysis showed a weight loss of about 6.5% prior to 200°C.

¹H NMR, DSC and TGA analysis indicated that the crystal form V of compound A was a solvate of isobutanol.

### Example 4.6 Characterization of the crystal form VI of compound A

### The crystal form VI of compound A was obtained according to the industrial method in Example 2, and characterized by XRPD, PLM, NMR, MS, UV, DSC, TGA, DVS and IR. The specific results are as follows:

### XRPD analysis

FIG. 8 shows the XRPD data of the crystal form VI of compound A acquired according to General method 1. A list of XRPD peaks at diffraction angles 2θ° (°2θ)±0.2 °2θ and relative intensities thereof are provided in Table 4.6-1.

**Table 4.6-1: XRPD peak list (2θ°) of the crystal form VI of compound A**

| Peak | Angle °2θ±0.2 °2θ | Relative intensity % |
|---|---|---|
| 1 | 5.9 | 6.9 |
| 2 | 9.7 | 12.3 |
| 3 | 11.0 | 8.8 |
| 4 | 11.9 | 48.7 |
| 5 | 12.6 | 2.6 |
| 6 | 12.8 | 5.8 |
| 7 | 14.7 | 6.5 |
| 8 | 15.3 | 2.4 |
| 9 | 16.1 | 14 |
| 10 | 16.6 | 5.1 |
| 11 | 17.6 | 4.5 |
| 12 | 17.8 | 8.6 |
| 13 | 18.2 | 5.4 |
| 14 | 18.6 | 5.1 |
| 15 | 19.3 | 20 |
| 16 | 20.1 | 8.7 |
| 17 | 20.5 | 100 |
| 18 | 21.2 | 20.3 |
| 19 | 22.0 | 8 |
| 20 | 22.6 | 6.7 |
| 21 | 23.1 | 29.3 |
| 22 | 23.9 | 26.3 |
| 23 | 24.8 | 36.8 |
| 24 | 26.2 | 6.2 |
| 25 | 26.7 | 4.5 |
| 26 | 29.0 | 5.1 |
| 27 | 29.2 | 2.9 |
| 28 | 29.7 | 3.5 |
| 29 | 30.9 | 3.2 |
| 30 | 32.1 | 4.6 |
| 31 | 32.5 | 3.3 |
| 32 | 33.5 | 3.1 |
| 33 | 34.7 | 2.6 |
| 34 | 35.1 | 3 |
| 35 | 35.9 | 2.1 |
| 36 | 36.2 | 3.8 |
| 37 | 36.7 | 2.5 |
| 38 | 38.6 | 2.3 |
| 39 | 39.0 | 2 |

### PLM analysis

The collected crystal form VI of compound A was subjected to PLM analysis by General method 2. It was observed that the sample was microcrystalline.

### NMR analysis

Instrument and equipment: Bruker AVANCE III 400 MHz NMR instrument, solvent: DMSO-d6.

Sample analysis: 52.15 mg of the crystal form VI of compound A was weighed accurately, and 0.6 mL of DMSO-d6 was added. After dissolved completely, the solution was transferred into a NMR tube, and tested for ¹H-NMR, ¹³C-NMR, DEPT, ¹⁹F-NMR, HSQC, HMBC, COSY, NOESY and H-F NOESY.

Structural fragment of compound A:

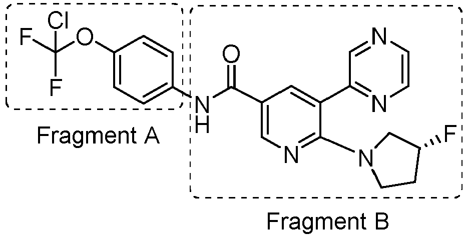

Critical HMBC and COSY related structure of compound A:

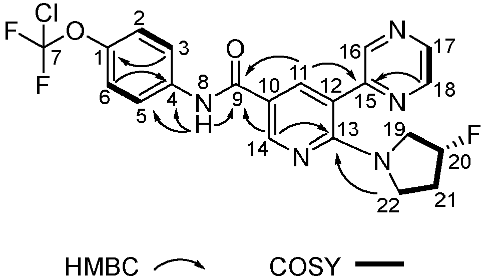

### ¹H-NMR

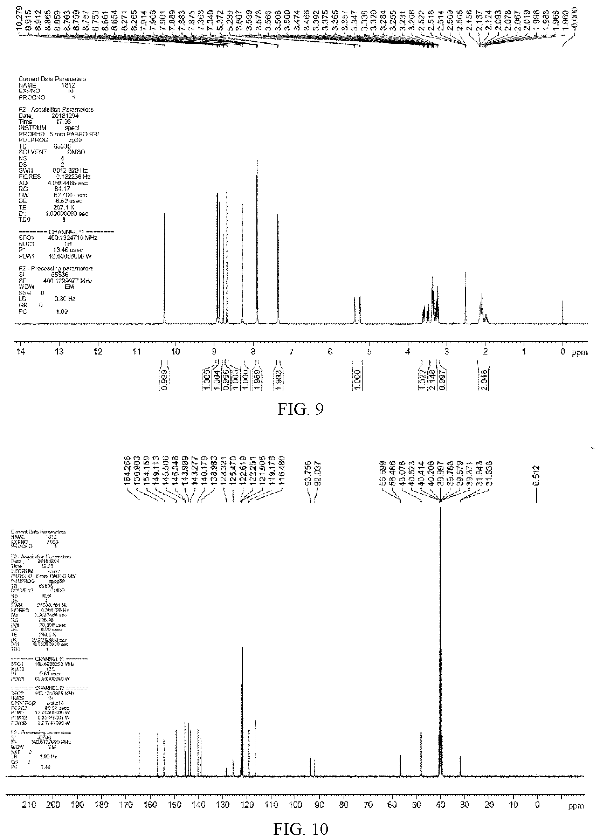

FIG. 9 shows the nuclear magnetic resonance hydrogen spectrum of the crystal form VI of compound A. Table 4.6-2 shows the test results of the nuclear magnetic resonance hydrogen spectrum of the crystal form VI of compound A. The results show that there are 17 hydrogen signals in the hydrogen spectrum, including 7 methylene hydrogens, 9 methenyl hydrogens, and 1 active hydrogen. The hydrogen signal with chemical shift at δ_{H} 10.28 (s, 1H) has no HSQC correlation and is assigned to NH-8 according to chemical shift. The hydrogen signals with chemical shifts at δ_{H} 7.89 (m, 2H) and δ_{H} 7.35 (d, J = 9.2 Hz, 2H) are typical characteristic signals of 1,4-disubstituted benzene ring, and are assigned to H-3, 5, H-2, 6 according to HMBC (Table 3.6-5). The hydrogen signals with chemical shifts at δ_{H} 5.37, 5.24 ( d, J=52 Hz, 1H), δH 3.53 (ddd, J=40, 13.6, 3.2 Hz, 1H), δ_{H} 3.35 (overlaping, 1H), δ_{H} 3.39-3.19 (overlaping, 2H), and δ_{H} 2.18-1.91 (m, 2H) are assigned to H-20, H-19a, H-19b, H-22, H-21 respectively according to chemical shifts, coupling constants and COSY (Table 3.6-6). The rest of the hydrogen signals are assigned according to COSY and HMBC. The hydrogen spectrum data are consistent with the structure of compound A.

**Table 4.6-2: The test results of the nuclear magnetic resonance hydrogen spectrum of the crystal form VI of compound A.**

| Chemical shift (ppm) | Peak shape | Number of protons | Assignment |
|---|---|---|---|
| 10.28 | s | 1H | NH-8 |
| 8.91 | d (J = 1.2 Hz) | 1H | H-16 |
| 8.86 | d (J = 2.4 Hz) | 1H | H-14 |
| 8.76 | m | 1H | H-17 |
| 8.66 | d (J = 2.8 Hz) | 1H | H-18 |
| 8.27 | d (J = 2.4 Hz) | 1H | H-11 |
| 7.89 | m | 2H | H-3, 5 |
| 7.35 | d (J = 9.2 Hz) | 2H | H-2, 6 |
| 5.37, 5.24 | d (J = 52 Hz) | 1H | H-20 |
| 3.53 | ddd (J = 40, 13.6, 3.2 Hz) | 1H | H-19a |
| 3.35 | Overlaping | 1H | H-19b |
| 3.39-3.19 | Overlaping | 2H | H-22 |
| 2.18-1.91 | m | 2H | H-21 |

### ¹³C-NMR

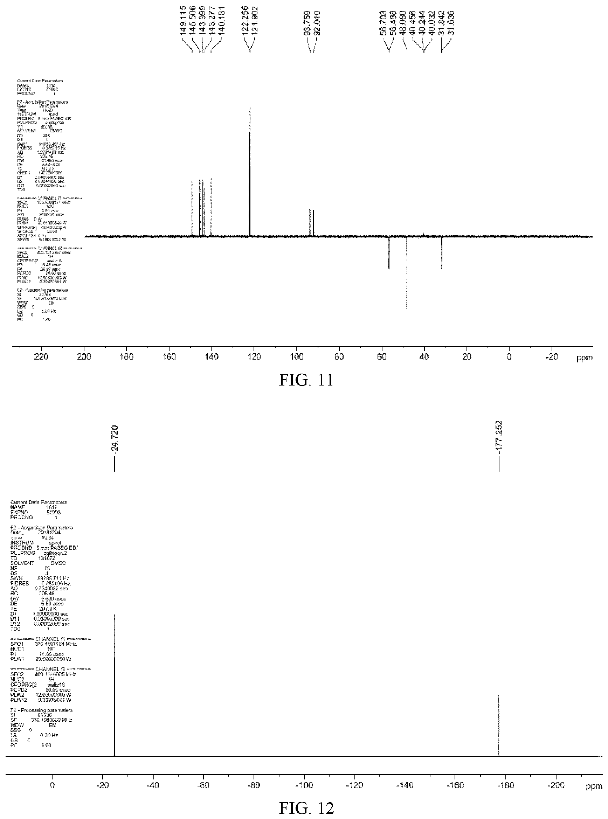

FIG. 10 shows the nuclear magnetic resonance carbon spectrum of the crystal form VI of compound A. FIG. 11 shows the DEPT NMR spectrum of the crystal form VI of compound A, and Table 4.6-3 shows the test results of the nuclear magnetic resonance carbon spectrum of the crystal form VI of compound A. The results show that there are a total of 21 carbon signals in the ¹³C-NMR spectrum. Combined with DEPT, it is showed that there are 4 methylene carbons, 9 methenyl carbons and 8 carbons which are not connected to hydrogen. The carbon signals of all the above carbons which are connected to hydrogen are assigned through the HSQC data (Table 3.6-5), and the remaining carbons which are not connected to hydrogen are assigned by chemical shift and HMBC: In the HMBC spectrum, δC 164.27 is associated with NH-8, H-11, and H-14, and is assigned to C-9 combined with its chemical shift; δC 156.90 is associated with H-11 and H-14, and is assigned to C-13; δC 154.16 is associated with H-11 and H-16, and is assigned to C-15; δC 149.11 is associated with H-11 and is assigned to C-14; δC 145.35 and δC 138.98 are associated with H-2, 6, H-3, 5, and are assigned to C-1 and C-4 respetively combined with their chemical shifts; δC 128.32, 125.47, 122.62 are assigned to C-7 according to peak shape and coupling constant (t, J = 285 Hz); δC 119.18 is associated with H-14 and is assigned to C-10; δC 116.48 is associated with H-14 and H-16, and is assigned to C-12. The carbon spectrum data are consistent with the structure of compound A.

**Table 4.6-3: The test results of the nuclear magnetic resonance carbon spectrum of the crystal form VI of compound A**

| Chemical shift (ppm) | Type of carbon | Assignment |
|---|---|---|
| 164.27 | C=O | C-9 |
| 156.90 | C | C-13 |
| 154.16 | C | C-15 |
| 149.11 | CH | C-14 |
| 145.51 | CH | C-16 |
| 145.35 | C | C-1 |
| 144.00 | CH | C-17 |
| 143.28 | CH | C-18 |
| 140.18 | CH | C-11 |
| 138.98 | C | C-4 |
| 128.32, 125.47, 122.62 (t, J = 285 Hz) | C | C-7 |
| 122.25 | CH | C-2, 6 |
| 121.91 | CH | C-3, 5 |
| 119.18 | C | C-10 |
| 116.48 | C | C-12 |
| 93.76, 92.04 (d, J = 172 Hz) | CH | C-20 |
| 56.70, 56.49 (d, J = 21 Hz) | CH₂ | C-19 |
| 48.08 | CH₂ | C-22 |
| 31.84, 31.64 (d, J = 20 Hz) | CH₂ | C-21 |

### ¹⁹F-NMR and H-F NOESY spectra

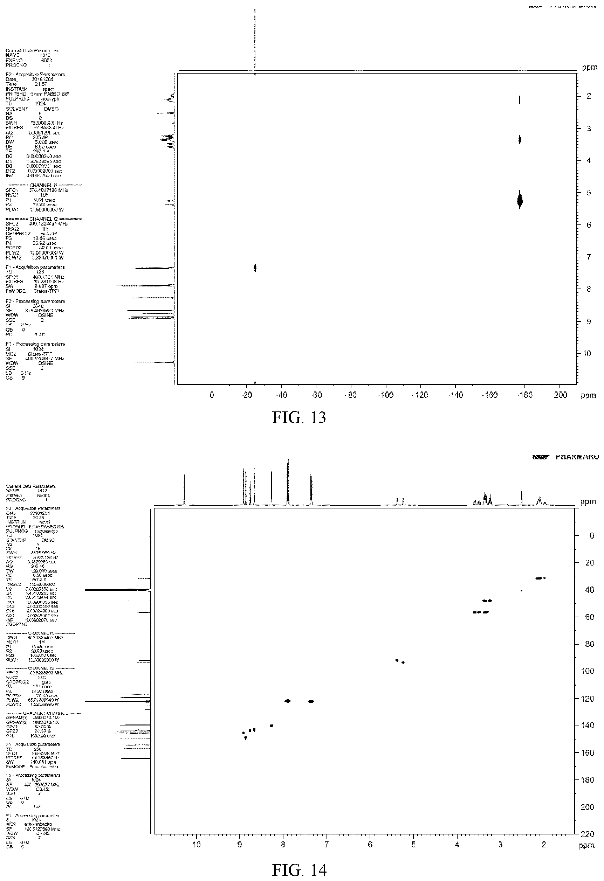

FIG. 12 shows the ¹⁹F-NMR spectrum of the crystal form VI of compound A, and FIG. 13 shows the NMR H-F NOESY spectrum of the crystal form VI of compound A. Table 4.6-4 shows the test results of the ¹⁹F-NMR and the H-F NOESY spectra of the crystal form VI of compound A. The results show that in the H-F NOESY spectrum, the chemical shift at δ -24.72 is associated with H-2, 6 and is assigened to F₂-7, and δ -177.25 is associated with H-19b, H-20, and H-21 and is assigened to F-20. The F and H-F NOESY spectra are consistent with the structure of compound A.

**Table 4.6-4: The test results of ¹⁹F-NMR and H-F NOESY spectra of the crystal form VI of compound A**

| Chemical shift (ppm) | Peak shape | Assignment | H-F NOESY |
|---|---|---|---|
| -24.72 | s | F₂-7 | H-2, 6 |
| -177.25 | s | F-20 | H-19b, H-20, H-21 |

### HSQC and HMBC spectra

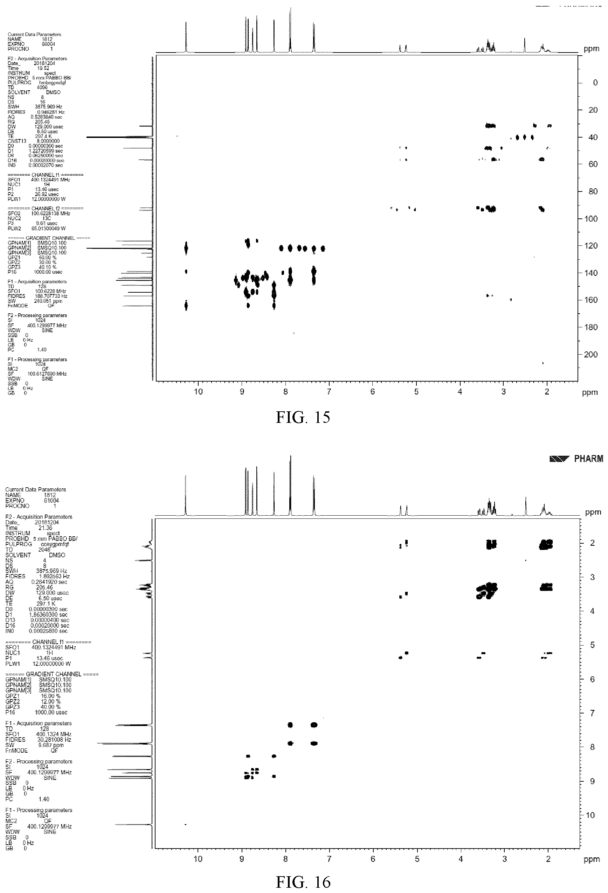

FIG. 14 shows the NMR HSQC spectrum of the crystal form VI of compound A, FIG. 15 shows the NMR HMBC spectrum of the crystal form VI of compound A, and Table 4.6-5 shows the test results of the HSQC and the HMBC spectra of the crystal form VI of compound A. The results show that: In the HMBC spectrum, H-11 is associated with C-9, C-13, C-14, and C-15, H-14 is associated with C-9, C-10, and C-13, H-16 is associated with C-12 and C-15, H-17 is associated with C-15 and C-18, H-18 is associated with C-12 and C-15, H-19a is associated with C-20, H-19b is associated with C-20 and C-22, H-20 is associated with C-19 and C-22, H-21 is associated with C-19 and C-20, and H-22 is associated with C-13, C-19, C-20, and C-21, which are consistent with the existence of Fragment B in the structure; H-2, 6, H-3, 5 are associated with C-1, C-4, which are consistent with the existence of Fragment A in the structure combined with chemical shift and molecular formula (C₂₁H₁₇ClF₃N₅O₂). The HSQC and HMBC data are consistent with the structure of compound A.

**Table 4.6-5: The test results of HSQC and HMBC spectra of the crystal form VI of compound A**

| Chemical shift (ppm) | Assignment | HSQC | HMBC |
|---|---|---|---|
| 10.28 | NH-8 | - | C-3, 5, C-4, C-9 |
| 8.91 | H-16 | 145.51 | C-12, C-15 |
| 8.86 | H-14 | 149.11 | C-9, C-10, C-13 |
| 8.76 | H-17 | 144.00 | C-15, C-18 |
| 8.66 | H-18 | 143.28 | C-12, C-15 |
| 8.27 | H-11 | 140.18 | C-9, C-13, C-14, C-15 |
| 7.89 | H-3, 5 | 121.91 | C-1, C-4 |
| 7.35 | H-2, 6 | 122.25 | C-1, C-4 |
| 5.37, 5.24 | H-20 | 93.76, 92.04 | C-19, C-22 |
| 3.53 | H-19a | 56.70, 56.49 | C-20 |
| 3.35 | H-19b | 56.70, 56.49 | C-20, C-22 |
| 3.39-3.19 | H-22 | 48.08 | C-13, C-19, C-20, C-21 |
| 2.18-1.91 | H-21 | 31.84, 31.64 | C-19, C-20 |

### COSY and NOESY spectra

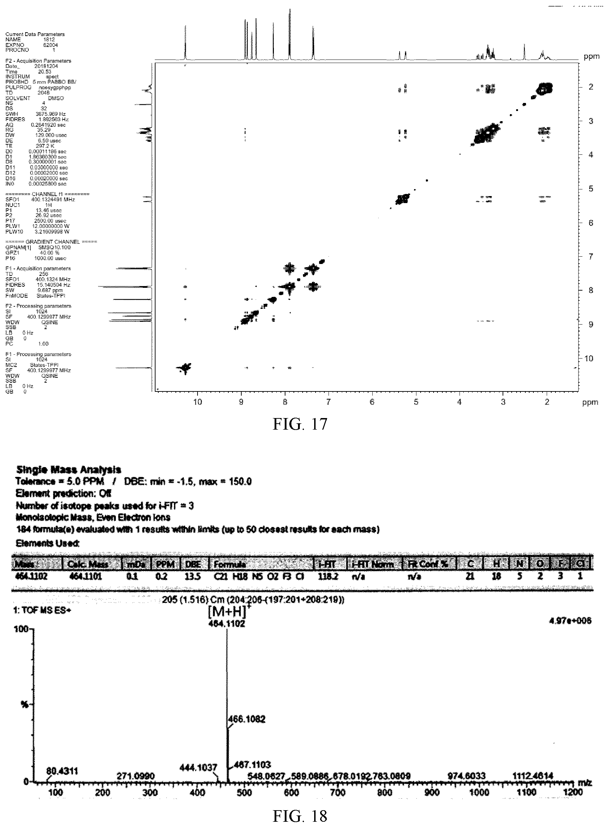

FIG. 16 shows the NMR COSY spectrum of the crystal form VI of compound A, FIG. 17 shows the NMR NOESY spectrum of the crystal form VI of compound A, and Table 4.6-6 shows the test results of COSY and NOESY spectra of the crystal form VI of compound A. The results show that in the COSY spectrum, H-17 is associated with H-18, H-20 is associated with H-19a and H-21, and H-22 is associated with H-21, which further proves the existence of Fragment B in the structure; H-2, 6 is associated with H-3, 5, which further proves the existence of Fragment A in the structure. In the NOESY spectrum, NH-8 is associated with H-3, 5, suggesting that C-4 of Fragment A is connected to N-8 of Fragment B. The COSY, NOESY spectral data are consistent with the structure of compound A.

**Table 4.6-6: The test results of COSY and NOESY spectra of the crystal form VI of compound A**

| ¹H-NMR (ppm) | Assignment | COSY | NOESY |
|---|---|---|---|
| 10.28 | NH-8 | - | H-3, 5, H-11, H-14 |
| 8.91 | H-16 | - | H-11 |
| 8.76 | H-17 | H-18 | - |
| 7.35 | H-2, 6 | H-3, 5 | - |
| 5.37, 5.24 | H-20 | H-19a, H-21 | - |
| 3.35 | H-19b | H-19a | H-16 |
| 3.39-3.19 | H-22 | H-21 | H-16 |

### MS analysis

Instrument and equipment: Waters Acquity Xevo G2-XS QTof UPLC/MS ultra-highperformance liquid chromatography combined with high-resolution mass spectrometry system

FIG. 18 shows the mass spectrum of the crystal form VI of compound A. The results show that the mass-to-charge ratio of the ion peak displayed in the high-resolution mass spectrum is 464.1102 [M+H] ⁺, and the deviation from the theoretical value is less than 5 ppm, (theoretical value 464.1101, C₂₁H₁₈ClF₃N₅O₂), suggesting that the molecular formula of the sample is C₂₁H₁₇ClF₃N₅O₂, which is consistent with the structure of compound A.

### UV analysis

Instrument and equipment: UV-2600 UV-Vis Spectrophotometer (Shimadzu Corporation, Japan)

Sample analysis: 24.59 mg of sample of the crystal form VI of compound A was weighed accurately, and dissolved in methanol. The solution was made up to 100 mL, and mixed well. 1.0 mL of the resulting solution was added into a 25 mL volumetric flask, and the solution was diluted, made up to a constant volume, and mixed well.

FIG. 19 shows the UV spectrum of the crystal form VI of compound A. Table 4.6-7 shows the test results of UV of the crystal form VI of compound A. The absorption peaks at λmax 306, 263, and 201 nm observed in methanol solution are π-π^{∗} transition absorption peaks of the long chain conjugated system and substituted benzene ring of compound A, which are consistent with the structure of compound A.

**Table 4.6-7: The test results of UV of the crystal form VI of compound A**

| Entry | Peak/Valley | Wavelength (nm) | ABS | ε (^{∗}10⁴) |
|---|---|---|---|---|
| 1 | Peak | 306 | 0.6223 | 2.93 |
| 2 | Peak | 263 | 0.3927 | 1.85 |
| 3 | Peak | 201 | 0.5900 | 2.78 |
| 4 | Valley | 277 | 0.3308 | - |
| 5 | Valley | 237 | 0.1963 | - |

### DSC and TGA thermal analysis

FIG. 20 shows the DSC curve of the crystal form VI of compound A acquired by General method 3. In the DSC, it is observed that the sample shows a melting endothermic peak at 174.95 °C with an onset temperature of 174.20 °C, indicating that the melting point of the crystal form VI of compound A is 175 °C. The crystal form of the crystal form VI of compound A is not changed after the DVS test, as shown in FIG. 3.

FIG. 21 shows the TGA curve of the crystal form VI of compound A acquired by general method 4. In the TGA, a weight loss of 0.003174 % prior to 200 °C is observed, indicating that the crystal form VI does not contain crystal water.

### DVS analysis

FIG. 22 shows the DVS curve of the crystal form VI of compound A acquired by General method 6. DVS analysis shows that the water absorption is 0.1389% under the condition of 10%RH, the water absorption is 1.1852% under the condition of 0-80%RH, and the water absorption is 1.7452% under the condition of 0-90%RH.

### IR analysis

Instrument and equipment: Nicolet iS10 Fourier Transform Infrared Spectrometer (Thermo)

Sample analysis: 303.12 mg of potassium bromide and 3.61 mg of sample of the crystal form VI of compound A were weighed and mixed well. The mixture was ground into powder. The KBr tabletting method was used, and the polystyrene film was used for correction. The infrared spectrum of this product was acquired within the range of infrared light of 4000-400 cm⁻¹.

FIG. 23 shows the IR spectrum of the crystal form VI of compound A. Table 4.6-8 shows the test results of IR of the crystal form VI of compound A. In IR, it is observed that: 3293 cm-¹ represents the stretching vibration absorption peak of N-H, indicating that the structure contains NH moiety; 1661 cm⁻¹ represents the stretching vibration absorption peak of C=O, indicating that the structure contains C=O moiety; 1599, 1491 cm⁻¹ represent the stretching vibration absorption peaks of the C=C double bond, 1062, 1020 cm⁻¹ represent the in-plane bending vibration absorption peaks of =C-H, 853 cm⁻¹ represents the out-of-plane bending vibration absorption peak of =C-H, indicating that the structure contains C=C moiety; 1466, 1408 cm⁻¹ represent the bending vibration absorption peak of C-H bond, indicating that the structure contains CH₂, CH moiety; 1210 cm⁻¹ represent the stretching vibration absorption peak of C-O-C bond, indicating that the structure contains the C-O-C moiety. The infrared spectral characteristics are consistent with the structure of compound A.

**Table 4.6-8: Test results of IR of the crystal form VI of the free base of compound A**

| Absorption wave number (cm⁻¹) | Type of vibration | Assignment |
|---|---|---|
| 3293 | ν_{N-H} | NH |
| 1661 | ν_{C=O} | C=O |
| 1599, 1491 | v_{C=C} | C=C |
| 1466, 1408 | δ_{C-H} | CH₂, CH |
| 1210 | ν_{C-O-C} | C-O-C |
| 1062, 1020 | δ_{=C-H} | C=C |
| 853 | γ_{=C-H} | C=C |

### Example 4.7 Characterization of the crystal form VII of compound A

The crystal form VII of compound A was obtained by suspension crystal transformation in a single toluene solvent according to the method in Example 3.2, and characterized by XRPD, PLM, ¹H NMR, DSC and TGA. The specific results are as follows:

### XRPD analysis

FIG. 24 shows the XRPD data of the crystal form VII of compound A acquired according to General method 1. A list of XRPD peaks at diffraction angles 2θ° (°2θ)±0.2 °2θ and relative intensities thereof are provided in Table 4.7.

**Table 4.7: XRPD peak list (2θ°) of the crystal form VII of compound A**

| Peak | Angle °2θ±0.2 °2θ | Relative intensity % |
|---|---|---|
| 1 | 5.8 | 8.6 |
| 2 | 10.2 | 5.1 |
| 3 | 11.7 | 92.9 |
| 4 | 12.0 | 6.2 |
| 5 | 12.9 | 9.8 |
| 6 | 13.2 | 24.9 |
| 7 | 13.4 | 25.2 |
| 8 | 15.1 | 7.7 |
| 9 | 16.0 | 39.8 |
| 10 | 16.9 | 97.3 |
| 11 | 18.0 | 3.6 |
| 12 | 18.3 | 58 |
| 13 | 19.6 | 12.4 |
| 14 | 20.7 | 29.4 |
| 15 | 20.9 | 99.3 |
| 16 | 21.3 | 13.2 |
| 17 | 21.7 | 76.5 |
| 18 | 22.2 | 35.4 |
| 19 | 23.2 | 68.5 |
| 20 | 23.8 | 100 |
| 21 | 25.7 | 14.5 |
| 22 | 25.9 | 8.7 |
| 23 | 26.5 | 4.4 |
| 24 | 27.1 | 56.3 |
| 25 | 28.4 | 3.4 |
| 26 | 29.6 | 3.6 |
| 27 | 29.9 | 8.7 |
| 28 | 30.4 | 4.7 |
| 29 | 30.9 | 12.1 |
| 30 | 31.4 | 3.4 |
| 31 | 32.1 | 4.6 |
| 32 | 32.3 | 3.4 |
| 33 | 34.0 | 4.8 |
| 34 | 34.7 | 3.4 |
| 35 | 35.7 | 3 |
| 36 | 36.0 | 6.2 |
| 37 | 36.5 | 3.4 |
| 38 | 38.0 | 3.1 |
| 39 | 38.6 | 3.3 |
| 40 | 39.2 | 2.8 |

### PLM analysis

The collected crystal form VII of compound A was subjected to PLM analysis by General method 2. It was observed that the sample was microcrystalline.

### ¹H NMR analysis

The ¹H NMR data of the crystal form VII of compound A are as follows: ¹H NMR (400 MHz, DMSO-d₆) δ 10.27 (s, 1H), 8.90 (d, *J=* 1.5 Hz, 1H), 8.84 (d, *J=* 2.4 Hz, 1H), 8.75 (dd, *J* = 2.6, 1.5 Hz, 1H), 8.65 (d, *J* = 2.6 Hz, 1H), 8.24 (d, *J* = 2.4 Hz, 1H), 7.90 - 7.84 (m, 2H), 7.35 (d, *J=* 9.1 Hz, 2H), 5.30 (d, *J=* 53.6 Hz, 1H), 3.58 - 3.46 (m, 1H), 3.37 (s, 2H), 3.21 (t, *J=* 9.5 Hz, 1H), 2.15 - 1.97 (m, 2H).

### DSC and TGA thermal analysis

The collected crystal form VII of compound A was subjected to DSC analysis by General method 4. DSC analysis showed a melting endothermic peak at 173.61 °C with an onset temperature of 172.39 °C.

The collected crystal form VII of compound A was subjected to TGA analysis by General method 5. TGA analysis showed a weight loss of about 3.4% prior to 200°C.

¹H NMR, DSC and TGA analysis indicated that the crystal form VII of compound A was a hydrate.

### Example 4.8 Characterization of the crystal form VIII of compound A

The crystal form VIII of compound A was obtained by evaporation in a single ethanol solvent according to the method in Example 3.3, and characterized by XRPD, PLM and ¹H NMR. The specific results are as follows:

### XRPD analysis

FIG. 25 shows the XRPD data of the crystal form VIII of compound A acquired according to General method 1. A list of XRPD peaks at diffraction angles 2θ° (°2θ)±0.2 °2θ and relative intensities thereof are provided in Table 4.8.

**Table 4.8: XRPD peak list (2θ°) of the crystal form VIII of compound A**

| Peak | Angle °2θ±0.2 °2θ | Relative intensity % |
|---|---|---|
| 1 | 9.6 | 100 |
| 2 | 10.7 | 6.2 |
| 3 | 12.1 | 5.1 |
| 4 | 12.7 | 19.2 |
| 5 | 16.1 | 3.7 |
| 6 | 16.6 | 2.6 |
| 7 | 17.1 | 3.7 |
| 8 | 18.3 | 82.8 |
| 9 | 19.1 | 12.6 |
| 10 | 19.4 | 9.7 |
| 11 | 21.3 | 3.6 |
| 12 | 21.8 | 7.4 |
| 13 | 22.5 | 8.2 |
| 14 | 22.8 | 13.6 |
| 15 | 23.3 | 2.2 |
| 16 | 24.3 | 22.7 |
| 17 | 24.7 | 6.8 |
| 18 | 27.6 | 8.1 |
| 19 | 28.0 | 3.4 |
| 20 | 29.2 | 4.1 |
| 21 | 30.5 | 2.7 |
| 22 | 31.8 | 1.8 |
| 23 | 38.8 | 2.4 |

### PLM analysis

The collected crystal form VIII of compound A was subjected to PLM analysis by General method 2. It was observed that the sample was microcrystalline.

### ¹H NMR analysis

The ¹H NMR data of the crystal form VIII of compound A are as follows: ¹H NMR (400 MHz, DMSO-d₆) δ 10.27 (s, 1H), 8.90 (d, *J=* 1.5 Hz, 1H), 8.84 (d, *J=* 2.4 Hz, 1H), 8.75 (dd, *J* = 2.6, 1.5 Hz, 1H), 8.65 (d, *J* = 2.6 Hz, 1H), 8.24 (d, *J* = 2.4 Hz, 1H), 7.91 - 7.84 (m, 2H), 7.35 (d, *J=* 9.1 Hz, 2H), 5.30 (d, *J=* 53.3 Hz, 1H), 3.58 - 3.48 (m, 1H), 3.35 (s, 2H), 3.21 (t, *J=* 9.3 Hz, 1H), 2.13 - 1.98 (m, 2H).

### Example 4.9 Characterization of the crystal form IX of compound A

The crystal form IX of compound A was obtained by dissolution-precipitation crystallization in tetrahydrofuran/isopropanol according to the method in Example 3.4, and characterized by XRPD, PLM, ¹H NMR, DSC and TGA. The specific results are as follows:

### XRPD analysis

FIG. 26 shows the XRPD data of the crystal form IX of compound A acquired according to General method 1. A list of XRPD peaks at diffraction angles 2θ° (°2θ)±0.2 °2θ and relative intensities thereof are provided in Table 4.9.

**Table 4.9: XRPD peak list (2θ°) of the crystal form IX of compound A**

| Peak | Angle °2θ±0.2 °2θ | Relative intensity % |
|---|---|---|
| 1 | 6.1 | 10 |
| 2 | 9.6 | 100 |
| 3 | 10.5 | 9.4 |
| 4 | 12.1 | 20 |
| 5 | 12.4 | 10.3 |
| 6 | 12.7 | 2.7 |
| 7 | 13.0 | 1.7 |
| 8 | 14.5 | 1.6 |
| 9 | 16.1 | 7.6 |
| 10 | 16.3 | 4.6 |
| 11 | 16.6 | 4.9 |
| 12 | 16.9 | 3.5 |
| 13 | 17.3 | 1.8 |
| 14 | 17.7 | 2.8 |
| 15 | 18.2 | 85.5 |
| 16 | 18.6 | 4.6 |
| 17 | 19.0 | 11.1 |
| 18 | 19.3 | 16.3 |
| 19 | 19.9 | 1.6 |
| 20 | 20.6 | 5 |
| 21 | 21.2 | 7.6 |
| 22 | 21.5 | 23.5 |
| 23 | 22.1 | 30 |
| 24 | 23.2 | 1.6 |
| 25 | 23.6 | 6.1 |
| 26 | 24.4 | 21.5 |
| 27 | 24.9 | 13.2 |
| 28 | 25.6 | 3.4 |
| 29 | 26.0 | 2.3 |
| 30 | 26.7 | 2.1 |
| 31 | 26.9 | 5 |
| 32 | 27.7 | 7.3 |
| 33 | 28.0 | 2.7 |
| 34 | 28.4 | 5.5 |
| 35 | 28.9 | 1.5 |
| 36 | 29.8 | 1.4 |
| 37 | 30.7 | 1.9 |
| 38 | 31.1 | 1.8 |
| 39 | 31.3 | 2.9 |
| 40 | 31.8 | 1.8 |
| 41 | 32.2 | 3.7 |
| 42 | 33.6 | 1.3 |
| 43 | 33.9 | 1.3 |
| 44 | 34.5 | 2.5 |
| 45 | 34.7 | 1.6 |
| 46 | 37.6 | 1.4 |
| 47 | 38.1 | 1.8 |
| 48 | 39.4 | 1.5 |

### PLM analysis

The collected crystal form IX of compound A was subjected to PLM analysis by General method 2. It was observed that the sample was microcrystalline.

### ¹H NMR analysis

The ¹H NMR data of the crystal form IX of compound A are as follows: ¹H NMR (400 MHz, DMSO-d₆) δ 10.27 (s, 1H), 8.90 (d, *J=* 1.5 Hz, 1H), 8.84 (d, *J=* 2.4 Hz, 1H), 8.75 (dd, *J* = 2.6, 1.5 Hz, 1H), 8.65 (d, *J* = 2.6 Hz, 1H), 8.24 (d, *J* = 2.4 Hz, 1H), 7.91 - 7.84 (m, 2H), 7.35 (d, *J* = 9.1 Hz, 2H), 5.30 (d, *J* = 53.4 Hz, 1H), 3.56 - 3.44 (m, 1H), 3.36 (d, *J* = 6.7 Hz, 2H), 3.21 (t, *J* = 9.5 Hz, 1H), 2.14 - 1.96 (m, 2H).

### DSC and TGA thermal analysis

The collected crystal form IX of compound A was subjected to DSC analysis by General method 4. DSC analysis showed a melting endothermic peak at 173.92 °C with an onset temperature of 172.14 °C.

The crystal form IX of compound A was subj ected to TGA analysis by General method 5. TGA analysis showed a weight loss of about 5.8% prior to 200°C.

¹H NMR, DSC and TGA analysis indicated that the crystal form IX of compound A was a solvate of tetrahydrofuran, ethanol and isopropanol.

### Example 4.10 Characterization of the crystal form X of compound A

The crystal form X of compound A was obtained by dissolution-precipitation crystallization in tetrahydrofuran/water according to the method in Example 3.4, and characterized by XRPD, PLM, ¹H NMR, DSC and TGA. The specific results are as follows:

### XRPD analysis

FIG. 27 shows the XRPD data of the crystal form X of compound A acquired according to General method 1. A list of XRPD peaks at diffraction angles 2θ° (°2θ)±0.2 °2θ and relative intensities thereof are provided in Table 4.10.

**Table 4.10: XRPD peak list (2θ°) of the crystal form X of compound A**

| Peak | Angle °2θ±0.2 °2θ | Relative intensity % |
|---|---|---|
| 1 | 7.0 | 70.1 |
| 2 | 9.3 | 63.1 |
| 3 | 12.3 | 100 |
| 4 | 14.2 | 80.4 |
| 5 | 15.4 | 15.3 |
| 6 | 16.3 | 54.8 |
| 7 | 17.4 | 4.1 |
| 8 | 18.8 | 60.4 |
| 9 | 19.2 | 20.1 |
| 10 | 19.5 | 18.8 |
| 11 | 19.9 | 93.8 |
| 12 | 20.5 | 8.8 |
| 13 | 21.4 | 63.3 |
| 14 | 22.2 | 42.5 |
| 15 | 23.4 | 18.5 |
| 16 | 23.9 | 80.1 |
| 17 | 24.8 | 12.8 |
| 18 | 25.8 | 10.5 |
| 19 | 26.2 | 21.9 |
| 20 | 26.7 | 20.8 |
| 21 | 27.9 | 7.7 |
| 22 | 28.8 | 14.8 |
| 23 | 29.2 | 16.4 |
| 24 | 31.0 | 6 |
| 25 | 31.7 | 4.5 |
| 26 | 32.2 | 5.1 |
| 27 | 32.9 | 6.8 |
| 28 | 34.2 | 5.4 |
| 29 | 34.6 | 6.2 |
| 30 | 35.9 | 3.5 |
| 31 | 36.4 | 3.7 |
| 32 | 37.4 | 4 |

### PLM analysis

The collected crystal form X of compound A was subjected to PLM analysis by General method 2. It was observed that the sample was microcrystalline.

### ¹H NMR analysis

The ¹H NMR data of the crystal form X of compound A are as follows: ¹H NMR (400 MHz, DMSO-d₆) δ 10.27 (s, 1H), 8.90 (d, *J=* 1.5 Hz, 1H), 8.84 (d, *J=* 2.4 Hz, 1H), 8.75 (dd, *J* = 2.6, 1.5 Hz, 1H), 8.65 (d, *J* = 2.6 Hz, 1H), 8.24 (d, *J* = 2.4 Hz, 1H), 7.91 - 7.83 (m, 2H), 7.35 (d, *J* = 9.1 Hz, 2H), 5.23 (s, 1H), 3.58 - 3.47 (m, 1H), 3.36 (d, *J* = 6.7 Hz, 2H), 3.21 (t, *J* = 9.4 Hz, 1H), 2.04 (dd, *J* = 42.1, 12.2 Hz, 2H).

### DSC and TGA thermal analysis

The collected the crystal form X of compound A was subjected to DSC analysis by General method 4. DSC analysis showed a melting endothermic peak at about 172.54 °C with an onset temperature of 171.38 °C.

The crystal form X of compound A was subjected to TGA analysis by General method 5. TGA analysis showed a weight loss of about 3.6% prior to 200°C.

¹H NMR, DSC and TGA analysis indicated that the crystal form X of compound A was a solvate of tetrahydrofuran.

### Example 4.11 Characterization of the crystal form XI of compound A

The crystal form XI of compound A was obtained by cooling crystallization in acetonitrile according to the method in Example 3.5, and characterized by XRPD, PLM, ¹H NMR, DSC and TGA. The specific results are as follows:

### XRPD analysis

FIG. 28 shows the XRPD data of the crystal form XI of compound A acquired according to General method 1. A list of XRPD peaks at diffraction angles 2θ° (°2θ)±0.2 °2θ and relative intensities thereof are provided in Table 4.11.

**Table 4.11: XRPD peak list (2θ°) of the crystal form XI of compound A**

| Peak | Angle °2θ±0.2 °2θ | Relative intensity % |
|---|---|---|
| 1 | 7.6 | 10.4 |
| 2 | 9.7 | 73.3 |
| 3 | 11.4 | 1.6 |
| 4 | 12.7 | 11.7 |
| 5 | 13.2 | 14.7 |
| 6 | 13.7 | 1.5 |
| 7 | 14.6 | 5.2 |
| 8 | 16.7 | 6.1 |
| 9 | 17.9 | 100 |
| 10 | 18.7 | 18.4 |
| 11 | 18.9 | 11 |
| 12 | 19.5 | 41.4 |
| 13 | 20.2 | 5.5 |
| 14 | 20.7 | 8.1 |
| 15 | 21.0 | 8 |
| 16 | 21.3 | 5.5 |
| 17 | 21.5 | 4.3 |
| 18 | 22.4 | 10.5 |
| 19 | 24.2 | 32.4 |
| 20 | 24.8 | 33.8 |
| 21 | 25.6 | 19.8 |
| 22 | 26.0 | 9 |
| 23 | 26.6 | 1.4 |
| 24 | 27.2 | 1.8 |
| 25 | 27.7 | 1.2 |
| 26 | 28.7 | 1.1 |
| 27 | 29.4 | 4.5 |
| 28 | 29.9 | 6.3 |
| 29 | 30.2 | 2.8 |
| 30 | 30.5 | 2 |
| 31 | 31.1 | 4.7 |
| 32 | 31.4 | 3.8 |
| 33 | 31.7 | 1 |
| 34 | 32.8 | 1.5 |
| 35 | 33.5 | 0.8 |
| 36 | 35.3 | 0.8 |
| 37 | 35.9 | 1.3 |
| 38 | 36.2 | 1.4 |
| 39 | 36.6 | 1.6 |
| 40 | 37.4 | 1 |
| 41 | 39.2 | 1 |
| 42 | 39.7 | 0.8 |

### PLM analysis

The collected crystal form XI of compound A was subjected to PLM analysis by General method 2. It was observed that the sample was microcrystalline.

### ¹H NMR analysis

The ¹H NMR data of the crystal form XI of compound A are as follows: ¹H NMR (400 MHz, DMSO-d₆) δ 10.27 (s, 1H), 8.90 (d, *J=* 1.5 Hz, 1H), 8.84 (d, *J=* 2.3 Hz, 1H), 8.75 (dd, *J* = 2.5, 1.5 Hz, 1H), 8.65 (d, *J* = 2.6 Hz, 1H), 8.24 (d, *J* = 2.3 Hz, 1H), 7.91 - 7.83 (m, 2H), 7.35 (d, *J* = 9.1 Hz, 2H), 5.30 (d, *J* = 53.2 Hz, 1H), 3.58 - 3.45 (m, 1H), 3.36 (d, *J* = 6.7 Hz, 2H), 3.21 (t, *J* = 9.4 Hz, 1H), 2.16 - 1.92 (m, 2H).

### DSC and TGA thermal analysis

The collected crystal form XI of compound A was subjected to DSC analysis by General method 4. DSC analysis showed a melting endothermic peak at 174.45 °C with an onset temperature of 172.7 °C.

The crystal form XI of compound A was subj ected to TGA analysis by General method 5. TGA analysis showed a weight loss of about 3.9% prior to 200°C.

¹H NMR, DSC and TGA analysis indicated that the crystal form XI of compound A was a solvate of acetonitrile.

### Example 5 Grinding research of the crystal form III, the crystal form VI and the crystal form VII of compound A

A certain amount of the crystal form III, the crystal form VI and the crystal form VII of compound A was added into a mortar and ground at room temperature. The solid XRPDs before and after grinding were compared.

The XRPD results showed that after grinding, the crystallinity of the crystal form VI (anhydrous crystal form) was slightly decreased, while the crystal form III (hydrate) and the crystal form VII (hydrate) were transformed into amorphous forms, as shown in FIG. 30.

### Example 6 Preparation of crystalline salts of compound A

The initial attempt to prepare crystalline salts of compound A, using the crystal form I of compound A as the starting material, was consisted of two stages. The first stage included a solubility study of the starting material and a salt-forming screening in a 96-well plate. In the second stage, the possible crystalline salts were prepared in milligram scale. These initial attempts identified five crystal forms of salts of compound A, namely the crystal form I of compound A hydrochloride, the crystal form I of compound A hydrobromide, the crystal form I of compound A mesylate, the crystal form I and the crystal form II of compound A p-toluenesulfonate, and the crystal form I of compound A benzenesulfonate.

### Example 6.1 Solubility study of the starting material (the crystal form I of compound A)

About 2 mg of the starting material was weighed and added into a sample bottle, and different solvents were added respectively, until the starting material was dissolved. The solubility results are listed in Table 6.1. The results show that the starting materials have good solubility in tetrahydrofuran, acetone, 2-butanone, acetonitrile and ethyl acetate.

**Table 6.1: Solubility of the starting material (the crystal form I of compound A) in different solvents**

| Solvent | Solubility (mg/mL) |
|---|---|
| Tetrahydrofuran | ∼121 |
| Acetone | ∼77 |
| 2-Butanone | ∼73.67 |
| Acetonitrile | ∼53.25 |
| Ethyl Acetate | ∼49.25 |
| Isopropyl Acetate | ∼22.1 |
| Methanol | ∼11.83 |
| Methyl Tert-Butyl Ether | ∼2.8 |
| Ethanol | ∼1.31 |
| Isopropyl Alcohol | ∼0.92 |
| Isobutanol | ∼0.73 |
| Toluene | ∼1.23 |
| Water | <<0.55 |

### Example 6.2 Salt-forming screening in a 96-well plate

A certain amount of hydrochloric acid, hydrobromic acid, sulfuric acid, p-toluenesulfonic acid, methanesulfonic acid, benzenesulfonic acid, maleic acid and phosphoric acid were dissolved in 10 mL of methanol respectively to prepare eight acid solutions with a concentration of 0.1 M. 364.3 mg of the starting material was dissolved in acetone and the solution was diluted with acetone to 12 mL to prepare a solution of the starting material with a concentration of 30 mg/mL. The 30 mg/mL solution of the starting material was dispensed into a 96-well plate in a volume of 100 µL per well. The eight acid solutions prepared above were then added to the wells of each row in a volume of 65 µL (33 µL for sulfuric acid) per well. After the solvent was completely evaporated, 200 µL of solvent (solvent for salt-forming was methanol, ethanol, isopropanol, 2-butanone, isobutanol, tetrahydrofuran, acetonitrile, methyl tert-butyl ether, acetone, water, ethyl acetate and isopropyl acetate) was added to each well. The solutions were sealed with a punctured sealing film, and allowed to evaporate to dryness in a fume hood at room temperature. One sample from each column was selected for ¹H NMR testing to determine whether the salt was formed. XRPD test was performed on the solid sample to determine whether the solid was a crystal form.

¹H NMR results showed that the samples obtained by reacting the free base of compound A with hydrochloric acid, hydrobromic acid, methanesulfonic acid, p-toluenesulfonic acid and benzenesulfonic acid had chemical shifts. The XRPD results showed that the samples obtained by the reaction with the above acids were crystals. The results are listed in Table 6.2.

**Table 6.2: Results of salt-forming screening in a 96-well plate**

| | | A | B | C | D | E | F | G | H |
|---|---|---|---|---|---|---|---|---|---|
| | | Hydrochl one acid | Sulfur ic acid | Hydrobro mic acid | Benzenesulf onic acid | Methanesulf onic acid | P-toluenesulf onic acid | Phospho ric acid | Male ic acid |
| 1 | Methanol | Solid | | | | Solid | Solid | | Solid |
| 2 | Ethanol | | | | | | | | |
| 3 | Isopropyl alcohol | | | | | Glassy | | | Glas sy |
| 4 | 2-Butanone | | | | | Solid | Glassy | Glassy | |
| 5 | Isobutanol | | | | | | Solid | | |
| 6 | Tetrahydrof uran | | | | | Glassy | | | |
| 7 | Acetonitrile | | | | | | | | |
| 8 | Methyl tert-butyl ether | | | | | Solid | | Solid | |
| 9 | Acetone | | | | | Glassy | Glassy | | |
| 1 0 | Water | | | | | Solid | | | Solid |
| 11 | Ethyl acetate | | | | | | Solid | Glassy | |
| 1 2 | Isopropyl acetate | | | | | | | | |

### Example 6.3 Small-scale preparation of the crystal form I of compound A hydrochloride

About 30 mg of the starting material (the crystal form I of compound A) was weighed and dissolved in different volumes of five solvents (acetone, ethyl acetate, acetonitrile, 2-butanone and isopropyl acetate) to form solutions. To each solution was added 5.93 µL or 16 µL of concentrated hydrochloric acid, and the mixture was stirred at room temperature for half a minute. Solids were precipitated from all solutions. The mixture was stirred for another 30 minutes and then filtered. The reaction conditions and results are listed in Table 6.3. XRPD results showed that the hydrochloride crystal was prepared.

**Table 6.3: Preparation conditions and results of the crystal form I of compound A hydrochloride**

| Batch No. | Solvent | Solvent volume (µL) | Hydrochloric acid (µL) | Result |
|---|---|---|---|---|
| HCl-S1 | Acetone | 300 | 5.93 | XRPD: Crystal, Form I. |
| HCl-S2 | Ethyl acetate | 800 | 5.93 | XRPD: Crystal, Form I. |
| HCl-S3 | Acetonitrile | 900 | 5.93 | XRPD: Crystal, Form I. |
| HCl-S4 | 2-butanone | 500 | 5.93 | XRPD: Crystal, Form I. |
| HCl-S5 | Isopropyl acetate | 1800 | 5.93 | XRPD: Crystal, Form I. |
| HCl-S6 | Ethyl acetate | 800 | 16 | XRPD: Crystal, Form I. |

### Example 6.4 Small-scale preparation of the crystal form I of compound A benzenesulfonate

About 30 mg of the starting material (the crystal form I of compound A) was weighed and dissolved in different volumes of five solvents (acetone, ethyl acetate, acetonitrile, 2-butanone and isopropyl acetate) to form solutions. To each solution was added about 13 mg of benzenesulfonic acid, and the mixture was stirred at room temperature for half a minute. Solids were precipitated from all solutions. The mixture was stirred for another 30 minutes and then filtered. The reaction conditions and results are listed in Table 6.4. XRPD results showed that the benzenesulfonate crystal was prepared.

**Table 6.4 Preparation conditions and results of the crystal form I of compound A benzenesulfonate**

| Batch No. | Solvent | Solvent volume (µL) | Result |
|---|---|---|---|
| PhSO₃H-S1 | Acetone | 300 | XRPD: Crystal, Form I. |
| PhSO₃H-S2 | Ethyl acetate | 800 | XRPD: Crystal, Form I. |
| PhSO₃H-S3 | Acetonitrile | 900 | XRPD: Crystal, Form I. |
| PhSO₃H-S4 | 2-butanone | 500 | XRPD: Crystal, Form I. |
| PhSO₃H-S5 | Isopropyl acetate | 2000 | XRPD: Crystal, Form I. |

### Example 6.5 Small-scale preparation of the crystal form I and the crystal form II of compound A p-toluenesulfonate

About 30 mg of the starting material (the crystal form I of compound A) was weighed and dissolved in different volumes of five solvents (acetone, ethyl acetate, acetonitrile, 2-butanone and isopropyl acetate) to form solutions. To each solution was added about 13 mL of a 1 M solution of p-toluenesulfonic acid in methanol, and the mixture was stirred at room temperature for half a minute. Solids were precipitated from all solutions. The mixture was stirred for another 30 minutes and then filtered. The reaction conditions and results are listed in Table 6.5. XRPD results showed that two crystalline forms of p-toluenesulfonate were prepared, named as the crystal form I of the p-toluenesulfonate and the crystal form II of the p-toluenesulfonate.

**Table 6.5 Preparation conditions and results of the crystal form I and the crystal form II of compound A p-toluenesulfonate**

| Batch No. | Solvent | Solvent volume (µL) | Result |
|---|---|---|---|
| PTSA-S1 | Acetone | 300 | XRPD: Crystal, Form I. |
| PTSA-S2 | Ethyl acetate | 800 | XRPD: Crystal, Form II. |
| PTSA-S3 | Acetonitrile | 900 | XRPD: Crystal, Form I. |
| PTSA-S4 | 2-butanone | 500 | XRPD: Crystal, Form II. |
| PTSA-S5 | Isopropyl acetate | 2000 | XRPD: Crystal, Form I. |

### Example 6.6 Scale-up preparation of the crystal form I of compound A p-toluenesulfonate

At room temperature, 500.1 mg of the starting material (the crystal form I of compound A) was added to 5 mL of acetone, followed by 1.1 mL of 1 M solution of p-toluenesulfonic acid in methanol. Solids formed after stirring for three minutes. After stirring for another 40 minutes, the mixture was filtered and dried at 50°C overnight. A total of 407.1 mg of salt was obtained in a yield of 81.4%. The results obtained are listed in Table 6.6.

**Table 6.6 Rerults of scale-up preparation of the crystal form I of compound A p-toluenesulfonate**

| Batch No. | PLM | XRPD | TGA | DSC |
|---|---|---|---|---|
| PTSA-A1 | irregular crystals | Crystal, Form I | 0.6% weight loss prior to 200°C | 236°C |

### Example 6.7 Small-scale preparation of the crystal form I of compound A mesylate

About 40.1 mg of the starting material (the crystal form I of compound A) was weighed and dissolved in 300 µL of acetone to form a solution. About 14 µL of methanesulfonic acid was then added, and the mixture was stirred at room temperature for 1 minute. Solids were precipitated from the solution. The mixture was stirred for another 30 minutes and then filtered. The resulting solid was left at 50°C overnight and then characterized. The reaction conditions and results are listed in Table 6.7.

**Table 6.7 Preparation conditions and results of the crystal form I of compound A mesylate**

| Batch No. | Solvent | Solvent volume (µL) | Result |
|---|---|---|---|
| CH₃SO₃H-S1 | Acetone | 300 | XRPD: Crystal, Form I. |

### Example 6.8 Small-scale preparation of the crystal form I of compound A hydrobromide

About 31.2 mg of the starting material (the crystal form I of compound A) was weighed and dissolved in 300 µL of acetone to form a solution. About 670 µL of about 0.1 M solution of hydrobromic acid in methanol was then added, and the mixture was stirred at room temperature for 1 hour. No solid was precipitated. The mixture was purged with nitrogen gas to dryness to become an oil. 300 µL of MTBE was added. The mixture was stirred for 1 hour, and then filtered. The resulting solid was left at 50°C overnight, and then characterized. The reaction conditions and results are listed in Table 6.8.

**Table 6.8 Preparation conditions and results of the crystal form I of compound A hydrobromide**

| Batch No. | Solvent | Solvent (µL) | Result |
|---|---|---|---|
| HBr-S 1 | Acetone/MTBE | 300 | XRPD: Crystal, Form I. |

### Example 7 Characterization of crystalline salts of compound A

### Example 7.1 Characterization of the crystal form I of compound A hydrochloride

### XRPD analysis

FIG. 31 shows the XRPD data of the crystal form I of compound A hydrochloride acquired according to General method 1. A list of XRPD peaks at diffraction angles 2θ° (°2θ)±0.2 °2θ and relative intensities thereof are provided in Table 7.1.

**Table 7.1: XRPD peak list (2θ°) of the crystal form I of compound A hydrochloride**

| Peak | Angle °2θ±0.2 °2θ | Relative intensity |
|---|---|---|
| 1 | 6.5 | 86.2 |
| 2 | 11.3 | 25.4 |
| 3 | 12.2 | 8.8 |
| 4 | 13.1 | 13.3 |
| 5 | 13.8 | 90.2 |
| 6 | 15.7 | 29.8 |
| 7 | 16.4 | 32.7 |
| 8 | 16.8 | 15.2 |
| 9 | 18.7 | 100 |
| 10 | 19.3 | 12.8 |
| 11 | 19.7 | 15.2 |
| 12 | 20.3 | 12.1 |
| 13 | 20.8 | 24.9 |
| 14 | 21.3 | 23.7 |
| 15 | 21.9 | 31.6 |
| 16 | 22.7 | 33.5 |
| 17 | 23.3 | 57.7 |
| 18 | 23.9 | 8.2 |
| 19 | 24.2 | 37.9 |
| 20 | 24.6 | 13.8 |
| 21 | 25.2 | 15.5 |
| 22 | 26.3 | 7.1 |
| 23 | 27.8 | 10.8 |
| 24 | 28.6 | 32.7 |
| 25 | 29.3 | 12 |
| 26 | 30.9 | 8.2 |
| 27 | 31.8 | 9.9 |
| 28 | 35.0 | 15.3 |
| 29 | 37.2 | 6.7 |
| 30 | 38.7 | 5.9 |

### PLM analysis

The collected crystal form I of compound A hydrochloride prepared according to the method in Example 6.3 was subjected to PLM analysis by General method 2. It was observed that both the samples of HCl-S1 and HCl-S2 batches were crystals of irregular sheet-like particles.

### ¹H NMR analysis

The ¹H NMR of the crystal form I of compound A hydrochloride from the HCl-S1 batch prepared by the method in Example 6.3 is as follows: ¹H NMR (400 MHz, DMSO-d₆) δ 10.49 (s, 1H), 8.95 (d, J = 1.4 Hz, 1H), 8.86 (d, J = 2.3 Hz, 1H), 8.75 (dd, J = 2.5, 1.5 Hz, 1H), 8.67 (d, J = 2.6 Hz, 1H), 8.35 (d, J = 2.2 Hz, 1H), 7.96 - 7.87 (m, 2H), 7.34 (d, J = 9.1 Hz, 2H), 5.31 (d, J = 53.3 Hz, 1H), 3.54 (ddd, J = 39.4, 13.3, 3.0 Hz, 1H), 3.34 (dt, J = 12.2, 9.0 Hz, 2H), 3.25 (t, J = 9.5 Hz, 1H), 2.19 - 1.94 (m, 2H).

The ¹H NMR of the crystal form I of compound A is as follows: ¹H NMR (300 MHz, DMSO-d₆) δ 10.27 (s, 1H), 8.90 (d, J = 1.4 Hz, 1H), 8.84 (d, J = 2.3 Hz, 1H), 8.75 (dd, J = 2.5, 1.5 Hz, 1H), 8.65 (d, J = 2.6 Hz, 1H), 8.25 (d, J = 2.3 Hz, 1H), 7.93 - 7.79 (m, 2H), 7.35 (d, J = 9.0 Hz, 2H), 5.30 (d, J = 53.4 Hz, 1H), 3.63 - 3.45 (m, 1H), 3.41 - 3.34 (m, 2H), 3.27 - 3.18 (m, 1H), 2.20 - 1.87 (m, 2H).

Compared with the ¹H NMR of the crystal form I of compound A, the ¹H NMR of the crystal form I of compound A hydrochloride has chemical shifts, confirming that it is a salt form.

### DSC and TGA thermal analysis

The collected crystal form I of compound A hydrochloride from the HCl-S1 batch prepared according to the method in Example 6.3 was subjected to DSC analysis by General method 4. In the DSC, the sample of the crystal form I of compound A hydrochloride showed a melting endothermic peak at 229.41 °C with an onset temperature of 209.08 °C.

The collected crystal form I of compound A hydrochloride from the HCl-S1 batch was subjected to TGA analysis by General method 5. In the TGA, the sample of the crystal form I of compound A hydrochloride had a weight loss of 0.6752% prior to 175 °C.

DSC and TGA analysis indicated that the crystal form I of compound A hydrochloride was an anhydrous crystal form.

### DVS analysis

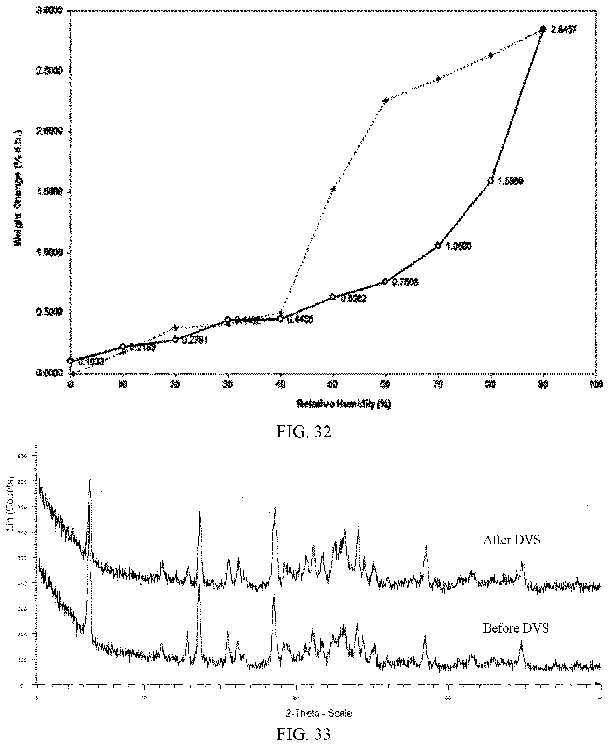

FIG. 32 shows the DVS curve of the crystal form I of compound A hydrochloride from the HCl-S1 batch prepared according to the method in Example 6.3 acquired by General method 6. In the DVS, the sample of the crystal form I of compound A hydrochloride was slightly hygroscopic. The water absorption was about 0.4% under the condition of 0-40%RH (relative humidity) and about 2.8% under the condition of 0-90%RH, as shown in FIG. 32.

The crystal form I of compound A hydrochloride was not changed after the sample was tested by DVS, as shown in FIG. 33.

### Example 7.2 Characterization of the crystal form I of compound A benzenesulfonate

### XRPD analysis

FIG. 34 shows the XRPD data of the crystal form I of compound A benzenesulfonate from the PhSO₃H-S1 batch in Example 6.4 acquired according to General method 1. A list of XRPD peaks at diffraction angles 2θ° (°2θ)±0.2 °2θ and relative intensities thereof are provided in Table 7.2.

**Table 7.2: XRPD peak list (2θ°) of the crystal form I of compound A benzenesulfonate**

| Peak | Angle °2θ±0.2 °2θ | Relative intensity |
|---|---|---|
| 1 | 7.6 | 50.1 |
| 2 | 10.2 | 100 |
| 3 | 11.4 | 4.3 |
| 4 | 14.3 | 6.6 |
| 5 | 14.7 | 6.8 |
| 6 | 15.3 | 11.8 |
| 7 | 16.4 | 46.3 |
| 8 | 17.4 | 12.8 |
| 9 | 17.9 | 11.9 |
| 10 | 18.3 | 12.3 |
| 11 | 18.9 | 6.6 |
| 12 | 19.1 | 27.7 |
| 13 | 19.9 | 26.9 |
| 14 | 20.5 | 17.8 |
| 15 | 20.7 | 11.9 |
| 16 | 21.3 | 25.9 |
| 17 | 21.6 | 23 |
| 18 | 21.9 | 25.6 |
| 19 | 22.8 | 69.2 |
| 20 | 23.3 | 43.1 |
| 21 | 25.0 | 15.2 |
| 22 | 25.3 | 8.2 |
| 23 | 26.3 | 4.2 |
| 24 | 26.8 | 8.8 |
| 25 | 28.0 | 10.1 |
| 26 | 29.1 | 13.4 |
| 27 | 30.4 | 8.4 |
| 28 | 31.5 | 3.9 |

### PLM analysis

The collected crystal form I of compound A benzenesulfonate from the PhSO₃H-S1 batch in Example 6.4 was subjected to PLM analysis by General method 2. It was observed that the sample from the PhSO₃H-S1 batch was irregular sheet-like particles mixed with a small amount of needle-like crystals.

### ¹H NMR analysis

The ¹H NMR data of the crystal form I of compound A benzenesulfonate from the PhSO₃H-S1 batch in Example 6.4 is as follows: ¹H NMR (400 MHz, DMSO-d₆) δ 10.29 (s, 1H), 8.91 (d, J = 1.5 Hz, 1H), 8.83 (d, J = 2.4 Hz, 1H), 8.75 (dd, J = 2.6, 1.5 Hz, 1H), 8.66 (d, J = 2.6 Hz, 1H), 8.26 (d, J = 2.3 Hz, 1H), 7.91 - 7.83 (m, 2H), 7.59 (dd, J = 7.6, 2.0 Hz, 2H), 7.35 (d, J = 9.0 Hz, 2H), 7.32 - 7.30 (m, 3H), 5.30 (d, J = 53.4 Hz, 1H), 3.52 (ddd, J = 39.4, 13.2, 3.1 Hz, 1H), 3.39 - 3.26 (m, 2H), 3.22 (t, J = 9.3 Hz, 1H), 2.03 (dd, J = 54.1, 12.1 Hz, 2H).

Compared with the ¹H NMR of the crystal form I of compound A, the ¹H NMR results of the crystal form I of compound A benzenesulfonate showed that this batch of samples had chemical shifts, and the ratio of the free base to benzenesulfonate was 1:1.

### DSC and TGA thermal analysis

The collected crystal form I of compound A benzenesulfonate from the PhSO₃H-S1 batch in Example 6.4 was subjected to DSC analysis by General method 4. In the DSC, the sample of the crystal form I of compound A benzenesulfonate showed a narrow melting endothermic peak at 252.81 °C with an onset temperature of 252.22 °C.

The collected crystal form I of compound A benzenesulfonate from the PhSO₃H-S1 batch was subjected to TGA analysis by General method 5. In the TGA, the sample of the crystal form I of compound A benzenesulfonate had a weight loss of 0.4773% prior to 200 °C.

DSC and TGA analysis indicated that the crystal form I of compound A benzenesulfonate was an anhydrous crystal form.

### DVS analysis

FIG. 35 shows the DVS curve of the crystal form I of compound A benzenesulfonate from the PhSO₃H-S1 batch in Example 6.4 acquired by General method 6. In the DVS, the sample of the crystal form I of compound A benzenesulfonate was slightly hygroscopic, and the water absorption was about 0.4% under the condition of 0-60%RH and about 1.5% under the condition of 0-90%RH.

The crystal form I of compound A benzenesulfonate was not changed after the sample was tested by DVS, as shown in FIG. 36.

### Example 7.3 Characterization of the crystal form I of compound A p-toluenesulfonate

### XRPD analysis

FIG. 37 shows the XRPD data of the crystal form I of compound A p-toluenesulfonate from the PTAS-S1 batch in Example 6.5 acquired according to General method 1. A list of XRPD peaks at diffraction angles 2θ° (°2θ)±0.2 °2θ and relative intensities thereof are provided in Table 7.3.

**Table 7.3: XRPD peak list (2θ°) of the crystal form I of compound A p-toluenesulfonate**

| Peak | Angle °2θ±0.2 °2θ | Relative intensity |
|---|---|---|
| 1 | 6.8 | 4.8 |
| 2 | 7.4 | 63.7 |
| 3 | 10.2 | 100 |
| 4 | 11.9 | 3.1 |
| 5 | 14.5 | 7.4 |
| 6 | 14.9 | 23.4 |
| 7 | 16.3 | 24.4 |
| 8 | 17.3 | 7.1 |
| 9 | 17.7 | 4.4 |
| 10 | 18.6 | 12.9 |
| 11 | 18.9 | 14.1 |
| 12 | 19.2 | 23.8 |
| 13 | 20.1 | 3.6 |
| 14 | 20.6 | 4.7 |
| 15 | 21.3 | 54.5 |
| 16 | 21.9 | 92.2 |
| 17 | 22.5 | 12.4 |
| 18 | 23.0 | 19.1 |
| 19 | 23.3 | 6.5 |
| 20 | 24.2 | 9.3 |
| 21 | 24.9 | 5.2 |
| 22 | 25.8 | 3.3 |
| 23 | 26.9 | 4.7 |
| 24 | 27.9 | 34.3 |
| 25 | 28.3 | 4.5 |
| 26 | 28.6 | 3.8 |
| 27 | 29.4 | 12.9 |
| 28 | 29.9 | 2.4 |
| 29 | 31.1 | 4.5 |
| 30 | 31.5 | 12.3 |
| 31 | 32.8 | 3.9 |
| 32 | 33.4 | 3 |
| 33 | 34.1 | 6.8 |
| 34 | 35.8 | 3.8 |
| 35 | 38.0 | 2.2 |

### PLM analysis

The collected crystal form I of compound A p-toluenesulfonate from the PTSA-S1 batch in Example 6.5 was subjected to PLM analysis by General method 2. It was observed that the sample from this batch was needle-like crystals.

### ¹H NMR analysis

The ¹H NMR data of the crystal form I of compound A p-toluenesulfonate from the PTSA-S1 batch in Example 6.5 is as follows: ¹H NMR (400 MHz, DMSO-d₆) δ 10.29 (s, 1H), 8.91 (d, J = 1.5 Hz, 1H), 8.83 (d, J = 2.3 Hz, 1H), 8.75 (dd, J = 2.5, 1.5 Hz, 1H), 8.66 (d, J = 2.6 Hz, 1H), 8.26 (d, J = 2.3 Hz, 1H), 7.91 - 7.84 (m, 2H), 7.47 (d, J = 8.1 Hz, 2H), 7.35 (d, J = 9.1 Hz, 2H), 7.11 (d, J = 7.8 Hz, 2H), 5.30 (d, J = 53.2 Hz, 1H), 3.52 (ddd, J = 39.7, 13.4, 3.2 Hz, 1H), 3.32 (dt, J = 22.8, 9.3 Hz, 2H), 3.23 (t, J = 9.6 Hz, 1H), 2.29 (s, 3H), 2.15 - 1.93 (m, 2H).

Compared with the ¹H NMR of the crystal form I of compound A, the ¹H NMR results of the crystal form I of compound A p-toluenesulfonate shows that this batch of samples has chemical shifts, and the ratio of the free base to p-toluenesulfonate is 1:1.

### DSC and TGA thermal analysis

The collected crystal form I of compound A p-toluenesulfonate from the PTSA-S1 batch in Example 6.5 was subjected to DSC analysis by General method 4. In the DSC, the sample of the crystal form I of compound A p-toluenesulfonate showed a narrow melting endothermic peak at 236.18 °C with an onset temperature of 234.56 °C.

The collected crystal form I of compound A p-toluenesulfonate from the PTSA-S1 batch was subjected to TGA analysis by General method 5. In the TGA, the sample of the crystal form I of compound A p-toluenesulfonate had a weight loss of 0.1415% prior to 200 °C.

DSC and TGA analysis indicated that the crystal form I of compound A p-toluenesulfonate was an anhydrous crystal form.

### DVS analysis

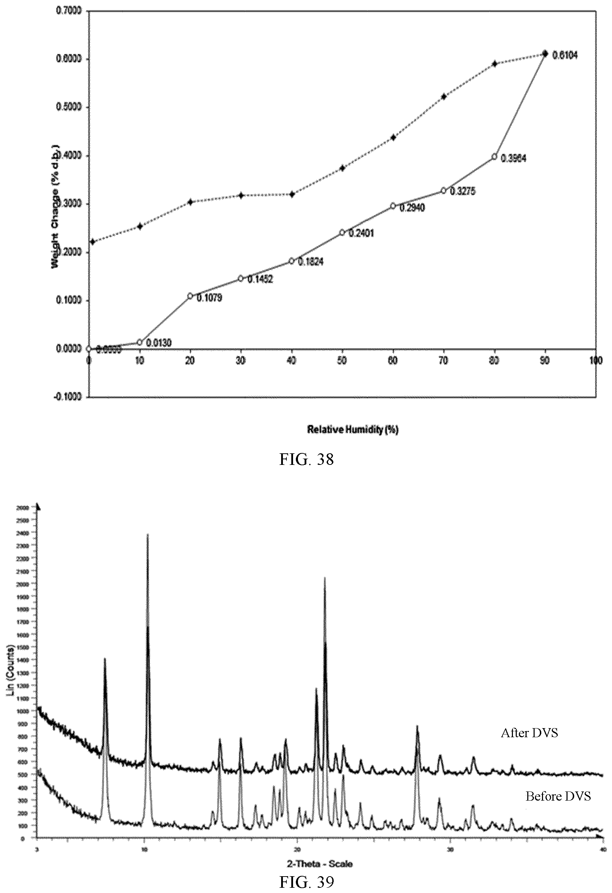

FIG. 38 shows the DVS curve of the crystal form I of compound A p-toluenesulfonate from the PTSA-S1 batch in Example 6.5 acquired by General method 6. In the DVS, the sample of the crystal form I of compound A p-toluenesulfonate was slightly hygroscopic, and the water absorption was about 0.39% under the condition of 0-80%RH and about 0.61% under the condition of 0-90%RH.

The crystal form I of compound Ap-toluenesulfonate was not changed after the sample was tested by DVS, as shown in FIG. 39.

### Example 7.4 Characterization of the crystal form II of compound A p-toluenesulfonate

### XRPD analysis

FIG. 40 shows the XRPD data of the crystal form II of compound A p-toluenesulfonate from the PTAS-S2 batch in Example 6.5 acquired according to General method 1. A list of XRPD peaks at diffraction angles 2θ° (°2θ)±0.2 °2θ and relative intensities thereof are provided in Table 7.4.

**Table 7.4: XRPD peak list (2θ°) of the crystal form II of compound A p-toluenesulfonate**

| Peak | Angle °2θ±0.2 °2θ | Relative intensity |
|---|---|---|
| 1 | 6.4 | 100 |
| 2 | 7.4 | 10.1 |
| 3 | 10.2 | 25.6 |
| 4 | 12.0 | 0.8 |
| 5 | 12.9 | 1.2 |
| 6 | 14.5 | 1 |
| 7 | 14.9 | 4.4 |
| 8 | 16.3 | 5.6 |
| 9 | 17.3 | 1.4 |
| 10 | 18.5 | 1.9 |
| 11 | 18.9 | 1.8 |
| 12 | 19.2 | 1.6 |
| 13 | 19.9 | 1.3 |
| 14 | 20.2 | 1 |
| 15 | 20.6 | 1.3 |
| 16 | 21.3 | 12.1 |
| 17 | 21.8 | 5.2 |
| 18 | 22.5 | 2 |
| 19 | 23.0 | 3.8 |
| 20 | 23.3 | 4 |
| 21 | 24.3 | 1.1 |
| 22 | 24.8 | 1 |
| 23 | 25.6 | 1.4 |
| 24 | 26.3 | 3.2 |
| 25 | 27.5 | 1.8 |
| 26 | 27.8 | 3.5 |
| 27 | 29.4 | 1.3 |
| 28 | 31.0 | 0.8 |
| 29 | 31.5 | 2.2 |
| 30 | 31.9 | 1 |
| 31 | 32.6 | 1.6 |
| 32 | 34.0 | 1.1 |
| 33 | 36.2 | 1.2 |
| 34 | 38.4 | 0.8 |

### PLM analysis

The collected crystal form II of compound A p-toluenesulfonate from the PTSA-S2 batch in Example 6.5 was subjected to PLM analysis by General method 2. It was observed that the sample from this batch was irregular crystals.

### ¹H NMR analysis

The ¹H NMR data of the crystal form II of compound A p-toluenesulfonate from the PTSA-S2 batch in Example 6.5 is as follows: ¹H NMR (400 MHz, DMSO-d₆) δ 10.30 (s, 1H), 8.91 (d, J = 1.2 Hz, 1H), 8.83 (d, J = 2.2 Hz, 1H), 8.75 (dd, J = 2.5, 1.5 Hz, 1H), 8.66 (d, J = 2.5 Hz, 1H), 8.27 (s, 1H), 7.92 - 7.83 (m, 2H), 7.48 (d, J = 8.0 Hz, 2H), 7.35 (d, J = 9.1 Hz, 2H), 7.12 (d, J = 7.9 Hz, 2H), 5.30 (d, J = 53.2 Hz, 1H), 3.60 - 3.44 (m, 1H), 3.39 - 3.29 (m, 2H), 3.23 (t, J = 9.5 Hz, 1H), 2.29 (s, 3H), 2.17 - 1.94 (m, 2H).

Compared with the ¹H NMR of the crystal form I of compound A, the ¹H NMR results of the crystal form II of compound A p-toluenesulfonate shows that this batch of samples has chemical shifts, and the ratio of the free base to p-toluenesulfonate is 1:1.

### DSC and TGA thermal analysis

The collected the crystal form II of compound A p-toluenesulfonate from the PTSA-S2 batch in Example 6.5 was subjected to DSC analysis by General method 4. In the DSC, the sample of the crystal form II of compound A p-toluenesulfonate showed a narrow melting endothermic peak at 234.45 °C with an onset temperature of 232.18 °C.

The collected crystal form II of compound A p-toluenesulfonate from the PTSA-S2 batch in Example 6.5 was subjected to TGA analysis by General method 5. In the TGA, the sample of the crystal form II of compound A p-toluenesulfonate had a weight loss of 0.2437% prior to 200 °C.

DSC and TGA analysis indicated that the crystal form II of compound A p-toluenesulfonate was an anhydrous crystal form.

### Example 7.5 Characterization of the crystal form I of compound A mesylate

### XRPD analysis

FIG. 41 shows the XRPD data of the crystal form I of compound A mesylate from the CH₃SO₃H-S1 batch in Example 6.7 acquired according to General method 1. A list of XRPD peaks at diffraction angles 2θ° (°2θ)±0.2 °2θ and relative intensities thereof are provided in Table 7.5.

**Table 7.5: XRPD peak list (2θ°) of the crystal form I of compound A mesylate**

| Peak | Angle °2θ±0.2 °2θ | Relative intensity % |
|---|---|---|
| 1 | 4.1 | 7.8 |
| 2 | 4.7 | 32.6 |
| 3 | 9.5 | 10.5 |
| 4 | 11.3 | 4.8 |
| 5 | 11.6 | 7.1 |
| 6 | 13.1 | 10.3 |
| 7 | 13.7 | 3.8 |
| 8 | 15.3 | 4.3 |
| 9 | 16.4 | 32.5 |
| 10 | 17.1 | 3.9 |
| 11 | 18.7 | 48.5 |
| 12 | 19.2 | 49.8 |
| 13 | 19.7 | 74.8 |
| 14 | 20.1 | 19.4 |
| 15 | 21.5 | 7.7 |
| 16 | 22.3 | 28.1 |
| 17 | 22.7 | 42.3 |
| 18 | 23.7 | 100 |
| 19 | 24.5 | 8.9 |
| 20 | 24.9 | 11.1 |
| 21 | 25.4 | 8.1 |
| 22 | 27.0 | 4 |
| 23 | 28.1 | 19.1 |
| 24 | 28.7 | 9.1 |
| 25 | 29.0 | 7.5 |
| 26 | 29.3 | 9 |
| 27 | 29.9 | 3.9 |
| 28 | 30.3 | 5.4 |
| 29 | 30.7 | 6.1 |
| 30 | 31.0 | 3.7 |
| 31 | 32.5 | 4.5 |
| 32 | 32.9 | 4.1 |
| 33 | 34.0 | 5.1 |
| 34 | 34.6 | 4.9 |
| 35 | 37.1 | 17 |
| 36 | 38.9 | 3.8 |
| 37 | 39.2 | 3.1 |

### PLM analysis

The collected crystal form I of compound A mesylate from the CH₃SO₃H-S1 batch in Example 6.7 was subjected to PLM analysis by General method 2. It was observed that the sample from this batch was irregular crystals.

### ¹H NMR analysis

The ¹H NMR data of the crystal form I of compound A mesylate from the CH₃SO₃H-S1 batch in Example 6.7 is as follows: ¹H NMR (400 MHz, DMSO-d₆) δ 10.29 (s, 1H), 8.90 (d, J = 1.5 Hz, 1H), 8.83 (d, J = 2.3 Hz, 1H), 8.75 (dd, J = 2.6, 1.5 Hz, 1H), 8.66 (d, J = 2.6 Hz, 1H), 8.26 (d, J = 2.3 Hz, 1H), 7.91 - 7.83 (m, 2H), 7.35 (d, J = 9.1 Hz, 2H), 5.30 (d, J = 53.4 Hz, 1H), 3.52 (ddd, J = 39.9, 13.6, 3.3 Hz, 1H), 3.38 - 3.27 (m, 2H), 3.22 (t, J = 9.5 Hz, 1H), 2.34 (d, J = 1.4 Hz, 3H), 2.03 (dd, J = 54.4, 11.6 Hz, 2H).

Compared with the ¹H NMR of the crystal form I of compound A, the ¹H NMR results of the crystal form I of compound A mesylate shows that this batch of samples has chemical shifts, and the ratio of the free base to mesylate is 1:2.

### DSC and TGA thermal analysis

The collected crystal form I of compound A mesylate from the CH₃SO₃H-S1 batch in Example 6.7 was subjected to DSC analysis by General method 4. In the DSC, the crystal form I of compound A mesylate showed a narrow melting endothermic peak at 206.37 °C with an onset temperature of 204.41 °C.

The collected crystal form I of compound A mesylate from the CH₃SO₃H-S1 batch in Example 6.7 was subjected to TGA analysis by General method 5. In the TGA, the sample of the crystal form I of compound A mesylate had a weight loss of 0.6541% prior to 200 °C.

DSC and TGA analysis indicated that the crystal form I of compound A mesylate was an anhydrous crystal form.

### DVS analysis

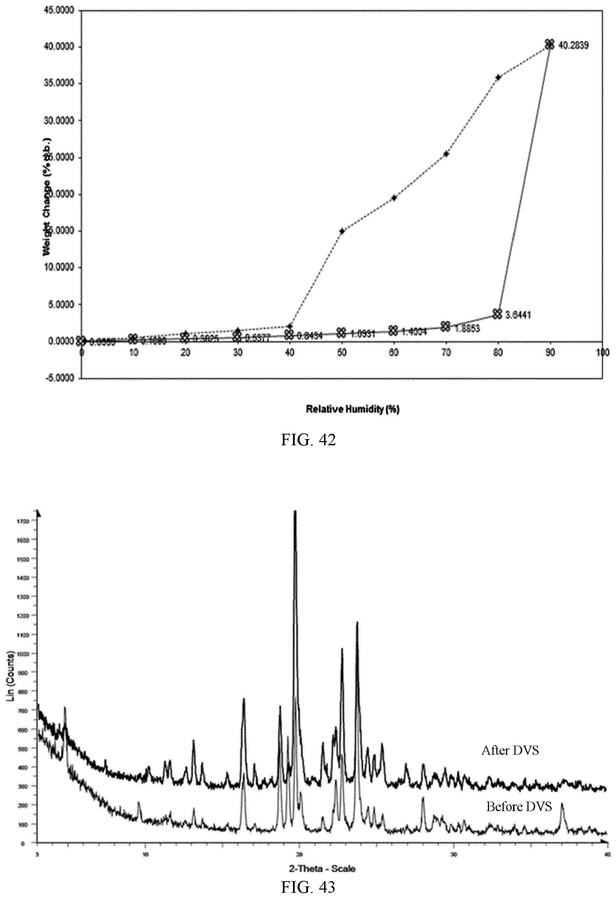

FIG. 42 shows the DVS curve of the crystal form I of compound A mesylate from the CH₃SO₃H-S1 batch in Example 6.7 acquired by General method 6. In the DVS, the sample of the crystal form I of compound A mesylate was hygroscopic, and the water absorption was about 3.6% under the condition of 0-80%RH and about 40.3% under the condition of 0-90%RH.

The crystal form I of compound A mesylate was not changed after the sample was tested by DVS, as shown in FIG. 43.

### Example 7.6 Characterization of the crystal form I of compound A hydrobromide

### XRPD analysis

FIG. 44 shows the XRPD data of the crystal form I of compound A hydrobromide from the HBr-S 1 batch in Example 6.8 acquired according to General method 1. A list of XRPD peaks at diffraction angles 2θ° (°2θ)±0.2 °2θ and relative intensities thereof are provided in Table 7.6.

**Table 7.6: XRPD peak list (2θ°) of the crystal form I of compound A hydrobromide**

| Peak | Angle °2θ±0.2 °2θ | Relative intensity % |
|---|---|---|
| 1 | 6.4 | 100 |
| 2 | 11.3 | 6.5 |
| 3 | 11.8 | 6.2 |
| 4 | 13.0 | 18.3 |
| 5 | 13.5 | 30.7 |
| 6 | 14.6 | 8.2 |
| 7 | 15.8 | 10.8 |
| 8 | 18.6 | 9.4 |
| 9 | 20.6 | 34.9 |
| 10 | 21.1 | 25.5 |
| 11 | 21.8 | 12.7 |
| 12 | 22.7 | 9.6 |
| 13 | 23.3 | 6.2 |
| 14 | 23.8 | 31.1 |
| 15 | 24.0 | 40.2 |
| 16 | 26.1 | 31.2 |
| 17 | 26.7 | 5.3 |
| 18 | 28.5 | 19.4 |
| 19 | 29.4 | 6.9 |
| 20 | 31.7 | 7 |
| 21 | 33.3 | 12.4 |
| 22 | 34.1 | 5.6 |
| 23 | 35.8 | 7.8 |
| 24 | 36.9 | 5.3 |
| 25 | 37.1 | 6.4 |
| 26 | 39.6 | 7.2 |

### PLM analysis

The collected crystal form I of compound A hydrobromide from the HBr-S1 batch in Example 6.8 was subjected to PLM analysis by General method 2. It was observed that the sample from this batch was irregular crystals.

### ¹H NMR analysis

The ¹H NMR data of the crystal form I of compound A hydrobromide from the HBr-S1 batch in Example 6.8 is as follows: ¹H NMR (400 MHz, DMSO-d₆) δ 10.30 (s, 1H), 8.91 (d, J = 1.5 Hz, 1H), 8.83 (d, J = 2.3 Hz, 1H), 8.75 (dd, J = 2.5, 1.5 Hz, 1H), 8.66 (d, J = 2.6 Hz, 1H), 8.27 (d, J = 2.3 Hz, 1H), 7.91 - 7.84 (m, 2H), 7.35 (d, J = 9.1 Hz, 2H), 5.30 (d, J = 53.5 Hz, 1H), 3.52 (ddd, J = 39.5, 13.3, 3.1 Hz, 1H), 3.32 (dt, J = 18.3, 10.0 Hz, 2H), 3.23 (t, J = 9.5

### Hz, 1H), 2.17 - 1.94 (m, 2H).

Compared with the ¹H NMR of the crystal form I of compound A, the ¹H NMR results of the crystal form I of compound A hydrobromide shows that this batch of samples has chemical shifts.

### DSC and TGA thermal analysis

The collected the crystal form I of compound A hydrobromide from the HBr-S1 batch in Example 6.8 was subjected to DSC analysis by General method 4. In the DSC, the sample of the crystal form I of compound A hydrobromide showed a broad melting endothermic peak at 250.54 °C with an onset temperature of 240.88 °C.

The collected crystal form I of compound A hydrobromide from the HBr-S1 batch in Example 6.8 was subjected to TGA analysis by General method 5. In the TGA, the crystal form I of compound A hydrobromide had a weight loss of 0.6011% prior to 200 °C.

DSC and TGA analysis indicated that the crystal form I of compound A hydrobromide was an anhydrous crystal form.

### DVS analysis

FIG. 45 shows the DVS curve of the crystal form I of compound A hydrobromide from the HBr-S1 batch in Example 6.8 acquired by General method 6. In the DVS, the sample of the crystal form I of compound A hydrobromide was slightly hygroscopic, and the water absorption was about 0.67% under the condition of 0-80%RH.

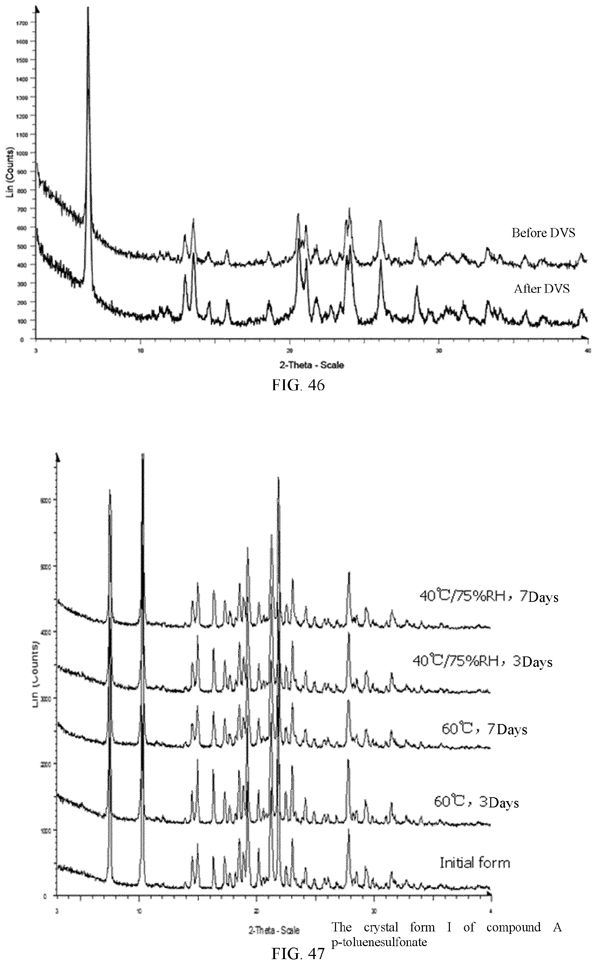

The crystal form I of compound A hydrobromide was not changed after the sample was tested by DVS, as shown in FIG.46.

### Example 8 Stability study of the crystal form I of compound A and the crystal form I of compound A p-toluenesulfonate

A certain amount of samples of the crystal form I of compound A and the crystal form I of compound A p-toluenesulfonate (Batch No.: PTSA-A1) were placed in a stability test chamber, and kept at 40 °C/75%RH and 60 °C for 7 days, respectively. The samples were tested by HPLC and XRPD at various time points. The specific results are listed in Table 8.

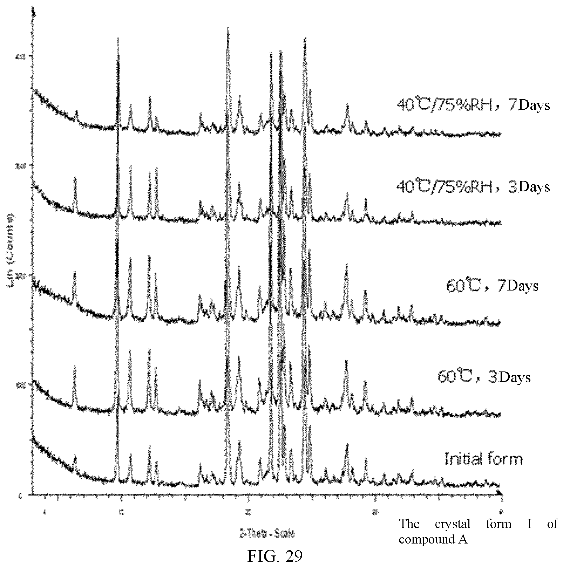

XRPD results showed that the crystal form I of compound A and the crystal form I of compound A p-toluenesulfonate remained unchanged for 7 days at 40 °C/75% RH and 60 °C, as shown in FIG. 29 and FIG. 47.

**Table 8: Stability study of the crystal form I of compound A and the crystal form I of compound A p-toluenesulfonate**

| Sample | Condition | Day 0 | Day 3 | | Day 7 | |
|---|---|---|---|---|---|---|
| | | Purity | Purity | Crystal form | Purity | Crystal form |
| The crystal form I of compound A p-toluenesulfonate (Batch No.: PTSA-A1) | 60 °C | 97.9% | 97.9% | Initial crystal form | 97.9% | Initial crystal form |
| | 40 °C/75% RH | 97.9% | 97.9% | Initial crystal form | 97.9% | Initial crystal form |
| The crystal form I of compound A | 60 °C | 96.1% | 96.7% | Initial crystal form | 97.1% | Initial crystal form |
| | 40 °C/75% RH | 96.1% | 97.1% | Initial crystal form | 97.1% | Initial crystal form |

### Example 9 Solubility study of the crystal form I of compound A and the crystal form I of compound A p-toluenesulfonate in different vehicles

The solubilities of the crystal form I of compound A and the crystal form I of compound A p-toluenesulfonate in SGF (simulated gastric fluid), FaSSIF (fasted state simulated intestinal fluid) and FeSSIF (fed state simulated intestinal fluid) were studied.

The solubilities of samples in SGF, FaSSIF and FeSSIF at 37 °C for 24 hours were determined. 40 mg of the sample was placed in a sample bottle, and 1.5 mL of solvent was added. The mixture was mixed well, and shaken on a shaker at 200 rpm for 24 hours. The suspension was filtered at 0.5, 2 and 24 hours. The filtrate was analyzed by HPLC, and the solid was tested by XRPD. The specific results are listed in Table 9.

**Table 9 Solubility results of the crystal form I of compound A and the crystal form I of compound A p-toluenesulfonate in different vehicles**

| Sample | Vehi cle | 0.5h | | | 2h | | | 24h | | |
|---|---|---|---|---|---|---|---|---|---|---|
| | | pH | Solubility (mg/m L) | Crystal form | pH | Solubility (mg/m L) | Crystal form | pH | Solubility (mg/m L) | Crystal form |
| The crystal form I of compound A p-toluenesulf onate | SGF | 1.2 3 | 0.097 | Initial crysta l form | 1.1 9 | 0.067 | Initial crysta l form | 1.1 8 | 0.045 | Chang ed |
| | FaSS IF | 6.3 8 | 0.0045 | Chang ed | 3.4 8 | 0.0037 | Chang ed | 1.9 1 | 0.016 | Chang ed |
| | FeSS IF | 4.5 3 | 0.10 | Chang ed | 4.5 1 | 0.093 | Chang ed | 4.5 0 | 0.18 | Chang ed |
| The crystal form I of compound A | SGF | 1.2 4 | 0.12 | Initial crysta l form | 1.2 4 | 0.063 | Chang ed | 1.2 3 | 0.046 | Chang ed |
| | FaSS IF | 6.6 2 | 0.0042 | Initial crysta l form | 6.6 3 | 0.0048 | Chang ed | 6.6 3 | 0.0043 | Chang ed |
| | FeSS IF | 5.0 4 | 0.22 | Initial crysta l form | 5.0 5 | 0.18 | Chang ed | 5.0 2 | 0.057 | Chang ed |

The results showed that the solubility of the free base and the p-toluenesulfonate was affected by pH value. The p-toluenesulfonate showed higher solubility than that of the free base in FaSSIF and FeSSIF vehicles for 24 hours. The free base showed higher solubility than that of the p-toluenesulfonate in FeSSIF vehicle for 0.5 and 2 hours. Under other conditions, the solubility of the two was equivalent.

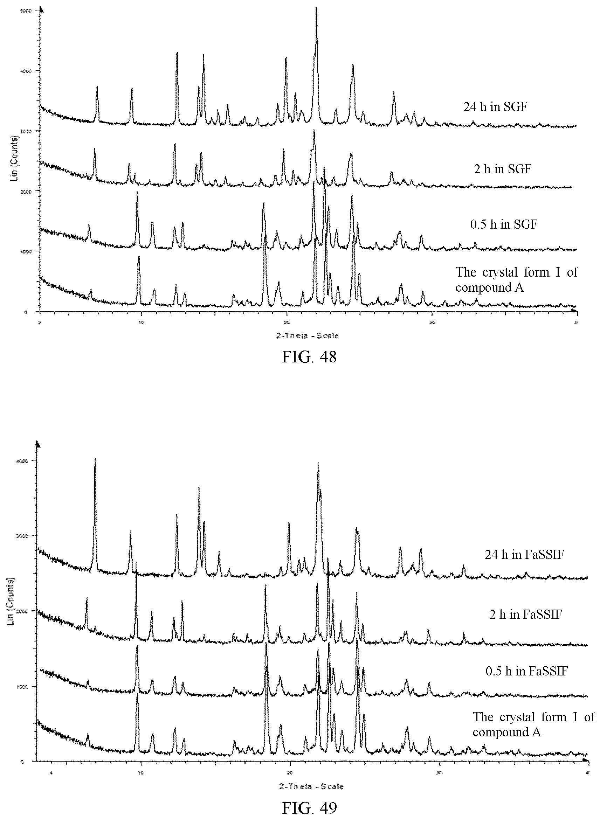

The crystal form of the crystal form I of compound A was changed when the crystal form I of compound A was dissolved in SGF, FaSSIF and FeSSIF for 2 hours, as shown in FIGs 48, 49 and 50.

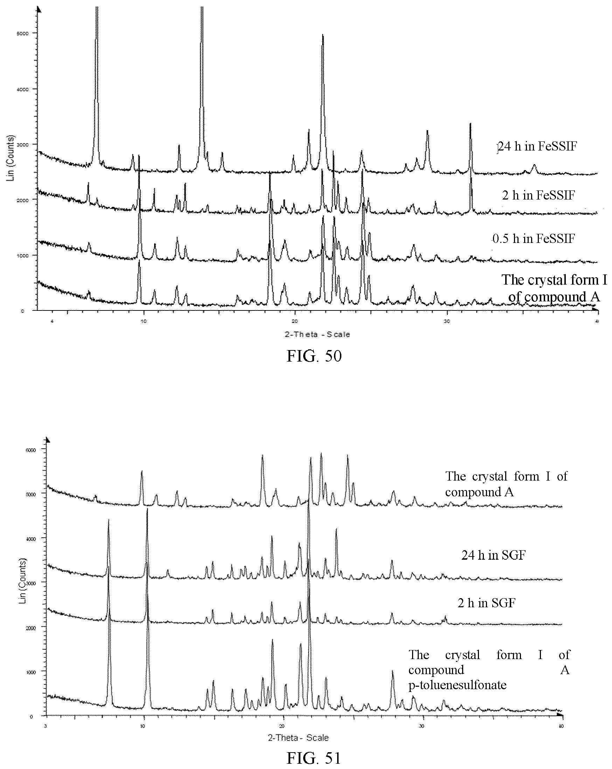

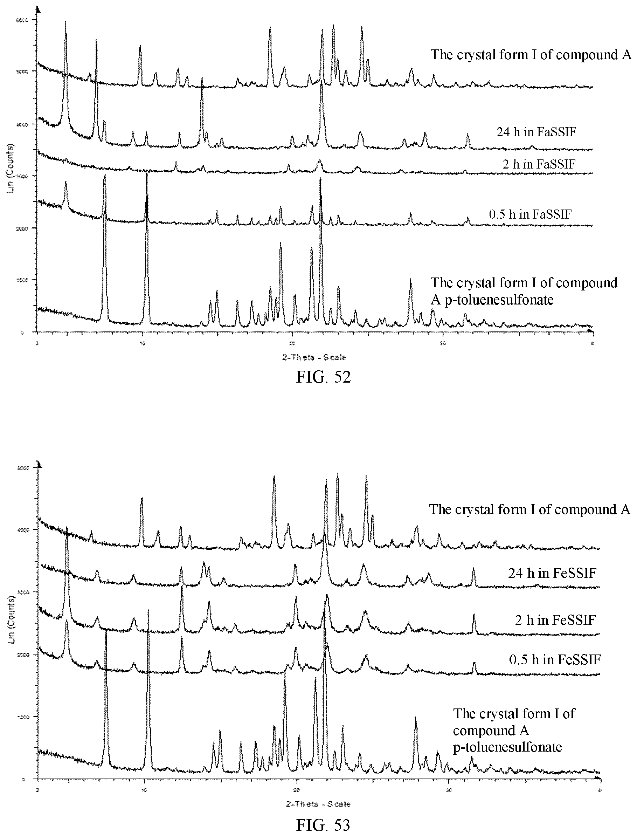

The crystal form of the crystal form I of compound A p-toluenesulfonate was changed when the crystal form I of compound A p-toluenesulfonate was dissolved in SGF for 24 hours and in FaSSIF and FeSSIF for 0.5 hours, as shown in FIGs 51, 52 and 53.

P-toluenesulfonate had a higher solubility after 2 hours in a solution with a higher pH value, which was due to the dissociation of p-toluenesulfonate with the increase of pH value.

### Example 10 Single crystal X-ray structure and absolute stereochemistry of compound A

Single crystal of compound A was grown by slow volatilizatin at room temperature. Method 1: About 100 mg of the crystal form VI of compound A was dissolved in acetone or methanol, respectively, and the anti-solvent n-heptane was then added until the solution was slightly cloudy. 1-2 drops of solvent was added or the temperature was increased, until the solution became clear again. The above solution was allowed to volatilize slowly at room temperature. Method 2: About 100 mg of the crystal form VI of compound A was dissolved in methanol to prepare a saturated solution, and the above solution was allowed to volatilize slowly at room temperature.

The collection and analysis of single crystal structure data were carried out by the Crystallographic Laboratory of Peking University. Single-crystal diffraction data of the samples were acquired using a Rigaku XtaLAB PRO 007HF(Mo) diffractometer at 180 K using the Mo target Kα spectral line (λ = 0.71073 Å). Data modification and absorption correction were performed using the CrysAlisPro program, and the structure was resolved by a dual-space algorithm using the SHELXT program. Non-hydrogen atoms may be located in different Fourier spectra, and hydrogen atoms were geometrically filled into their parent atoms. The refinement of the final structure was done based on the *F*² full-matrix least squares method using the SHELXL program.

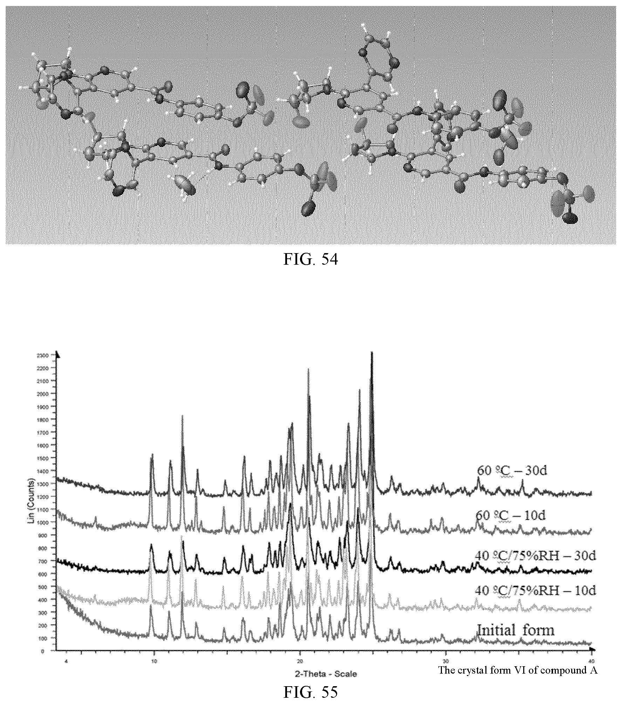

The single crystal of compound A was obtained by slowly volatilizing in methanol solution at room temperature, and the single crystal structure was analyzed. The single crystal was a lump crystal, with a structural formula of C₂₁H₁₇ClF₃N₅O₂▪0.5CH₃OH, which was a solvate of methanol. The single crystal belonged to the monoclinic system and the C2 space group. The structure of the single crystal was shown in FIG. 54. Each unit includeed two molecules of compound A and one molecule of methanol, and the main molecule had the same molecular structure as that of compound A. Crystal structure parameters obtained by resolution are shown in Table 10.

**Table 10 Crystal structure parameters of compound A**

| | |
|---|---|
| Molecular formula | C₄₃H₃₈Cl₂F₆N₁₀O₅ |
| Molecular weight | 959.73 |
| Temperature/K | 179.99(19) |
| Crystal system | Monoclinic |
| Space group | *C*2 |
| *a*/Å | 31.9592(11) |
| *b*/Å | 9.58750(10) |
| *c*/Å | 31.2990(8) |
| *α*/Å | 90 |
| *β*/° | 116.850(3) |
| *γ*/° | 90 |
| Volume/A³ | 8556.4(4) |
| *Z* | 8 |
| *Density* (*calculated value*) g/cm³ | 1.490 |
| *Absorption coefficient*/mm⁻¹ | 0.238 |
| *F*(000) | 3952.0 |
| Crystal size/mm³ | 0.42 × 0.2 × 0.12 |
| Radiation source | Mo Kα (λ = 0.71073) |
| 2*θ* range for data collection /° | 4.002 to 54.966 |
| Index ranges | -41 ≤ *h* ≤ 41, -12 ≤ *k* ≤ 12, -40 ≤ *l* ≤ 40 |
| Reflections collected | 101906 |
| Independent reflections | 19637 [*R*ᵢₙₜ = 0.0455, *R*_{sigma} = 0.0346] |
| Data / restraints / parameters | 19637/1/1193 |
| Goodness-of-fit on *F*² | 1.071 |
| Final R indices [*I*>=2*σ* (*I*)] | *R*₁ = 0.0670, *wR*₂ = 0.1586 |
| Final R indices [all data] | *R*₁ = 0.0801, *wR*₂ = 0.1660 |
| Largest diff. peak/ hole/ e Å⁻³ | 0.99/-0.58 |
| Absolute structure parameter | 0.04(2) |

### Example 11 Stability study of the crystal form VI of compound A

Samples of the crystal form VI of compound A were placed in open glass bottles under test conditions of high temperature (60°C), high humidity (25°C/92.5%RH), high temperature and high humidity (40°C/75%RH), and light (visible light 4500 Lux±500 Lux, near-ultraviolet light 85µw/cm²). The samples were taken and tested on day 0, day 10, and day 30 to study the physical stability of the API of the crystal form VI of compound A. The test items included appearance, crystal form, hygroscopic weight gain (only measured under the condition of high humidity), content and related substances. The specific results are listed in Table 11.

**Table 11 Stability results of the crystal form VI of compound A**

| Condition | | Appearance | Hygroscopic weight gain | Content | Impurity content | Crystal form |
|---|---|---|---|---|---|---|
| | 0 day | Off-white powder | N/A | 99.40% | 0.87% | The crystal form VI |
| 60°C | 10 days | Off-white powder | N/A | 99.90% | 0.81% | The crystal form VI |
| | 30 days | Off-white powder | N/A | 96.90% | 0.84% | The crystal form VI |
| 25°C/92.5%RH | 10 days | Off-white powder | 0.34% | 98.40% | 0.80% | The crystal form VI |
| | 30 days | Off-white powder | 2.72% | 98.80% | 0.85% | The crystal form VI |
| 40°C/75%RH | 10 days | Off-white powder | 1.26% | 98.40% | 0.79% | The crystal form VI |
| | 30 days | Off-white powder | 1.26% | 101.40% | 0.80% | The crystal form VI |
| Light | 10 days | Off-white powder | N/A | 99.70% | 0.85% | The crystal form VI |

The test results of appearance, crystal form and related substances showed that the crystal form VI of compound A was stable for 30 days under the conditions of high temperature (60°C), high humidity (25°C/92.5%RH), high temperature and high humidity (40°C/75%RH), and light (visible light 4500 Lux±500 Lux, near-ultraviolet light 85µw/cm²) and stable for 10 days under the condition of light. Moreover, the crystal form was not changed, as shown in FIG. 55.

### Example 12 Dissolution study of compound A

In order to improve the dissolution of formulations containing compound A, the inventors investigated the effect of adding solubilizers and glidants on the dissolution of formulations of compound A. It was found that with the addition of sodium lauryl sulfate (a solubilizer), the dissolution of compound A in the formulation was decreased as the amount of sodium lauryl sulfate was increased. However, it was found that when colloidal silica 200 (a glidant) was added, the dissolution of compound A could be improved.

**Table 12-1 Tablet composition used in the glidant study**

| Batch No. | A | | B | | C | |
|---|---|---|---|---|---|---|
| Ingredient | Amount in a single tablet (mg) | % | Amount in a single tablet (mg) | % | Amount in a single tablet (mg) | % |
| Compound A | 40 | 10 | 40 | 10 | 40 | 10 |
| Lactose monohydrate FLOWLAC^{®} 100 | 116 | 29 | 112 | 28 | 112 | 28 |
| Microcrystalline cellulose 102 | 228 | 57 | 228 | 57 | 220 | 55 |
| Croscarmellose sodium | 12 | 3 | 12 | 3 | 12 | 3 |
| **Colloidal silica 200** | **N/A** | **N/A** | **4** | **1** | **12** | **3** |
| Magnesium stearate | 4 | 1 | 4 | 1 | 4 | 1 |
| Total | 400 | 100 | 400 | 100 | 400 | 100 |

**Table 12-2 Method for dissolution**

| | |
|---|---|
| Dissolution apparatus | Chinese Pharmacopoeia 2015 Edition, 0931, the second method (paddle method) |
| Dissolution medium | 0.1N HCl + 0.8%SDS |
| Medium volume | 900 mL |
| Medium temperature | 37 ± 0.5 °C |
| Rotation speed of stirring paddle | 75 rpm (the ultimate speed test at 250 rpm for 60-75 min) |
| Sampling time point | 5 min, 15 min, 30 min, 45 min, 60 min, 75 min |

**Table 12-3 Dissolution results for tablets used in the glidant study**

| **Batch No.** | **Dissolution results (%) (n=3)** | | | | | |
|---|---|---|---|---|---|---|
| | **5 min** | **15 min** | **30 min** | **45 min** | **60 min** | **75 min** |
| A | 41 | 53 | 60 | 65 | 68 | 80 |
| RSD (%) | 2.51 | 1.89 | 1.98 | 1.99 | 1.91 | 1.42 |
| B | 52 | 72 | 83 | 88 | 92 | 97 |
| RSD (%) | 3.56 | 2.66 | 3.68 | 4.35 | 4.50 | 3.43 |
| C | 51 | 73 | 88 | 94 | 98 | 100 |
| RSD (%) | 1.52 | 2.47 | 2.98 | 2.84 | 2.43 | 2.27 |

From the above results, it can be seen that the dissolution result is increased with the increase of the amount of colloidal silica, and the product is dissolved completely when the amount of colloidal silica reaches 3%. Therefore, 3% of colloidal silica 200 was chosen as the amount of glidant for further study.

### Example 13 Representative tablet formulation of the crystal form VI of compound A

Immediate release film-coated tablets in 10 mg and 40 mg doses were prepared using a direct powder compression process. The composition of the tablets is provided in Table 13.

**Table 13-1 Composition of a unit dose product of a tablet**

| Ingredient of formulations | Specification of 10 mg | | Specification of 40 mg | | Function |
|---|---|---|---|---|---|
| | mg/tablet | weight % | mg/tablet | weight % | |
| Plain tablet | | | | | |
| The crystal form VI of compound A | 10 | 10 | 40 | 10 | API |
| Lactose monohydrate | 28 | 28 | 112 | 28 | Diluent |
| FLOWLAC^{®} 100 | | | | | |
| Microcrystalline cellulose 102 | 55 | 55 | 220 | 55 | Diluent |
| Croscarmellose sodium | 3 | 3 | 12 | 3 | Disintegrant |
| Colloidal silica 200 | 3 | 3 | 12 | 3 | Glidant |
| Magnesium stearate | 1 | 1 | 4 | 1 | Lubricant |
| Total of plain tablet | 100 | 100 | 400 | 100 | N/A |

| Coating material | | | | | |
|---|---|---|---|---|---|
| Opadry II 85F620077 | 3 | N/A | 12 | N/A | Coating materials |
| Total of coated tablet | 103 | N/A | 412 | N/A | N/A |

The tablets with two specifications (10 mg and 40 mg) were formulated in equal proportions, which were prepared using the same batch of mixed materials, and then compressed into tablets with different specifications. A total mixed batch for the preparation of tablets with specifications of 10 mg and 40 mg (14,000 tablets for each) was used as a representive, and the formulation information of the batch was shown in Table 13-2 below.

**Table 13-2 Formulation information of the GMP batch with 10mg/40mg specifications**

| Tablet core | | | | | | |
|---|---|---|---|---|---|---|
| Ingredient | Amount (g) | % | | | | |
| The crystal form VI of compound A | 700.00 | 10 | | | | |
| Lactose monohydrate FLOWLAC^{®} 100 | 1960.00 | 28 | | | | |
| Microcrystalline cellulose 102 | 3850.00 | 55 | | | | |
| Croscarmellose sodium | 210.00 | 3 | | | | |
| Colloidal silica 200 | 210.00 | 3 | | | | |
| Magnesium stearate | 70.00 | 1 | | | | |
| Total of tablet core | 7000.00 | 100 | | | | |

| Coating | | | | | | |
|---|---|---|---|---|---|---|
| Ingredient | Specification 10 | Specification 40 mg/tablet | | | | |

| | | mg/tablet | | | | |
|---|---|---|---|---|---|---|
| | | Amount (g) | % | Amount (g) | | % |
| Opadry II 85F620077 | | 42.00 | 12% | 168.00 | | 12% |
| Purified water | | 308.00 | 88% | 1232.00 | | 88% |

| Package | | | | | | |
|---|---|---|---|---|---|---|
| Product specification | Packaging bottle specification | Sealing | | Packaging specification | Packaging material | |
| 10 mg/tablet | 45 mL | Child safety polypropylene cover with aluminum foil closure | | 30 tablet/ bottle | High density polyethylene bottle for oral solid drug | |
| 40 mg/tablet | 75 mL | Child safety polypropylene cover with aluminum foil closure | | 30 tablet/ bottle | High density polyethylene bottle for oral solid drug | |

The method of preparing the tablet was as follows:
1. Adjuvants and APIs were weighed according to the formulation amount.
2. Sieving: the crystal form VI of compound A was passed through a 80-mesh sieve; lactose monohydrate was passed through a 60-mesh sieve; the microcrystalline cellulose and colloidal silica were placed in the same medicinal LDPE bag and passed through a 30-mesh sieve, which was denoted as Mixture 1; croscarmellose sodium and magnesium stearate were passed through a 30-mesh sieve, respectively.
3. Main mixing: Mixture 1, lactose monohydrate, the crystal form VI of compound A, and croscarmellose sodium were added sequentially into a hopper, and mixed with a mixing speed of 15 rpm and a mixing period of 15 minutes.
4. Total mixing: The magnesium stearate was added into the hopper mixer for total mixing, with a mixing speed of 15 rpm and a mixing period of 5 minutes.
5. Tableting: The punch for the 10 mg tablet was a 6 mm dimple round punch, and the punch for the 40 mg tablet was a 10.0 mm dimple round punch. After the equipment had a trial run, the formal production was carried out. Weight, hardness and friability of tablets were monitored online to make the tablets meet the tabletting standards as shown in Table 13-3:

**Table 13-3 Tabletting standards for the preparation process**

| Specification | Item | Standard |
|---|---|---|
| 10 mg | Target weight for single tablet | 100.0 mg |
| | Target weight range for single tablet | 100.0 ± 5 mg |
| | Hardness range for single tablet | 30 N - 90 N |
| | Friability | ≤0.5% |
| 40 mg | Target weight for single tablet | 400.0 mg |
| | Target weight range for single tablet | 400.0 ± 20 mg |
| | Hardness range for single tablet | 80 N - 160 N |
| | Friability | ≤0.5% |

6. Coating: 12% Opadry coating solution was freshly prepared with purified water. The air inlet temperature was set at 50-60°C and the coating pan was preheated at a rotation speed of 2 rpm. When the coating pan was preheated so that the air exhaust temperature reached 42°C, coating was carried out by spraying, while the air inlet temperature was set at 50~70°C, the rotation speed of the pot was set at 5~12 rpm, and the air inlet volume was set at 300±100 m³/h. Tablet in 10 mg sepecification: Pump flow: 8 ml/min ~30 ml/min, atomization pressure: 0.5±0.3bar, and pressure for controlling atomization angle: 1 ± 0.5 bar; Tablet in 40 mg sepecification: pump flow: 8 ml/min ~ 40 ml/min, atomization pressure: 1.5 ± 1bar, and pressure for controlling atomization angle: 1.5 ± 1 bar. Coating parameters and coating weight gain were monitored. When the coating weight gain reached the target range of 2.5-3.5%, the spraying was stopped, the heating was stopped, and the coating pan was adjusted to a rotation speed of 5 rpm and an air inlet volume of 200-500 m³/h. The product was discharged after drying for 5 minutes.
7. Packaging: The packaging materials of 10 mg tablets and 40 mg tablets were 45 mL and 75 mL high-density polyethylene bottles for oral solid drugs, respectively, and each bottle contained 30 tablets.
8. Labeling: Bottle labels were affixed to product bottles, one label per bottle.

### Example 14 Chemical stability study (I) of representative tablet formulations

For the 10 mg and 40 mg tablets prepared in Example 13, chemical stability data was collected on day 10 after 10 days of light irradation, and stability data at 40 °C/75% RH in open and closed containers were collected on day 10 and day 30. Dissolution and impurities were tested to assess the chemical stability of the test formulations. The specific results are listed in Table 14-1 and Table 14-2.

**Table 14-1 Tablet stability and dissolution results (%) of the crystal form VI of compound A**

| Speci ficati on | Storage condition | Dissolution method: Chinese Pharmacopoeia 2015 Edition, 0931, the second method (paddle method) | | | | | | |
|---|---|---|---|---|---|---|---|---|
| | | Dissolution medium: 0.1N HCl+0.8% SDS | | | | | | |
| | | Medium volume: 900 ml | | | | | | |
| | | Medium temperature: 37 ± 0.5 °C | | | | | | |
| | | Rotation speed: 75 rpm (the ultimate speed test at 250 rpm for 60-75 min) | | | | | | |
| | | Dissolution Results | 5 min | 15 min | 30 min | 45 min | 60 min | 75 min |
| 10 mg | 0 day | Dissolution (%) | 53 | 82 | 95 | 98 | 100 | 100 |
| | | RSD (%) | 4.10 | 3.43 | 2.90 | 3.03 | 2.83 | 3.02 |
| | Light - 10 days (1 bottle) | Dissolution (%) | 58 | 86 | 95 | 99 | 100 | 101 |
| | | RSD (%) | 3.72 | 0.70 | 1.28 | 2.10 | 2.16 | 2.42 |
| | 40°C/75%R H (open) - 10 days (1 bottle) | Dissolution (%) | 51 | 80 | 90 | 95 | 98 | 99 |
| | | RSD (%) | 1.27 | 0.60 | 1.12 | 1.81 | 1.84 | 2.11 |
| | 40°C/75%R H (open) - 30 days (1 bottle) | Dissolution (%) | 52 | 76 | 90 | 94 | 97 | 99 |
| | | RSD (%) | 3.08 | 3.44 | 3.06 | 2.92 | 2.68 | 3.09 |
| | 40°C/75%R H (closed) - 30 days (1 bottle) | Dissolution (%) | 56 | 79 | 91 | 95 | 97 | 98 |
| | | RSD (%) | 4.83 | 5.77 | 4.35 | 4.18 | 3.93 | 4.18 |
| 40 | 0 day | Dissolution | 53 | 78 | 92 | 98 | 100 | 101 |
| mg | | (%) | | | | | | |
| | | RSD (%) | 4.01 | 2.39 | 2.05 | 1.73 | 1.51 | 1.45 |
| | Light - 10 days (1 bottle) | Dissolution (%) | 57 | 79 | 94 | 98 | 100 | 99 |
| | | RSD (%) | 2.58 | 2.08 | 0.81 | 0.83 | 1.08 | 1.10 |
| | 40°C/75%R H (open) - 10 days (1 bottle) | Dissolution (%) | 54 | 73 | 87 | 94 | 97 | 98 |
| | | RSD (%) | 6.91 | 4.57 | 4.22 | 3.09 | 2.83 | 2.83 |
| | 40°C/75%R H (open) - 30 days (1 bottle) | Dissolution (%) | 47 | 71 | 87 | 94 | 98 | 100 |
| | | RSD (%) | 2.36 | 1.61 | 1.37 | 1.06 | 0.71 | 0.54 |
| | 40°C/75%R H (closed) - 30 days (1 bottle) | Dissolution (%) | 50 | 77 | 92 | 97 | 99 | 100 |
| | | RSD (%) | 5.60 | 3.79 | 3.47 | 2.24 | 1.25 | 1.03 |

It can be seen from the data in the above Table that the dissolution of the tablets had no change under the light condition for 10 days; had no significant change under the accelerated 40 °C/75% RH (open) condition for 10 days and 30 days; and had no significant change under the accelerated 40 °C/75% RH (closed) condition for 10 days and 30 days.

**Table 14-2 Results (%) of tablet stability related substances of the crystal form VI of compound A**

| Specification | Storage condition | Total impurities (%) |
|---|---|---|
| 10 mg | 0 day | 0.78 |
| | Light - 10 days (1 bottle) | 0.70 |
| | 40°C/75%RH (open) - 10 days (1 bottle) | 0.80 |
| | 40°C/75%RH (open) - 30 days (1 bottle) | 0.80 |
| | 40°C/75%RH (closed) - 30 days (1 bottle) | 0.73 |
| | 40°C/75%RH (closed) - 30 days (1 bottle) | 0.75 |
| 40 mg | 0 day | 0.80 |
| | Light - 10 days (1 bottle) | 0.79 |
| | 40°C/75%RH (open) - 10 days (1 bottle) | 0.80 |
| | 40°C/75%RH (open) - 30 days (1 bottle) | 0.80 |
| | 40°C/75%RH (closed) - 30 days (1 bottle) | 0.78 |
| | 40°C/75%RH (closed) - 30 days (1 bottle) | 0.77 |

It can be seen from the data in the above Table that the tablets with specifications of 10 mg and 40 mg have no increase in impurities under the light condition for 10 days and under accelerated conditions of 40°C/75%RH (open and closed) for 10 days and 30 days.

### Example 15 Chemical stability study (II) of representative tablet formulations

For the 10 mg and 40 mg tablets prepared in Example 12, the stability influencing factors, accelerated stability test and long-term stability test were carried out respectively according to the storage conditions specified in the ICH Q1 guideline, and the corresponding results are listed in Tables 15-1 , 15-2, 15-3 and 15-4.

**Table 15-1 Test data of influencing factors of the stability of 10 mg tablets**

| Factor | | Content | Total impurity content | Chiral purity | Dissolution (average) |
|---|---|---|---|---|---|
| High temperature (60°C) | 0 day | 100.7% | 0.25% | 99.5% | 99% |
| | 5 days | 100.2% | 0.25% | 99.5% | 96% |
| | 10 days | 100.2% | 0.25% | 99.5% | 95% |
| | 30 days | 99.4% | 0.25% | 99.5% | 94% |
| High humidity (92.5%, 25°C) | 0 day | 100.7% | 0.25% | 99.5% | 99% |
| | 5 days | 99.4% | 0.25% | 99.5% | 98% |
| | 10 days | 100.0% | 0.26% | 99.5% | 97% |
| | 30 days | 98.4% | 0.25% | 99.5% | 87% |
| Light (5000 | 0 day | 100.7% | 0.25% | 99.5% | 99% |
| | 5 days | 98.7% | 0.24% | 99.5% | 100% |
| lux+80 uW/cm²) | 10 days | 99.2% | 0.25% | 99.5% | 100% |

**Table 15-2 Test data of influencing factors of the stability of 40 mg tablets**

| Factor | | Content | Total impurity content | Chiral purity | Dissolution (average) |
|---|---|---|---|---|---|
| High temperature (60°C) | 0 day | 100.3% | 0.32% | 99.5% | 100% |
| | 5 days | 98.7% | 0.25% | 99.5% | 95% |
| | 10 days | 100.3% | 0.25% | 99.5% | 92% |
| | 30 days | 99.2% | 0.25% | 99.6% | 90% |
| High humidity (92.5%, 25°C) | 0 day | 100.3% | 0.32% | 99.5% | 100% |
| | 5 days | 99.6% | 0.25% | 99.5% | 95% |
| | 10 days | 99.7% | 0.25% | 99.5% | 93% |
| | 30 days | 98.7% | 0.25% | 99.5% | 89% |
| Light (5000 lux+80 uW/cm²) | 0 day | 100.3% | 0.32% | 99.5% | 100% |
| | 5 days | 100.1% | 0.25% | 99.5% | 100% |
| | 10 days | 100.5% | 0.26% | 99.5% | 98% |

**Table 15-3 Accelerated stability test data of tablets with different specifications**

| Specification | Factor | | Content | Total impurity content | Dissolution (average) |
|---|---|---|---|---|---|
| 10 mg | 40 ± 2°C / 75% ± 5%RH | 0 day | 100.7% | 0.25% | 99% |
| | | 1 month | 100.1% | 0.25% | 99% |
| | | 3 months | 100.1% | 0.24% | 100% |
| 40 mg | 40 ± 2°C / 75% ± 5%RH | 0 day | 100.3% | 0.38% | 102% |
| | | 1 month | 100.7% | 0.29% | 99% |
| | | 3 months | 100.3% | 0.27% | 99% |

**Table 15-4 Long-term stability test data of tablets with different specifications**

| Specification | Factor | | Content | Total impurity content | Dissolution (average) |
|---|---|---|---|---|---|
| 10 mg | 25 ± 2°C / 60% ± 5%RH | 0 day | 100.2% | 0.39% | 100% |
| | | 3 months | 99.7% | 0.33% | 101% |
| 40 mg | 25 ± 2°C / 60% ± 5%RH | 0 day | 100.3% | 0.32% | 100% |
| | | 3 months | 100.2% | 0.23% | 95% |

The results showed that there was no significant difference in the content, total impurity content, chiral purity and dissolution of the 10 mg and 40 mg tablets under high temperature, high humidity and light conditions, as shown in Tables 15-1 and 15-2.

The content, total impurity content, dissolution and moisture of the 10 mg and 40 mg tablets were not changed significantly under the accelerated conditions within 3 months, as shown in Table 15-3.

The content, total impurity content, dissolution and moisture of the 10 mg and 40 mg tablets were not changed significantly under the long-term conditions within 3 months, as shown in Table 15-4.

## Claims

1. The crystal form VI of a compound of formula (A): 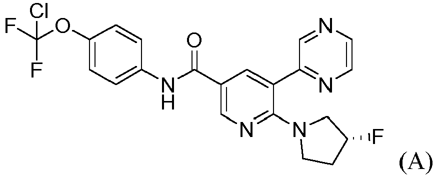 which is **characterized in that**: the X-ray powder diffraction pattern thereof obtained using CuK_{α} radiation includes at least the characteristic peaks located at the following °2θ: 11.9±0.2, 20.5±0.2, 23.1±0.2, 23.9±0.2 and 24.8±0.2.

2. The crystal form VI of the compound of formula (A) of claim 1, which is **characterized in that**: the X-ray powder diffraction pattern thereof obtained using CuK_{α} radiation further includes the characteristic peaks located at the following °2θ: 9.7±0.2, 16.1±0.2, 19.3±0.2 and 21.2±0.2.

3. The crystal form VI of the compound of formula (A) of claim 2, which is **characterized in that**: the X-ray powder diffraction pattern thereof obtained using CuK_{α} radiation has the following characteristic peaks:
| Angle °2θ±0.2 °2θ | Relative intensity % |
|---|---|
| 9.7 | 12.3 |
| 11.9 | 48.7 |
| 16.1 | 14 |
| 19.3 | 20 |
| 20.5 | 100 |
| 21.2 | 20.3 |
| 23.1 | 29.3 |
| 23.9 | 26.3 |
| 24.8 | 36.8 |

4. The crystal form VI of the compound of formula (A) of claim 2 or 3, which is **characterized in that**: the X-ray powder diffraction pattern thereof obtained using CuK_{α} radiation further includes the characteristic peaks located at the following °2θ: 5.9±0.2, 11.0±0.2, 12.8±0.2, 14.7±0.2, 16.6±0.2, 17.8±0.2, 18.2±0.2, 18.6±0.2, 20.1±0.2, 22.0±0.2, 22.6±0.2, 26.2±0.2 and 29.0±0.2.

5. The crystal form VI of the compound of formula (A) of claim 1, which is **characterized by**: having an X-ray powder diffraction pattern substantially as shown in FIG. 8.

6. The crystal form VI of the compound of formula (A) of any one of claims 1-5, which is also **characterized by**: having a melting endothermic peak at 175±2°C in differential scanning calorimetry analysis.

7. The crystal form VI of the compound of formula (A) of any one of claims 1-6, which is also **characterized by**: having substantially no weight loss prior to 200°C in thermogravimetric analysis.

8. The crystal form VI of the compound of formula (A) of any one of claims 1-7, which is also **characterized by**: having absorption peaks in the infrared absorption spectrum at the following cm⁻¹: 853±2, 1020±2, 1062±2, 1210±2, 1408±2, 1466±2, 1491±2, 1599±2, 1661±2, 3293±2.

9. The crystal form VI of the compound of formula (A) of claim 8, which is also **characterized by**: having an infrared absorption spectrum substantially as shown in FIG. 23.

10. The crystal form VI of the compound of formula (A) of any one of claims 1-9, which is also **characterized by**: having absorption peaks in the UV spectrum at the following nm: 201±2, 263±2 and 306±2.

11. The crystal form VI of the compound of formula (A) of claim 10, which is also **characterized by**: having a UV spectrum substantially as shown in FIG. 19.

12. The crystal form I of the compound of formula (A), which is **characterized in that**: the X-ray powder diffraction pattern thereof obtained using CuK_{α} radiation includes at least the characteristic peaks located at the following °2θ: 18.3±0.2 and 24.4±0.2.

13. The crystal form I of the compound of formula (A) of claim 12, which is **characterized in that**: the X-ray powder diffraction pattern thereof obtained using CuK_{α} radiation further includes the characteristic peaks located at the following °2θ: 9.6±0.2, 19.3±0.2, 21.8±0.2, 22.5±0.2 and 24.9±0.2.

14. The crystal form I of the compound of formula (A) of claim 13, which is **characterized in that**: the X-ray powder diffraction pattern thereof obtained using CuK_{α} radiation has the following characteristic peaks:
| Angle °2θ±0.2 °2θ | Relative intensity % |
|---|---|
| 9.6 | 48.1 |
| 18.3 | 59.6 |
| 19.3 | 25.8 |
| 21.8 | 31.2 |
| 22.5 | 31.3 |
| 24.4 | 100 |
| 24.9 | 36.3 |

15. The crystal form I of the compound of formula (A) of claim 13 or 14, which is **characterized in that**: the X-ray powder diffraction pattern thereof obtained using CuK_{α} radiation further includes the characteristic peaks located at the following °2θ: 10.7±0.2, 12.2±0.2, 16.2±0.2, 22.8±0.2, 23.4±0.2 and 27.8±0.2.

16. The crystal form I of the compound of formula (A) of claim 12, which is **characterized by**: having an X-ray powder diffraction pattern substantially as shown in FIG. 1.

17. The crystal form I of the compound of formula (A) of any one of claims 12-16, which is also **characterized by**: having a melting endothermic peak at 173±2°C in differential scanning calorimetry analysis.

18. The crystal form I of the compound of formula (A) of any one of claims 12-17, which is also **characterized by**: having a weight loss of about 5.1% prior to 200°C in thermogravimetric analysis.

19. The crystal form II of the compound of formula (A), which is **characterized in that**: the X-ray powder diffraction pattern thereof obtained using CuK_{α} radiation includes at least the characteristic peaks located at the following °2θ: 11.0±0.2, 11.6±0.2, 13.0±0.2, 17.1±0.2, 19.6±0.2, 19.8±0.2 and 22.9±0.2.

20. The crystal form II of the compound of formula (A) of claim 19, which is **characterized in that**: the X-ray powder diffraction pattern thereof obtained using CuK_{α} radiation has the following characteristic peaks:
| Angle °2θ±0.2 °2θ | Relative intensity % |
|---|---|
| 11.0 | 90.1 |
| 11.6 | 60.1 |
| 13.0 | 52.5 |
| 17.1 | 100 |
| 19.6 | 54.4 |
| 19.8 | 50.6 |
| 22.9 | 53.7 |

21. The crystal form II of the compound of formula (A) of claim 19 or 20, which is **characterized in that**: the X-ray powder diffraction pattern thereof obtained using CuK_{α} radiation further includes the characteristic peaks located at the following °2θ: 5.8±0.2, 6.4±0.2, 10.8±0.2, 14.9±0.2, 15.3±0.2, 16.4±0.2, 19.3±0.2, 20.6±0.2, 23.3±0.2, 25.1±0.2 and 26.9±0.2.

22. The crystal form II of the compound of formula (A) of claim 19, which is **characterized by**: having an X-ray powder diffraction pattern substantially as shown in FIG. 4.

23. The crystal form II of the compound of formula (A) of any one of claims 19-22, which is also **characterized by**: having a melting endothermic peak at 115±2°C in differential scanning calorimetry analysis.

24. The crystal form II of the compound of formula (A) of any one of claims 19-23, which is also **characterized by**: having a weight loss of about 1.9% prior to 200°C in thermogravimetric analysis.

25. The crystal form VII of the compound of formula (A), which is **characterized in that**: the X-ray powder diffraction pattern thereof obtained using CuK_{α} radiation includes at least the characteristic peaks located at the following °2θ: 11.7±0.2, 16.9±0.2, 18.3±0.2, 20.9±0.2, 21.7±0.2, 23.2±0.2, 23.8±0.2 and 27.1±0.2.

26. The crystal form VII of the compound of formula (A) of claim 25, which is **characterized in that**: the X-ray powder diffraction pattern thereof obtained using CuK_{α} radiation further includes the characteristic peaks located at the following °2θ: 13.4±0.2, 16.0±0.2, 20.7±0.2 and 22.2±0.2.

27. The crystal form VII of the compound of formula (A) of claim 26, which is **characterized in that**: the X-ray powder diffraction pattern thereof obtained using CuK_{α} radiation has the following characteristic peaks:
| Angle °2θ±0.2 °2θ | Relative intensity % |
|---|---|
| 11.7 | 92.9 |
| 13.4 | 25.2 |
| 16.0 | 39.8 |
| 16.9 | 97.3 |
| 18.3 | 58 |
| 20.7 | 29.4 |
| 20.9 | 99.3 |
| 21.7 | 76.5 |
| 22.2 | 35.4 |
| 23.2 | 68.5 |
| 23.8 | 100 |
| 27.1 | 56.3 |

28. The crystal form VII of the compound of formula (A) of claim 26 or 27, which is **characterized in that**: the X-ray powder diffraction pattern thereof obtained using CuK_{α} radiation further includes the characteristic peaks located at the following °2θ: 13.2±0.2, 19.6±0.2, 21.3±0.2, 25.7±0.2 and 30.9±0.2.

29. The crystal form VII of the compound of formula (A) of claim 25, which is **characterized by**: having an X-ray powder diffraction pattern substantially as shown in FIG. 24.

30. The crystal form VII of the compound of formula (A) of any one of claims 25-29, which is also **characterized by**: having a melting endothermic peak at 174±2°C in differential scanning calorimetry analysis.

31. The crystal form VII of the compound of formula (A) of any one of claims 25-30, which is also **characterized by**: having a weight loss of about 3.4% prior to 200°C in thermogravimetric analysis.

32. The crystal form III of the compound of formula (A), which is **characterized in that**: the X-ray powder diffraction pattern thereof obtained using CuK_{α} radiation includes at least the characteristic peaks located at the following °2θ: 12.4±0.2, 14.2±0.2, 20.0±0.2 and 21.9±0.2.

33. The crystal form III of the compound of formula (A) of claim 32, which is **characterized in that**: the X-ray powder diffraction pattern thereof obtained using CuK_{α} radiation further includes the characteristic peaks located at the following °2θ: 7.0±0.2, 9.3±0.2, 13.9±0.2 and 24.5±0.2.

34. The crystal form III of the compound of formula (A) of claim 33, which is **characterized in that**: the X-ray powder diffraction pattern thereof obtained using CuK_{α} radiation has the following characteristic peaks:
| Angle °2θ±0.2 °2θ | Relative intensity % |
|---|---|
| 7.0 | 27.5 |
| 9.3 | 39.6 |
| 12.4 | 94.4 |
| 13.9 | 27.3 |
| 14.2 | 91.4 |
| 20.0 | 100 |
| 21.9 | 65.9 |
| 24.5 | 41.9 |

35. The crystal form III of the compound of formula (A) of claim 33 or 34, which is **characterized in that**: the X-ray powder diffraction pattern thereof obtained using CuK_{α} radiation further includes the characteristic peaks located at the following °2θ: 15.3±0.2, 20.6±0.2, 23.4±0.2, 27.5±0.2, 28.3±0.2 and 28.8±0.2.

36. The crystal form III of the compound of formula (A) of claim 32, which is **characterized by**: having an X-ray powder diffraction pattern substantially as shown in FIG. 5.

37. The crystal form III of the compound of formula (A) of any one of claims 32-36, which is also **characterized by**: having a melting endothermic peak at 173±2°C in differential scanning calorimetry analysis.

38. The crystal form III of the compound of formula (A) of any one of claims 32-37, which is also **characterized by**: having a weight loss of about 3.5% prior to 200°C in thermogravimetric analysis.

39. The crystal form X of the compound of formula (A), which is **characterized in that**: the X-ray powder diffraction pattern thereof obtained using CuK_{α} radiation includes at least the characteristic peaks located at the following °2θ: 7.0±0.2, 9.3±0.2, 12.3±0.2, 14.2±0.2, 16.3±0.2, 18.8±0.2, 19.9±0.2, 21.4±0.2 and 23.9±0.2.

40. The crystal form X of the compound of formula (A) of claim 39, which is **characterized in that**: the X-ray powder diffraction pattern thereof obtained using CuK_{α} radiation has the following characteristic peaks:
| Angle °2θ±0.2 °2θ | Relative intensity % |
|---|---|
| 7.0 | 70.1 |
| 9.3 | 63.1 |
| 12.3 | 100 |
| 14.2 | 80.4 |
| 16.3 | 54.8 |
| 18.8 | 60.4 |
| 19.9 | 93.8 |
| 21.4 | 63.3 |
| 23.9 | 80.1 |

41. The crystal form X of the compound of formula (A) of claim 39 or 40, which is **characterized in that**: the X-ray powder diffraction pattern thereof obtained using CuK_{α} radiation further includes the characteristic peaks located at the following °2θ: 15.4±0.2, 19.2±0.2, 19.5±0.2, 22.2±0.2, 23.4±0.2, 24.8±0.2, 25.8±0.2, 26.2±0.2, 26.7±0.2, 28.8±0.2 and 29.2±0.2.

42. The crystal form X of the compound of formula (A) of claim 39, which is **characterized by**: having an X-ray powder diffraction pattern substantially as shown in FIG. 27.

43. The crystal form X of the compound of formula (A) of any one of claims 39-42, which is also **characterized by**: having a melting endothermic peak at 173±2°C in differential scanning calorimetry analysis.

44. The crystal form X of the compound of formula (A) of any one of claims 39-43, which is also **characterized by**: having a weight loss of about 3.6% prior to 200°C in thermogravimetric analysis.

45. The crystal form IV of the compound of formula (A), which is **characterized in that**: the X-ray powder diffraction pattern thereof obtained using CuK_{α} radiation includes at least the characteristic peaks located at the following °2θ: 9.7±0.2, 22.5±0.2 and 24.4±0.2.

46. The crystal form IV of the compound of formula (A) of claim 45, which is **characterized in that**: the X-ray powder diffraction pattern thereof obtained using CuK_{α} radiation further includes the characteristic peaks located at the following °2θ: 6.2±0.2, 12.9±0.2, 18.4±0.2, 21.8±0.2, 23.2±0.2 and 24.8±0.2.

47. The crystal form IV of the compound of formula (A) of claim 46, which is **characterized in that**: the X-ray powder diffraction pattern thereof obtained using CuK_{α} radiation has the following characteristic peaks:
| Angle °2θ±0.2 °2θ | Relative intensity % |
|---|---|
| 6.2 | 43.4 |
| 9.7 | 59.9 |
| 12.9 | 40.3 |
| 18.4 | 38.3 |
| 21.8 | 48.2 |
| 22.5 | 100 |
| 23.2 | 38.8 |
| 24.4 | 82.9 |
| 24.8 | 29.7 |

48. The crystal form IV of the compound of formula (A) of claim 46 or 47, which is **characterized in that**: the X-ray powder diffraction pattern thereof obtained using CuK_{α} radiation further includes the characteristic peaks located at the following °2θ: 6.4±0.2, 10.8±0.2, 12.3±0.2, 16.0±0.2, 19.2±0.2, 21.1±0.2 and 27.9±0.2.

49. The crystal form IV of the compound of formula (A) of claim 45, which is **characterized by**: having an X-ray powder diffraction pattern substantially as shown in FIG. 6.

50. The crystal form IV of the compound of formula (A) of any one of claims 45-49, which is also **characterized by**: having endothermic peaks at 176±2°C and 251±2°C in differential scanning calorimetry analysis.

51. The crystal form IV of the compound of formula (A) of any one of claims 45-50, which is also **characterized by**: having a weight loss of about 3.7% prior to 200°C in thermogravimetric analysis.

52. The crystal form IV of the compound of formula (A), which is **characterized by**: having the following parameters:
| Space group | *C*2 |
|---|---|
| *a*/Å | 31.9592(11) |
| *b*/Å | 9.58750(10) |
| *c*/Å | 31.2990(8) |
| *α*/° | 90 |
| *β*/° | 116.850(3) |
| *γ*/° | 90 |
| Volume/Å³ | 8556.4(4) |

53. The crystal form XI of the compound of formula (A), which is **characterized in that**: the X-ray powder diffraction pattern thereof obtained using CuK_{α} radiation includes at least the characteristic peaks located at the following °2θ: 9.7±0.2, 17.9±0.2, 19.5±0.2, 24.2±0.2 and 24.8±0.2.

54. The crystal form XI of the compound of formula (A) of claim 53, which is **characterized in that**: the X-ray powder diffraction pattern thereof obtained using CuK_{α} radiation further includes the characteristic peaks located at the following °2θ: 7.6±0.2, 12.7±0.2, 13.2±0.2, 18.7±0.2, 18.9±0.2, 22.4±0.2 and 25.6±0.2.

55. The crystal form XI of the compound of formula (A) of claim 54, which is **characterized in that**: the X-ray powder diffraction pattern thereof obtained using CuK_{α} radiation has the following characteristic peaks:
| Angle °2θ±0.2 °2θ | Relative intensity % |
|---|---|
| 7.6 | 10.4 |
| 9.7 | 73.3 |
| 12.7 | 11.7 |
| 13.2 | 14.7 |
| 17.9 | 100 |
| 18.7 | 18.4 |
| 18.9 | 11 |
| 19.5 | 41.4 |
| 22.4 | 10.5 |
| 24.2 | 32.4 |
| 24.8 | 33.8 |
| 25.6 | 19.8 |

56. The crystal form XI of the compound of formula (A) of claim 54 or 55, which is **characterized in that**: the X-ray powder diffraction pattern thereof obtained using CuK_{α} radiation further includes the characteristic peaks located at the following °2θ: 14.6±0.2, 16.7±0.2, 20.2±0.2, 20.7±0.2, 21.0±0.2, 21.3±0.2, 26.0±0.2 and 29.9±0.2.

57. The crystal form XI of the compound of formula (A) of claim 53, which is **characterized by**: having an X-ray powder diffraction pattern substantially as shown in FIG. 28.

58. The crystal form XI of the compound of formula (A) of any one of claims 53-57, which is also **characterized by**: having a melting endothermic peak at 174±2°C in differential scanning calorimetry analysis.

59. The crystal form XI of the compound of formula (A) of any one of claims 53-58, which is also **characterized by**: having a weight loss of about 3.9% prior to 200°C in thermogravimetric analysis.

60. The crystal form IX of the compound of formula (A), which is **characterized in that**: the X-ray powder diffraction pattern thereof obtained using CuK_{α} radiation includes at least the characteristic peaks located at the following °2θ: 9.6±0.2, 18.2±0.2 and 22.1±0.2.

61. The crystal form IX of the compound of formula (A) of claim 60, which is **characterized in that**: the X-ray powder diffraction pattern thereof obtained using CuK_{α} radiation further includes the characteristic peaks located at the following °2θ: 6.1±0.2, 12.1±0.2, 12.4±0.2, 19.0±0.2, 19.3±0.2, 21.5±0.2, 24.4±0.2 and 24.9±0.2.

62. The crystal form IX of the compound of formula (A) of claim 61, which is **characterized in that**: the X-ray powder diffraction pattern thereof obtained using CuK_{α} radiation has the following characteristic peaks:
| Angle °2θ±0.2 °2θ | Relative intensity % |
|---|---|
| 6.1 | 10 |
| 9.6 | 100 |
| 12.1 | 20 |
| 12.4 | 10.3 |
| 18.2 | 85.5 |
| 19.0 | 11.1 |
| 19.3 | 16.3 |
| 21.5 | 23.5 |
| 22.1 | 30 |
| 24.4 | 21.5 |
| 24.9 | 13.2 |

63. The crystal form IX of the compound of formula (A) of claim 61 or 62, which is **characterized in that**: the X-ray powder diffraction pattern thereof obtained using CuK_{α} radiation further includes the characteristic peaks located at the following °2θ: 10.5±0.2, 16.1±0.2, 20.6±0.2, 21.2±0.2, 23.6±0.2, 26.9±0.2, 27.7±0.2 and 28.4±0.2.

64. The crystal form IX of the compound of formula (A) of claim 60, which is **characterized by**: having an X-ray powder diffraction pattern substantially as shown in FIG. 26.

65. The crystal form IX of the compound of formula (A) of any one of claims 60-64, which is also **characterized by**: having a melting endothermic peak at 174±2°C in differential scanning calorimetry analysis.

66. The crystal form IX of the compound of formula (A) of any one of claims 60-65, which is also **characterized by**: having a weight loss of about 5.8% prior to 200°C in thermogravimetric analysis.

67. The crystal form V of the compound of formula (A), which is **characterized in that**: the X-ray powder diffraction pattern thereof obtained using CuK_{α} radiation includes at least the characteristic peaks located at the following °2θ: 9.4±0.2, 18.1±0.2, 18.9±0.2, 21.3±0.2 and 23.6±0.2.

68. The crystal form V of the compound of formula (A) of claim 67, which is **characterized in that**: the X-ray powder diffraction pattern thereof obtained using CuK_{α} radiation further includes the characteristic peaks located at the following °2θ: 24.3±0.2 and 26.8±0.2.

69. The crystal form V of the compound of formula (A) of claim 68, which is **characterized in that**: the X-ray powder diffraction pattern thereof obtained using CuK_{α} radiation has the following characteristic peaks:
| Angle °2θ±0.2 °2θ | Relative intensity % |
|---|---|
| 9.4 | 81.6 |
| 18.1 | 90.8 |
| 18.9 | 51.7 |
| 21.3 | 61.6 |
| 23.6 | 100 |
| 24.3 | 45.7 |
| 26.8 | 34.3 |

70. The crystal form V of the compound of formula (A) of claim 68 or 69, which is **characterized in that**: the X-ray powder diffraction pattern thereof obtained using CuK_{α} radiation further includes the characteristic peaks located at the following °2θ: 9.9±0.2, 11.8±0.2, 15.6±0.2, 15.9±0.2, 17.6±0.2, 18.4±0.2, 19.1±0.2, 19.8±0.2, 21.8±0.2 and 23.2±0.2.

71. The crystal form V of the compound of formula (A) of claim 67, which is **characterized by**: having an X-ray powder diffraction pattern substantially as shown in FIG. 7.

72. The crystal form V of the compound of formula (A) of any one of claims 67-71, which is also **characterized by**: having a melting endothermic peak at 173±2°C in differential scanning calorimetry analysis.

73. The crystal form V of the compound of formula (A) of any one of claims 67-72, which is also **characterized by**: having a weight loss of about 6.5% prior to 200°C in thermogravimetric analysis.

74. The crystal form VIII of the compound of formula (A), which is **characterized in that**: the X-ray powder diffraction pattern thereof obtained using CuK_{α} radiation includes at least the characteristic peaks located at the following °2θ: 9.6±0.2, 12.7±0.2, 18.3±0.2, 19.1±0.2, 22.8±0.2 and 24.3±0.2.

75. The crystal form VIII of the compound of formula (A) of claim 74, which is **characterized in that**: the X-ray powder diffraction pattern thereof obtained using CuK_{α} radiation has the following characteristic peaks:
| Angle °2θ±0.2 °2θ | Relative intensity % |
|---|---|
| 9.6 | 100 |
| 12.7 | 19.2 |
| 18.3 | 82.8 |
| 19.1 | 12.6 |
| 22.8 | 13.6 |
| 24.3 | 22.7 |

76. The crystal form VIII of the compound of formula (A) of claim 74 or 75, which is **characterized in that**: the X-ray powder diffraction pattern thereof obtained using CuK_{α} radiation further includes the characteristic peaks located at the following °2θ: 10.7±0.2, 12.1±0.2, 19.4±0.2, 21.8±0.2, 22.5±0.2, 24.7±0.2 and 27.6±0.2.

77. The crystal form VIII of the compound of formula (A) of claim 74, which is **characterized by**: having an X-ray powder diffraction pattern substantially as shown in FIG. 25.

78. The crystal form I of p-toluenesulfonate of the compound of formula (A) (1:1), which is **characterized in that**: the X-ray powder diffraction pattern thereof obtained using CuK_{α} radiation includes at least the characteristic peaks located at the following °2θ: 7.4±0.2, 10.2±0.2, 21.3±0.2 and 21.9±0.2.

79. The crystal form I of p-toluenesulfonate of the compound of formula (A) (1:1) of claim 78, which is **characterized in that**: the X-ray powder diffraction pattern thereof obtained using CuK_{α} radiation further includes the characteristic peak located at the following °2θ: 27.9±0.2.

80. The crystal form I of p-toluenesulfonate of the compound of formula (A) (1:1) of claim 79, which is **characterized in that**: the X-ray powder diffraction pattern thereof obtained using CuK_{α} radiation has the following characteristic peaks:
| Angle °2θ±0.2 °2θ | Relative intensity % |
|---|---|
| 7.4 | 63.7 |
| 10.2 | 100 |
| 21.3 | 54.5 |
| 21.9 | 92.2 |
| 27.9 | 34.3 |

81. The crystal form I of p-toluenesulfonate of the compound of formula (A) (1:1) of claim 79 or 80, which is **characterized in that**: the X-ray powder diffraction pattern thereof obtained using CuK_{α} radiation further includes the characteristic peaks located at the following °2θ: 14.9±0.2, 16.3±0.2, 19.2±0.2 and 23.0±0.2.

82. The crystal form I of p-toluenesulfonate of the compound of formula (A) (1:1) of claim 78, which is **characterized by**: having an X-ray powder diffraction pattern substantially as shown in FIG. 37.

83. The crystal form I of p-toluenesulfonate of the compound of formula (A) (1:1) of any one of claims 78-82, which is also **characterized by**: having a melting endothermic peak at 236±2°C in differential scanning calorimetry analysis.

84. The crystal form I of p-toluenesulfonate of the compound of formula (A) (1:1) of any one of claims 78-83, which is also **characterized by**: having a weight loss of about 0.1% prior to 200°C in thermogravimetric analysis.

85. The crystal form I of benzenesulfonate of the compound of formula (A) (1:1), which is **characterized in that**: the X-ray powder diffraction pattern thereof obtained using CuK_{α} radiation includes at least the characteristic peaks located at the following °2θ: 7.6±0.2, 10.2±0.2 and 22.8±0.2.

86. The crystal form I of benzenesulfonate of the compound of formula (A) (1:1) of claim 85, which is **characterized in that**: the X-ray powder diffraction pattern thereof obtained using CuK_{α} radiation further includes the characteristic peaks located at the following °2θ: 16.4±0.2, 19.1±0.2, 19.9±0.2, 21.3±0.2, 21.9±0.2 and 23.3±0.2.

87. The crystal form I of benzenesulfonate of the compound of formula (A) (1:1) of claim 86, which is **characterized in that**: the X-ray powder diffraction pattern thereof obtained using CuK_{α} radiation has the following characteristic peaks:
| Angle °2θ±0.2 °2θ | Relative intensity % |
|---|---|
| 7.6 | 50.1 |
| 10.2 | 100 |
| 16.4 | 46.3 |
| 19.1 | 27.7 |
| 19.9 | 26.9 |
| 21.3 | 25.9 |
| 21.9 | 25.6 |
| 22.8 | 69.2 |
| 23.3 | 43.1 |

88. The crystal form I of benzenesulfonate of the compound of formula (A) (1:1) of claim 86 or 87, which is **characterized in that**: the X-ray powder diffraction pattern thereof obtained using CuK_{α} radiation further includes the characteristic peaks located at the following °2θ: 20.5±0.2, 21.6±0.2 and 25.0±0.2.

89. The crystal form I of benzenesulfonate of the compound of formula (A) (1:1) of claim 85, which is **characterized by**: having an X-ray powder diffraction pattern substantially as shown in FIG. 34.

90. The crystal form I of benzenesulfonate of the compound of formula (A) (1:1) of any one of claims 85-89, which is also **characterized by**: having a melting endothermic peak at 253±2°C in differential scanning calorimetry analysis.

91. The crystal form I of benzenesulfonate of the compound of formula (A) (1:1) of any one of claims 85-90, which is also **characterized by**: having a weight loss of about 0.5% prior to 200°C in thermogravimetric analysis.

92. The crystal form I of hydrochloride of the compound of formula (A), which is **characterized in that**: the X-ray powder diffraction pattern thereof obtained using CuK_{α} radiation includes at least the characteristic peaks located at the following °2θ: 6.5±0.2, 13.8±0.2, 18.7±0.2 and 23.3±0.2.

93. The crystal form I of hydrochloride of the compound of formula (A) of claim 92, which is **characterized in that**: the X-ray powder diffraction pattern thereof obtained using CuK_{α} radiation further includes the characteristic peaks located at the following °2θ: 11.3±0.2, 15.7±0.2, 16.4±0.2, 21.9±0.2, 22.7±0.2, 24.2±0.2 and 28.6±0.2.

94. The crystal form I of hydrochloride of the compound of formula (A) of claim 93, which is **characterized in that**: the X-ray powder diffraction pattern thereof obtained using CuK_{α} radiation has the following characteristic peaks:
| Angle °2θ±0.2 °2θ | Relative intensity % |
|---|---|
| 6.5 | 86.2 |
| 11.3 | 25.4 |
| 13.8 | 90.2 |
| 15.7 | 29.8 |
| 16.4 | 32.7 |
| 18.7 | 100 |
| 21.9 | 31.6 |
| 22.7 | 33.5 |
| 23.3 | 57.7 |
| 24.2 | 37.9 |
| 28.6 | 32.7 |

95. The crystal form I of hydrochloride of the compound of formula (A) of claim 93 or 94, which is **characterized in that**: the X-ray powder diffraction pattern thereof obtained using CuK_{α} radiation further includes the characteristic peaks located at the following °2θ: 16.8±0.2, 19.7±0.2, 20.8±0.2, 21.3±0.2, 25.2±0.2 and 35.0±0.2.

96. The crystal form I of hydrochloride of the compound of formula (A) of claim 92, which is **characterized by**: having an X-ray powder diffraction pattern substantially as shown in FIG. 31.

97. The crystal form I of hydrochloride of the compound of formula (A) of any one of claims 92-96, which is also **characterized by**: having a melting endothermic peak at 229±2°C in differential scanning calorimetry analysis.

98. The crystal form I of hydrochloride of the compound of formula (A) of any one of claims 92-97, which is also **characterized by**: having a weight loss of about 0.7% prior to 175°C in thermogravimetric analysis.

99. The crystal form I of mesylate of the compound of formula (A) (1:2), which is **characterized in that**: the X-ray powder diffraction pattern thereof obtained using CuK_{α} radiation includes at least the characteristic peaks located at the following °2θ: 19.7±0.2 and 23.7±0.2.

100. The crystal form I of mesylate of the compound of formula (A) (1:2) of claim 99, which is **characterized in that**: the X-ray powder diffraction pattern thereof obtained using CuK_{α} radiation further includes the characteristic peaks located at the following °2θ: 4.7±0.2, 16.4±0.2, 18.7±0.2, 19.2±0.2, 22.3±0.2 and 22.7±0.2.

101. The crystal form I of mesylate of the compound of formula (A) (1:2) of claim 100, which is **characterized in that**: the X-ray powder diffraction pattern thereof obtained using CuK_{α} radiation has the following characteristic peaks:
| Angle °2θ±0.2 °2θ | Relative intensity % |
|---|---|
| 4.7 | 32.6 |
| 16.4 | 32.5 |
| 18.7 | 48.5 |
| 19.2 | 49.8 |
| 19.7 | 74.8 |
| 22.3 | 28.1 |
| 22.7 | 42.3 |
| 23.7 | 100 |

102. The crystal form I of mesylate of the compound of formula (A) (1:2) of claim 100 or 101, which is **characterized in that**: the X-ray powder diffraction pattern thereof obtained using CuK_{α} radiation further includes the characteristic peaks located at the following °2θ: 20.1±0.2, 28.1±0.2 and 37.1±0.2.

103. The crystal form I of mesylate of the compound of formula (A) (1:2) of claim 99, which is **characterized by**: having an X-ray powder diffraction pattern substantially as shown in FIG. 41.

104. The crystal form I of mesylate of the compound of formula (A) (1:2) of any one of claims 99-103, which is also **characterized by**: having a melting endothermic peak at 206±2°C in differential scanning calorimetry analysis.

105. The crystal form I of mesylate of the compound of formula (A) (1:2) of any one of claims 99-104, which is also **characterized by**: having a weight loss of about 0.7% prior to 200°C in thermogravimetric analysis.

106. The crystal form I of hydrobromide of the compound of formula (A), which is **characterized in that**: the X-ray powder diffraction pattern thereof obtained using CuK_{α} radiation includes at least the characteristic peaks located at the following °2θ: 6.4±0.2, 13.5±0.2, 20.6±0.2, 21.1±0.2, 23.8±0.2, 24.0±0.2 and 26.1±0.2.

107. The crystal form I of hydrobromide of the compound of formula (A) of claim 106, which is **characterized in that**: the X-ray powder diffraction pattern thereof obtained using CuK_{α} radiation has the following characteristic peaks:
| Angle °2θ±0.2 °2θ | Relative intensity % |
|---|---|
| 6.4 | 100 |
| 13.5 | 30.7 |
| 20.6 | 34.9 |
| 21.1 | 25.5 |
| 23.8 | 31.1 |
| 24.0 | 40.2 |
| 26.1 | 31.2 |

108. The crystal form I of hydrobromide of the compound of formula (A) of claim 106 or 107, which is **characterized in that**: the X-ray powder diffraction pattern thereof obtained using CuK_{α} radiation further includes the characteristic peaks located at the following °2θ: 13.0±0.2 and 28.5±0.2.

109. The crystal form I of hydrobromide of the compound of formula (A) of claim 106, which is **characterized by**: having an X-ray powder diffraction pattern substantially as shown in FIG. 44.

110. The crystal form I of hydrobromide of the compound of formula (A) of any one of claims 106-109, which is also **characterized by**: having a melting endothermic peak at 251±2°C in differential scanning calorimetry analysis.

111. The crystal form I of hydrobromide of the compound of formula (A) of any one of claims 106-110, which is also **characterized by**: having a weight loss of about 0.6% prior to 200°C in thermogravimetric analysis.

112. The crystal form II of p-toluenesulfonate of the compound of formula (A) (1:1), which is **characterized in that**: the X-ray powder diffraction pattern thereof obtained using CuK_{α} radiation includes at least the characteristic peaks located at the following °2θ: 6.4±0.2, 7.4±0.2, 10.2±0.2, 16.3±0.2, 21.3±0.2 and 21.8±0.2.

113. The crystal form II of p-toluenesulfonate of the compound of formula (A) (1:1) of claim 112, which is **characterized in that**: the X-ray powder diffraction pattern thereof obtained using CuK_{α} radiation has the following characteristic peaks:
| Angle °2θ±0.2 °2θ | Relative intensity % |
|---|---|
| 6.4 | 100 |
| 7.4 | 10.1 |
| 10.2 | 25.6 |
| 16.3 | 5.6 |
| 21.3 | 12.1 |
| 21.8 | 5.2 |

114. The crystal form II of p-toluenesulfonate of the compound of formula (A) (1:1) of claim 112, which is **characterized by**: having an X-ray powder diffraction pattern substantially as shown in FIG. 40.

115. The crystal form II of p-toluenesulfonate of the compound of formula (A) (1:1) of any one of claims 112-113, which is also **characterized by**: having a melting endothermic peak at 234±2°C in differential scanning calorimetry analysis.

116. The crystal form II of p-toluenesulfonate of the compound of formula (A) (1:1) of any one of claims 112-114, which is also **characterized by**: having a weight loss of about 0.2% prior to 200°C in thermogravimetric analysis.

117. A method for the preparation of the compound of formula (A): 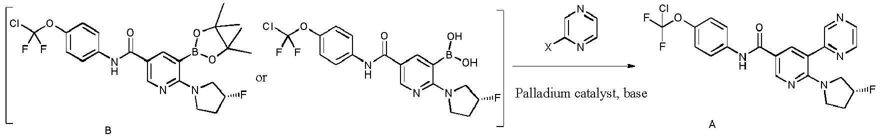 wherein X is a halogen; alternatively I or Br; still alternatively I;
comprising reacting the compound of formula (B), boronic acid hydrolysis product thereof, or a mixture of the two with 2-halopyrazine in DMSO or DMF in the presence of a palladium catalyst and a base.

118. The method of claim 117, wherein the DMSO or DMF contains water in a volume ratio of 0.01-0.5:1; alternatively, in a volume ratio of 0.05-0.2:1, alternatively, 0.067:1, 0.1:1, or 0.2:1.

119. The method of claim 117 or 118, wherein the base is selected from sodium phosphate, potassium phosphate, sodium carbonate, potassium carbonate, cesium carbonate, quaternary ammonium salt, NaF, KF, CsF, sodium bicarbonate, potassium bicarbonate, dibasic sodium phosphate, or dibasic potassium phosphate.

120. The method of claim 119, wherein the quaternary ammonium salt is selected from organic quaternary ammonium salts including tetrabutylammonium fluoride, tetrabutylammonium bromide, tetraethylammonium fluoride, tetraethylammonium bromide, tetramethylammonium fluoride, tetramethylammonium bromide, or tetramethylammonium chloride; alternatively, tetrabutylammonium fluoride.

121. The method of claim 117, wherein the base is selected from sodium phosphate, potassium phosphate, sodium carbonate, potassium carbonate, cesium carbonate, tetrabutylammonium fluoride, NaF, KF, or CsF.

122. The method of any one of claims 117-121, wherein the palladium catalyst is selected from 1,1'-bis(diphenylphosphino)ferrocene palladium dichloride, palladium acetate, tetrakis(triphenylphosphine)palladium, tris(dibenzylideneacetone)dipalladium, palladium dichloride or bis(triphenylphosphine)palladium dichloride; alternatively, 1,1'-bis(diphenylphosphino)ferrocene palladium dichloride, tetrakis(triphenylphosphine)palladium, tris(dibenzylideneacetone)dipalladium or bis(triphenylphosphine)palladium dichloride; alternatively, 1,1'-bis(diphenylphosphino)ferrocene palladium dichloride.

123. The method of any one of claims 117-122, wherein the molar ratio of 2-halopyrazine to compound B or boronic acid hydrolysis product thereof or a mixture of the two is 0.8-1.5:1; alternatively, 1.2:1.

124. The method of any one of claims 117-123, wherein the amount of the base is 1-3.5 times that of compound B or boronic acid hydrolysis product thereof or a mixture of the two; alternatively, 1.2-2.7 times; alternatively, 1.2, 1.5, 1.8, 2.0, 2.1, 2.2, 2.4 or 2.7 times.

125. The method of any one of claims 117-124, wherein the amount of the palladium catalyst is 0.005-0.1 times that of compound B or boronic acid hydrolysis product thereof or a mixture of the two; alternatively, 0.01-0.05 times; alternatively, 0.01, 0.02, 0.03, 0.04 or 0.05 times.

126. The method of any one of claims 117-125, wherein the reaction is carried out at a temperature of room temperature to the temperature of solvent reflux, alternatively, 30-80°C, or still alternatively, 30±5°C, 50±5°C, 65±5°C or 80±5°C; alternatively, for a reaction period of at least 1 hour, alternatively, at least 3 hours.

127. A method for the preparation of the compound of formula (A): 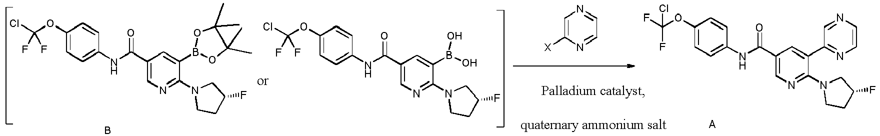 wherein X is a halogen; alternatively I or Br; still alternatively I;
comprising reacting the compound of formula (B), boronic acid hydrolysis product thereof, or a mixture of the two with 2-halopyrazine in the presence of a palladium catalyst and a quaternary ammonium salt.

128. The method of claim 127, wherein the quaternary ammonium salt is selected from organic quaternary ammonium salts including tetrabutylammonium fluoride, tetrabutylammonium bromide, tetraethylammonium fluoride, tetraethylammonium bromide, tetramethylammonium fluoride, tetramethylammonium bromide, or tetramethylammonium chloride; alternatively, tetrabutylammonium fluoride.

129. The method of claim 127 or 128, wherein the palladium catalyst is selected from 1,1'-bis(diphenylphosphino)ferrocene palladium dichloride, palladium acetate, tetrakis(triphenylphosphine)palladium, tris(dibenzylideneacetone)dipalladium, palladium dichloride or bis(triphenylphosphine)palladium dichloride; alternatively, 1,1'-bis(diphenylphosphino)ferrocene palladium dichloride, tetrakis(triphenylphosphine)palladium, tris(dibenzylideneacetone)dipalladium or bis(triphenylphosphine)palladium dichloride; alternatively, 1,1'-bis(diphenylphosphino)ferrocene palladium dichloride.

130. The method of any one of claims 127-129, wherein the molar ratio of 2-halopyrazine to compound B or boronic acid hydrolysis product thereof or a mixture of the two is 0.8-1.5:1; alternatively, 1.2:1.

131. The method of any one of claims 127-130, wherein the amount of the base is 1-3.5 times that of compound B or boronic acid hydrolysis product thereof or a mixture of the two; alternatively, 1.2-2.7 times; alternatively, 1.2, 1.5, 1.8, 2.0, 2.1, 2.2, 2.4 or 2.7 times.

132. The method of any one of claims 127-131, wherein the amount of the palladium catalyst is 0.005-0.1 times that of compound B or boronic acid hydrolysis product thereof or a mixture of the two; alternatively, 0.01-0.05 times; alternatively, 0.01, 0.02, 0.03, 0.04 or 0.05 times.

133. The method of any one of claims 127-132, wherein the reaction is carried out in a solvent selected from DCM, DCE, ethyl acetate, methyl acetate, isopropyl acetate, n-hexane, n-heptane, petroleum ether, acetone, acetonitrile, toluene, methyl tert-butyl ether, tetrahydrofuran, 2-methyltetrahydrofuran, isopropanol, water, ethylene glycol monomethyl ether, ethylene glycol dimethyl ether, DMF, DMA, DMSO, or a mixture thereof; alternatively, the solvent is selected from tetrahydrofuran, 2-methyltetrahydrofuran, ethylene glycol monomethyl ether, isopropanol, water, toluene, DMF, DMSO or a mixture thereof; alternatively, the solvent is tetrahydrofuran, DMF, isopropanol, water, toluene or a mixture thereof.

134. The method of any one of claims 127-133, wherein the reaction is carried out at a temperature of room temperature to the temperature of solvent reflux, alternatively, 30-80°C, or still alternatively, 30±5°C, 50±5°C, 65±5°C or 80±5°C; alternatively, for a reaction period of at least 1 hour, alternatively, at least 3 hours.

135. A method for the preparation of compound B or boronic acid hydrolysis product thereof or a mixture of the two, comprising: 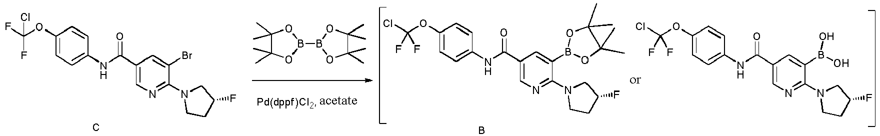 reacting a compound of formula (C) with bis(pinacolato)diboron in a solvent in the presence of a palladium catalyst Pd(dppf)Cl₂ and an acetate, wherein the solvent is selected from DMSO, DCM, DCE, ethyl acetate, methyl acetate, isopropyl acetate, acetone, acetonitrile, methyl tert-butyl ether, ethylene glycol monomethyl ether, ethylene glycol dimethyl ether, DMF or DMA; alternatively, DMSO, ethylene glycol monomethyl ether, or DMF; alternatively, DMSO.

136. The method of claim 135, wherein the acetate is selected from potassium acetate, sodium acetate, or cesium acetate; alternatively, potassium acetate.

137. The method of claim 135 or 136, wherein the amount of the acetate is 2-5 times that of compound C; alternatively, 2.5 times.

138. The method of any one of claims 135-137, wherein the amount of the bis(pinacolato)diboron is 3-6 times that of compound C; alternatively, 3 times.

139. The method of any one of claims 135-138, wherein the amount of the catalyst is 0.01-0.1 times that of compound C, alternatively, 0.05 times.

140. The method of any one of claims 135-139, wherein the reaction is carried out at a temperature of 60°C to the temperature of solvent reflux, alternatively, 60-100°C, or still alternatively, 80±10°C; alternatively, for a reaction period of at least 1 hour, alternatively, at least 2 hours.

141. A pharmaceutical composition, comprising the following ingredients:
(i) the crystal form of any one of claims 1-116,
(ii) a diluent,
(iii) a disintegrant,
(iv) a glidant, and
(v) a lubricant.

142. The pharmaceutical composition of claim 141, wherein the crystal form accounts for 1-30%, alternatively, 5-20%, alternatively, 8-15%, or still alternatively, about 10% by weight of the total weight of the pharmaceutical composition, based on the weight of the free base of the compound; alternatively, wherein the amount of the crystal form in a unit dose is 1-100 mg, alternatively, 5-50 mg, alternatively, 8-40 mg, alternatively, about 10, 20, 30 or 40 mg.

143. The pharmaceutical composition of any one of claims 141-142, wherein the diluent accounts for 65-95%, alternatively, 70-90%, alternatively, about 80%, 81%, 82%, 83%, 84% or 85% by weight of the total weight of the pharmaceutical composition; alternatively, wherein the amount of the diluent in a unit dose is 65-380 mg, alternatively, 70-360 mg, alternatively, 80-350 mg, such as, about 83 mg or 332 mg.

144. The pharmaceutical composition of any one of claims 141-143, wherein the diluent is selected from lactose monohydrate, microcrystalline cellulose, anhydrous calcium hydrogen phosphate, mannitol and pregelatinized starch, and mixtures thereof; alternatively, where both of lactose monohydrate and microcrystalline cellulose are present, the weight ratio of lactose monohydrate to microcrystalline cellulose is 5:1 to 1:5, alternatively, 2:1 to 1:3, alternatively, about 1:2, e.g. 1:1.96.

145. The pharmaceutical composition of any one of claims 141-144, wherein the disintegrant accounts for 1-5%, alternatively, 2-4%, alternatively, about 3% by weight of the total weight of the pharmaceutical composition; alternatively, wherein the amount of the disintegrant in a unit dose is 1-20 mg, alternatively, 2-16 mg, alternatively, about 3, 6, 9 or 12 mg.

146. The pharmaceutical composition of any one of claims 141-145, wherein the disintegrant is croscarmellose sodium.

147. The pharmaceutical composition of any one of claims 141-146, wherein the glidant accounts for 1-5%, alternatively, 2-4%, alternatively, about 3% by weight of the total weight of the pharmaceutical composition; alternatively, wherein the amount of (iv) glidant in a unit dose is 1-20 mg, alternatively, 2-16 mg, alternatively, about 3, 6, 9 or 12 mg.

148. The pharmaceutical composition of any one of claims 141-147, wherein the glidant is colloidal silica.

149. The pharmaceutical composition of any one of claims 141-148, wherein the lubricant accounts for 0.1-5%, alternatively, 0.5-2%, alternatively, about 1% by weight of the total weight of the pharmaceutical composition; alternatively, wherein the amount of (v) lubricant in a unit dose is 0.1-20 mg, alternatively, 0.5-8 mg, alternatively, about 1, 2, 3 or 4 mg.

150. The pharmaceutical composition of any one of claims 141-149, wherein the lubricant is magnesium stearate or sodium stearyl fumarate.

151. The pharmaceutical composition of any one of claims 141-150, comprising the following ingredients:
(i) 1-30% of the crystal form VI of compound A, by weight,
(ii) 70-90% of lactose monohydrate and microcrystalline cellulose (1:2 by weight), by weight,
(iii) 2-4% of croscarmellose sodium, by weight,
(iv) 2-4% of colloidal silica, by weight, and
(v) 0.1-5% of magnesium stearate, by weight.

152. The pharmaceutical composition of claim 151, wherein a unit dose comprises the following components:
(i) about 10 mg of the crystal form VI of compound A,
(ii) about 28 mg of lactose monohydrate and about 55 mg of microcrystalline cellulose,
(iii) about 3 mg of croscarmellose sodium,
(iv) about 3 mg of colloidal silica, and
(v) about 1 mg of magnesium stearate.

153. The pharmaceutical composition of claim 151, wherein a unit dose comprises the following components:
(i) about 40 mg of the crystal form VI of compound A,
(ii) about 112 mg of lactose monohydrate and about 220 mg of microcrystalline cellulose,
(iii) about 12 mg of croscarmellose sodium,
(iv) about 12 mg of colloidal silica, and
(v) about 4 mg of magnesium stearate.

154. The pharmaceutical composition of any one of claims 141-153, which is a tablet, alternatively, a coated tablet.

155. A pharmaceutical composition comprising:
(i) a compound of formula (A),
(ii) a diluent,
(iii) a disintegrant,
(iv) a glidant, and
(v) a lubricant,
wherein the diluent accounts for 1-5%, alternatively, 2-4%, alternatively, about 3% by weight of the total weight of the pharmaceutical composition.

156. The pharmaceutical composition of claim 155, wherein the glidant is colloidal silica.

157. The pharmaceutical composition of claim 155 or 156, wherein the amount of the glidant in a unit dose is 1-20 mg, alternatively, 2-16 mg, alternatively, about 3, 6, 9 or 12 mg.

158. The pharmaceutical composition of any one of claims 155-157, wherein the diluent accounts for 65-95%, alternatively, 70-90%, alternatively, about 80%, 81%, 82%, 83%, 84% or 85% by weight of the total weight of the pharmaceutical composition; alternatively, wherein the amount of the diluent in a unit dose is 65-380 mg, alternatively, 70-360 mg, alternatively, 80-350 mg, such as about 83 mg or 332 mg.

159. The pharmaceutical composition of any one of claims 155-158, wherein the diluent is selected from lactose monohydrate, microcrystalline cellulose, anhydrous calcium hydrogen phosphate, mannitol and pregelatinized starch, and a mixture thereof; alternatively, where both of lactose monohydrate and microcrystalline cellulose are present, the weight ratio of lactose monohydrate to microcrystalline cellulose is 5:1 to 1:5, alternatively, 2:1 to 1:3, alternatively, about 1:2, such as 1:1.96.

160. The pharmaceutical composition of any one of claims 155-159, wherein the disintegrant accounts for 1-5%, alternatively, 2-4%, alternatively, about 3% by weight of the total weight of the pharmaceutical composition; alternatively, wherein the amount of the disintegrant in a unit dose is 1-20 mg, alternatively, 2-16 mg, alternatively, about 3, 6, 9 or 12 mg.

161. The pharmaceutical composition of any one of claims 155-160, wherein the disintegrant is croscarmellose sodium.

162. The pharmaceutical composition of any one of claims 155-161, wherein the lubricant accounts for 0.1-5%, alternatively, 0.5-2%, alternatively, about 1% by weight of the total weight of the pharmaceutical composition; alternatively, wherein the amount of the lubricant in a unit dose is 0.1-20 mg, alternatively, 0.5-8 mg, alternatively, about 1, 2, 3 or 4 mg.

163. The pharmaceutical composition of any one of claims 155-162, wherein the lubricant is magnesium stearate or sodium stearyl fumarate.

164. Use of the crystal form of the compound of formula (A) of any one of claims 1-116 in the manufacture of a medicament for the treatment and/or prevention of diseases caused by Bcr-Abl.

165. Use of the crystal form of the compound of formula (A) of any one of claims 1-116 in the manufacture of a medicament for the treatment and/or prevention of the following diseases: solid tumors, sarcomas, acute lymphocytic leukemia, acute myelocytic leukemia, chronic lymphocytic leukemia, chronic myelocytic leukemia, gastrointestinal stromal tumor, thyroid cancer, gastric cancer, rectal cancer, multiple myeloma, neoplasia, and other proliferative diseases; or the disease caused by the Bcr-Abl1 is metastatic invasive cancer, viral infection, or CNS disorder.

166. The crystal form of the compound of formula (A) of any one of claims 1-116 for use in the treatment and/or prevention of diseases caused by Bcr-Abl.

167. The crystal form of the compound of formula (A) of any one of claims 1-116 for use in the treatment and/or prevention of solid tumors, sarcomas, acute lymphocytic leukemia, acute myelocytic leukemia, chronic lymphocytic leukemia, chronic myelocytic leukemia, gastrointestinal stromal tumor, thyroid cancer, gastric cancer, rectal cancer, multiple myeloma, neoplasia, and other proliferative diseases; or the disease caused by the Bcr-Abl1 is metastatic invasive cancer, viral infection, or CNS disorder.

168. A method of treating and/or preventing a disease caused by Bcr-Abl in a subject, comprising administering to the subject the crystal form of the compound of formula (A) of any one of claims 1-116.

169. A method of treating and/or preventing the following diseases in a subject, comprising administering to the subject the crystal form of the compound of formula (A) of any one of claims 1-116: solid tumors, sarcomas, acute lymphocytic leukemia, acute myelocytic leukemia, chronic lymphocytic leukemia, chronic myelocytic leukemia, gastrointestinal stromal tumor, thyroid cancer, gastric cancer, rectal cancer, multiple myeloma, neoplasia, and other proliferative diseases; or the disease caused by the Bcr-Abl1 is metastatic invasive cancer, viral infection, or CNS disorder.
